# EUROPEAN PATENT APPLICATION

(11) **EP 2 902 387 A1**
(43) Date of publication of application: **05.08.2015**
(21) Application number: 13840288.8
(22) Date of filing: 25.09.2013
(51) Int. Cl.: C07D 307/62, A01N 43/08, A01N 43/14, A01N 43/28, A01N 43/40, A01P 1/00, C07D 405/14, C07D 407/04, C07H 17/04

(54) **ASCORBIC ACID-RELATED COMPOUND AND ANTI-PLANT-VIRUS AGENT**

(30) Priority: 27.09.2012 JP 2012214282
(71) Applicant: Nippon Soda Co., Ltd., Tokyo 100-8165 (JP)
(72) Inventor: IHORI Yoichi, Odawara-shi Kanagawa 250-0280 (JP); INOUE Shuji, Odawara-shi Kanagawa 250-0280 (JP); FUJII Takayuki, Odawara-shi Kanagawa 250-0280 (JP); OSAWA Yoko, Odawara-shi Kanagawa 250-0280 (JP); TANAKA Tetsuya, Odawara-shi Kanagawa 250-0280 (JP); IKEUCHI Hideyuki, Odawara-shi Kanagawa 250-0280 (JP)
(74) Representative: Wibbelmann, Jobst
(86) International application number: PCT/JP2013/075920
(87) International publication number: WO 2014/050894

(57) **Abstract**

The present invention provides a compound represented by formula (1) (wherein each of X¹ and X² independently represents -OR¹, -NR²R³ or -CR⁴R⁵R⁶ or the like, each of R¹, R², R³, R⁴, R⁵ and R⁶ independently represents an unsubstituted or substituent-bearing glycosyl group or an unsubstituted or substituent-bearing C1 to C30 alkyl group or the like, and A represents an unsubstituted or substituent-bearing C1 to C30 alkyl group or the like) or a salt thereof.

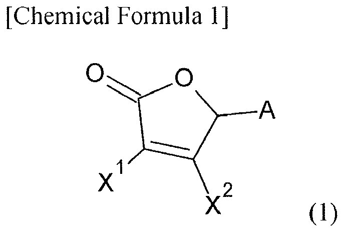

## Description

### TECHNICAL FIELD

The present invention relates to an ascorbic acid-related compound that is useful as the active ingredient of a plant antiviral agent or the like.

Priority is claimed on Japanese Patent Application No. 2012-214282, filed September 27, 2012, the content of which is incorporated herein by reference.

### BACKGROUND ART

Ascorbic acid and derivatives thereof are used in pharmaceuticals, cosmetics, foodstuffs and animal feed and the like. Further, other uses that have been proposed include the prevention of skin blemishes and freckles and the like caused by sunburn, and hair growth agents (for example, see Patent Document 1 and Patent Document 2). Furthermore, it is also known that specific ascorbic acid derivatives have efficacy as a control agent for plant viral diseases (Patent Document 3).

Further, ascorbic acid and derivatives thereof are also typically known as reducing agents, and are used, for example, as the principal agent in photographic developing solutions and the like (for example, see Patent Document 4 and Patent Document 5).

Moreover, other varieties of ascorbic acid derivatives have been proposed for use as pharmaceutical agents having an anti-inflammatory effect or the like (Patent Document 6).

### PRIOR ART LITERATURE

### Patent Documents

Patent Document 1: Japanese Unexamined Patent Application, First Publication No. 2000-151905
Patent Document 2: Japanese Unexamined Patent Application, First Publication No. 2001-354522
Patent Document 3: International Patent Publication No. WO 2011/030816
Patent Document 4: Japanese Unexamined Patent Application, First Publication No. 2001-324782
Patent Document 5: Japanese Unexamined Patent Application, First Publication No. Hei 08-297350
Patent Document 6: Published Japanese Translation No. Hei 10-504523 of PCT

### DISCLOSURE OF INVENTION

### Problems to be Solved by the Invention

An object of the present invention is to provide a novel ascorbic acid-related compound that is useful as the active ingredient of a plant antiviral agent or the like. Means for Solving the Problems

In order to achieve the above object, the inventors of the present invention conducted an intensive search for compounds having plant antiviral activity. As a result, they discovered that a conventionally unknown ascorbic acid-related compound, and another ascorbic acid-related compound, which although being a known compound, was not known to possess a plant antiviral effect, exhibited excellent plant antiviral effects. The present invention was completed by performing further investigations based upon these findings.

In other words, the present invention provides the following aspects.
[1] A compound represented by formula (1), or a salt thereof.

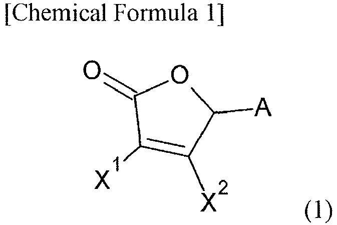

In formula (1), each of X¹ and X² independently represents a hydrogen atom, halogeno group, cyano group, nitro group, -OR¹, -NR²R³ or -CR⁴R⁵R⁶, and X¹ and X² may be linked to form an unsubstituted or substituent-bearing 5- to 8-membered ring.

Each of R¹, R², R³, R⁴, R⁵ and R⁶ in X¹ and X² independently represents a hydrogen atom, unsubstituted or substituent-bearing glycosyl group, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing 5- to 10-membered heterocyclic group, -COR¹¹, -COOR¹², -CONR¹³R¹⁴, -SO₂R¹⁵,-PO(OH)OR¹⁶, -SO₂NR¹⁷R¹⁸, or -SiR¹⁹R²⁰R²¹.

Any of R² and R³, R⁴ and R⁵, R⁶ and R⁴, or R⁵ and R⁶ may be linked to form an unsubstituted or substituent-bearing 3- to 8-membered ring, and any of R⁴ and R⁵, R⁶ and R⁴, or R⁵ and R⁶ may be combined to form =O, =S or =NR²².

Each of R¹¹, R¹², R¹³, R¹⁴, R¹⁵, R¹⁶, R¹⁷, R¹⁸, R¹⁹, R²⁰ and R²¹ independently represents a hydrogen atom, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing 5- to 10-membered heterocyclic group, or a substituent-bearing carbonyl group.

R²² represents a hydrogen atom, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, or unsubstituted or substituent-bearing 5- to 10-membered heterocyclic group.

In formula (1), A represents an unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, or unsubstituted or substituent-bearing C5 to C10 heterocyclic group.

However, those cases in which each of X¹, X², R²³ and R²⁴ simultaneously represents a hydroxyl group, glycosyloxy group, -OSO₃H, -OPO₃H₂ or -COR' are excluded, and
when A represents an unsubstituted ethyl group, those cases in which X¹ and X² both represent hydrogen atoms are excluded.

R' represents an unsubstituted or substituent-bearing C1 to C30 alkyl group or an unsubstituted or substituent-bearing C2 to C30 alkenyl group.
[2] A compound represented by formula (2), or a salt thereof.

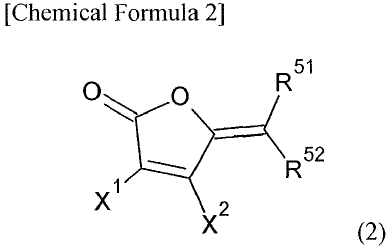

In formula (2), each of X¹ and X² independently represents a hydrogen atom, halogeno group, cyano group, nitro group, -OR¹, -NR²R³ or -CR⁴R⁵R⁶, and X¹ and X² may be linked to form an unsubstituted or substituent-bearing 5- to 8-membered ring.

Each of R¹, R², R³, R⁴, R⁵ and R⁶ independently represents a hydrogen atom, unsubstituted or substituent-bearing glycosyl group, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing 5- to 10-membered heterocyclic group, -COR", -COOR¹², -CONR¹³R¹⁴, -SO₂R¹⁵, - PO(OH)OR¹⁶, -SO₂NR¹⁷R¹⁸, or -SiR¹⁹R²⁰R²¹.

Any of R² and R³, R⁴ and R⁵, R⁶ and R⁴, or R⁵ and R⁶ may be linked to form an unsubstituted or substituent-bearing 3- to 8-membered ring, and any of R⁴ and R⁵, R⁶ and R⁴, or R³ and R⁶ may be combined to form =O, =S or =NR²².

Each of R¹¹, R¹², R¹³, R¹⁴, R¹⁵, R¹⁶, R¹⁷, R¹⁸, R¹⁹, R²⁰ and R²¹ independently represents a hydrogen atom, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing 5- to 10-membered heterocyclic group, or a substituent-bearing carbonyl group.

R²² represents a hydrogen atom, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, or unsubstituted or substituent-bearing 5- to 10-membered heterocyclic group.

In formula (2), each of R⁵¹ and R⁵² independently represents a hydrogen atom, hydroxyl group, unsubstituted or substituent-bearing amino group, halogeno group, cyano group, nitro group, unsubstituted or substituent-bearing C1 to C30 alkyl group, unsubstituted or substituent-bearing C2 to C30 alkenyl group, unsubstituted or substituent-bearing C2 to C30 alkynyl group, carboxyl group, formyl group, substituent-bearing carbonyl group, substituent-bearing carbonyloxy group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing C5 to C10 heterocyclic group, unsubstituted or substituent-bearing C1 to C30 alkoxy group, unsubstituted or substituent-bearing C2 to C30 alkenyloxy group, unsubstituted or substituent-bearing C2 to C30 alkynyloxy group, unsubstituted or substituent-bearing C3 to C20 cycloalkyloxy group, unsubstituted or substituent-bearing C4 to C20 cycloalkenyloxy group, unsubstituted or substituent-bearing C8 to C20 cycloalkynyloxy group, unsubstituted or substituent-bearing C6 to C10 aryloxy group, or unsubstituted or substituent-bearing C5 to C10 heterocyclic oxy group. R⁵¹ and R⁵² may be linked to form an unsubstituted or substituent-bearing 5- to 8-membered ring.
[3] A plant antiviral agent comprising the compound described in [1] or a salt thereof.
[4] A plant antiviral agent comprising the compound described in [2] or a salt thereof.
[5] A method of preventing or curing a plant virus, the method comprising use of a plant antiviral agent comprising the compound described in [1] or a salt thereof.
[6] A method of preventing or curing a plant virus, the method comprising use of a plant antiviral agent comprising the compound described in [2] or a salt thereof.

### Effects of the Invention

The compound of the present invention or a salt thereof is effective as an active ingredient of a plant antiviral agent.

The plant antiviral agent of the present invention contains at least one component selected from the group consisting of compounds of the present invention and salts thereof, and has a high plant antiviral activity. When the plant antiviral agent of the present invention is applied to a normal plant, infection with plant viruses can be effectively prevented (preventative effect). Further, when the plant antiviral agent of the present invention is applied to a plant that has been infected with a plant virus, onset of the plant disease can be suppressed (curative effect).

Further, the compound of the present invention or a salt thereof exhibits excellent stability, and is therefore suitable for agricultural and horticultural use.

### BEST MODE FOR CARRYING OUT THE INVENTION

The compound of the present invention or a salt thereof is a compound represented by formula (1) or a salt thereof (hereafter also referred to as "compound (1)"), or a compound represented by formula (2) or a salt thereof (hereafter also referred to as "compound (2)").

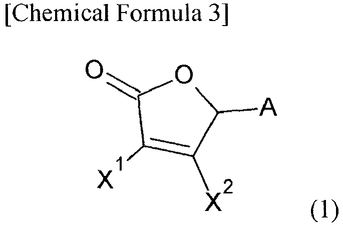

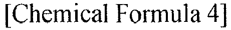

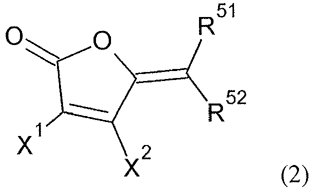

The compound (1) and the compound (2) of the present invention also include the hydrates, various solvates and crystal polymorphs and the like of the compounds. Moreover, the compound of the present invention or the salt thereof also includes the various stereoisomers or mixtures thereof resulting from chiral carbon atoms or double bonds.

The meanings of the various symbols used in the formula (1) and the formula (2) are described below.

First, the meanings of the terms "unsubstituted" and "substituent-bearing" are described.

The term "unsubstituted" means only the core group. In those cases where the term "substituent-bearing" is not included and only the name of the core group is listed, unless specifically stated otherwise, the groups is deemed to be "unsubstituted".

On the other hand, the term "substituent-bearing" means that one of the hydrogen atoms of the core group has been substituted with a structure that is either the same as the core group or different from the core group. Accordingly, a "substituent" describes another group that is bonded to the core group. There may be one substituent, or two or more substituents. In the case of two or more substituents, the substituents may be the same or different.

The terms such as "C1 to C6" mean that the number of carbon atoms within the core group is from 1 to 6 or the like. This number of carbon atoms does not include the number of carbon atoms contained within any substituents. For example, a butyl group having an ethoxy group as a substituent is classified as a C2-alkoxy C4-alkyl group.

There are no particular limitations on the "substituent", provided it is chemically permissible and allows the compound to exhibit the effects of the present invention.

Examples of groups that can function as the "substituent" include halogeno groups such as a fluoro group, chloro group, bromo group and iodo group; C1 to C6 alkyl groups such as a methyl group, ethyl group, n-propyl group, i-propyl group, n-butyl group, s-butyl group, i-butyl group, t-butyl group, n-pentyl group and n-hexyl group; C3 to C6 cycloalkyl groups such as a cyclopropyl group, cyclobutyl group, cyclopentyl group and cyclohexyl group; C2 to C6 alkenyl groups such as a vinyl group, 1-propenyl group, 2-propenyl group, 1-butenyl group, 2-butenyl group, 3-butenyl group, 1-methyl-2-propenyl group, 2-methyl-2-propenyl group, 1-pentenyl group, 2-pentenyl group, 3-pentenyl group, 4-pentenyl group, 1-methyl-2-butenyl group, 2-methyl-2-butenyl group, 1-hexenyl group, 2-hexenyl group, 3-hexenyl group, 4-hexenyl group and 5-hexenyl group; C3 to C6 cycloalkenyl groups such as a 2-cyclopropenyl group, 2-cyclopentenyl group and 3-cyclohexenyl group; and C2 to C6 alkynyl groups such as an ethynyl group, 1-propynyl group, 2-propynyl group, 1-butynyl group, 2-butynyl group, 3-butynyl group, 1-methyl-2-propynyl group, 2-methyl-3-butynyl group, 1-pentynyl group, 2-pentynyl group, 3-pentynyl group, 4-pentynyl group, 1-methyl-2-butynyl group, 2-methyl-3-pentynyl group, 1-hexynyl group and 1,1-dimethyl-2-butynyl group;

C1 to C6 alkoxy groups such as a methoxy group, ethoxy group, n-propoxy group, i-propoxy group, n-butoxy group, s-butoxy group, i-butoxy group and t-butoxy group; C2 to C6 alkenyloxy groups such as a vinyloxy group, allyloxy group, propenyloxy group and butenyloxy group; C2 to C6 alkynyloxy groups such as an ethynyloxy group and propargyloxy group; C6 to C10 aryl groups such as a phenyl group and naphthyl group; C6 to C10 aryloxy groups such as a phenoxy group and 1-naphthoxy group; C7 to C 11 aralkyl groups such as a benzyl group and phenethyl group; C7 to C11 aralkyloxy groups such as a benzyloxy group and phenethyloxy group; C1 to C7 acyl groups such as a formyl group, acetyl group, propionyl group, benzoyl group and cyclohexylcarbonyl group; C1 to C7 acyloxy groups such as a formyloxy group, acetyloxy group, propionyloxy group, benzoyloxy group and cyclohexylcarbonyloxy group; C 1 to C6 alkoxycarbonyl groups such as a methoxycarbonyl group, ethoxycarbonyl group, n-propoxycarbonyl group, i-propoxycarbonyl group, n-butoxycarbonyl group and t-butoxycarbonyl group; a carboxyl group;

a hydroxyl group; an oxo group; C1 to C6 haloalkyl groups such as a chloromethyl group, chloroethyl group, trifluoromethyl group, 1,2-dichloro-n-propyl group, 1-fluoro-n-butyl group and perfluoro-n-pentyl group; C2 to C6 haloalkenyl groups such as a 2-chloro-1-propenyl group and 2-fluoro-1-butenyl group; C2 to C6 haloalkynyl groups such as a 4,4-dichloro-1-butynyl group, 4-fluoro-1-pentynyl group and 5-bromo-2-pentynyl group; C1 to C6 haloalkoxy groups such as a 2-chloro-n-propxy group and 2,3-dichlorobutoxy group; C2 to C6 haloalkenyloxy groups such as a 2-chloropropenyloxy group and 3-bromobutenyloxy group; C6 to C10 haloaryl groups such as a 4-chlorophenyl group, 4-fluorophenyl group and 2,4-dichlorophenyl group; C6 to C10 haloaryloxy groups such as a 4-fluorophenyloxy group and 4-chloro-1-naphthoxy group; C1 to C7 haloacyl groups such as a chloroacetyl group, trifluoroacetyl group, trichloroacetyl group and 4-chlorobenzoyl group;

a cyano group; isocyano group; nitro group; isocyanate group; cyanate group; amide group; amino group; C1 to C6 alkylamino groups such as a methylamino group, dimethylamino group and diethylamino group; C6 to C10 arylamino groups such as an anilino group and naphthylamino group; C7 to C11 aralkylamino groups such as a benzylamino group and phenylethylamino group; C1 to C7 acylamino groups such as a formylamino group, acetylamino group, propanoylamino group, butyrylamino group, i-propylcarbonylamino group and benzoylamino group; C1 to C6 alkoxycarbonylamino groups such as a methoxycarbonylamino group, ethoxycarbonylamino group, n-propoxycarbonylamino group and i-propoxycarbonylamino group; a carbamoyl group; substituted carbamoyl groups such as a dimethylcarbamoyl group, phenyl carbamoyl group and N-phenyl-N-methylcarbamoyl group; imino C1 to C6 alkyl groups such as an iminomethyl group, (1-imino)ethyl group, and (1-imino)-n-propyl group; hydroxyimino C1 to C6 alkyl groups such as a hydroxyiminomethyl group, (1-hydroxyimino)ethyl group and (1-hydroxyimino)propyl group; C1 to C6 alkoxyimino C1 to C6 alkyl groups such as a methoxyiminomethyl group and (1-methoxyimino)ethyl group;

a mercapto group; isothiocyanate group; thiocyanate group; C1 to C6 alkylthio groups such as a methylthio group, ethylthio group, n-propylthio group, i-propylthio group, n-butylthio group, i-butylthio group, s-butylthio group and t-butylthio group; C2 to C6 alkenylthio groups such as a vinylthio group and allylthio group; C2 to C6 alkynylthio groups such as an ethynylthio group and propargylthio group; C6 to C10 arylthio groups such as a phenylthio group and naphthylthio group; heteroarylthio groups such as a thiazolylthio group and pyridylthio group; C7 to C11 aralkylthio groups such as a benzylthio group and phenethylthio group; (C1 to C6 alkylthio)carbonyl groups such as a (methylthio)carbonyl group, (ethylthio)carbonyl group, (n-propylthio)carbonyl group, (i-propylthio)carbonyl group, (n-butylthio)carbonyl group, (i-butylthio)carbonyl group, (s-butylthio)carbonyl group and (t-butylthio)carbonyl group;

C1 to C6 alkylsulfinyl groups such as a methylsulfinyl group, ethylsulfinyl group and t-butylsulfinyl group; C2 to C6 alkenylsulfinyl groups such as an allylsulfinyl group; C2 to C6 alkynylsulfinyl groups such as a propargylsulfinyl group; C6 to C10 arylsulfinyl groups such as a phenylsulfinyl group; heteroarylsulfinyl groups such as a thiazolylsulfinyl group and pyridylsulfinyl group; C7 to C11 aralkylsulfinyl groups such as a benzylsulfinyl group and phenethylsulfinyl group; C1 to C6 alkylsulfonyl groups such as a methylsulfonyl group, ethylsulfonyl group and t-butylsulfonyl group; C2 to C6 alkenylsulfonyl groups such as an allylsulfonyl group; C2 to C6 alkynylsulfonyl groups such as a propargylsulfonyl group; C6 to C10 arylsulfonyl groups such as a phenylsulfonyl group; heteroarylsulfonyl groups such as a thiazolylsulfonyl group and pyridylsulfonyl group; C7 to C11 aralkylsulfonyl groups such as a benzylsulfonyl group and phenethylsulfonyl group;

5-membered heteroaryl groups such as a pyrrolyl group, furyl group, thienyl group, imidazolyl group, pyrazolyl group, oxazolyl group, isoxazolyl group, thiazolyl group, isothiazolyl group, triazolyl group, oxadiazolyl group, thiadiazolyl group and tetrazolyl group; 6-membered heteroaryl groups such as a pyridyl group, pyrazinyl group, pyrimidinyl group, pyridazinyl group and triazinyl group; saturated heterocyclic groups such as an aziridinyl group, epoxy group, pyrrolidinyl group, tetrahydrofuranyl group, piperidyl group, piperazinyl group and morpholinyl group; C1 to C6 alkylsilyl groups such as a trimethylsilyl group, triethylsilyl group and t-butyldimethylsilyl group; and a triphenylsilyl group.

Further, these "substituents" may further contain a separate "substituent" within the substituent. For example, a butyl group as a substituent may further contain an ethoxy group as a separate substituent, namely a group such as an ethoxybutyl group.

Furthermore, these "substituents" may be divalent or higher groups that are substituted simultaneously at two or more substitution positions. Examples of this type of divalent substituent include C1 to C8 alkylene groups such as a butylene group, pentylene group and hexylene group; and oxyoxalyloxy groups.

### [X¹ and X²]

In formula (1) and formula (2), each of X¹ and X² independently represents a hydrogen atom, halogeno group, cyano group, nitro group, -OR¹, -NR²R³ or -CR⁴R⁵R⁶, and X¹ and X² may be linked to form an unsubstituted or substituent-bearing 5- to 8-membered ring.

Each of R¹, R², R³, R⁴, R⁵ and R⁶ independently represents a hydrogen atom, unsubstituted or substituent-bearing glycosyl group, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing 5- to 10-membered heterocyclic group, -COR¹¹, -COOR¹², -CONR¹³R¹⁴, -SO₂R¹⁵, - PO(OH)OR¹⁶, -SO₂NR¹⁷R¹⁸, or -SiR¹⁹R²⁰R²¹,

Any of R² and R³, R⁴ and R⁵, R⁶ and R⁴ or R⁵ and R⁶ may be linked to form an unsubstituted or substituent-bearing 3- to 8-membered ring, and any of R⁴ and R⁵, R⁶ and R⁴, or R⁵ and R⁶ may be combined to form =O, =S or =NR²².

Examples of the unsubstituted or substituent-bearing glycosyl group include groups formed from monosaccharides, such as a glucosyl group, galactosyl group, fructosyl group and rhamnosyl group; groups formed from disaccharides in which an arbitrary combination of monosaccharide groups is bonded together by a 1→2 bond, 1→3 bond, 1→4 bond or 1→6 bond; and substituent-bearing glycosyl groups such as a 2,3,4,6-tetramethylglucosyl group.

Examples of the halogeno group include a fluoro group, chloro group, bromo group and iodo group.

Examples of the C1 to C30 alkyl group include a methyl group, ethyl group, n-propyl group, i-propyl group, n-butyl group, s-butyl group, i-butyl group, t-butyl group, n-pentyl group and n-hexyl group.

Examples of the substituent-bearing C1 to C30 alkyl group include cycloalkylalkyl groups, haloalkyl groups, arylalkyl groups, heteroarylalkyl groups, hydroxyalkyl groups, alkoxyalkyl groups, acyloxyalkyl groups and acylalkyl groups.

Examples of the unsubstituted or substituent-bearing C2 to C30 alkenyl group include a vinyl group, 1-propenyl group, 2-propenyl group (allyl group), 1-butenyl group, 2-butenyl group, 3-butenyl group, 1-methyl-2-propenyl group, 2-methyl-2-propenyl group, 1-pentenyl group, 2-pentenyl group, 3-pentenyl group, 4-pentenyl group, 1-methyl-2-butenyl group, 2-methyl-2-butenyl group, 1-hexenyl group, 2-hexenyl group, 3-hexenyl group, 4-hexenyl group and 5-hexenyl group.

Examples of the unsubstituted or substituent-bearing C2 to C30 alkynyl group include an ethynyl group, 1-propynyl group, 2-propynyl group, 1-butynyl group, 2-butynyl group, 3-butynyl group, 1-methyl-2-propynyl group, 2-methyl-3-butynyl group, 1-pentynyl group, 2-pentynyl group, 3-pentynyl group, 4-pentynyl group, 1-methyl-2-butynyl group, 2-methyl-3-pentynyl group, 1-hexynyl group and 1,1-dimethyl-2-butynyl group.

Examples of the C3 to C20 cycloalkyl group include a cyclopropyl group, cyclobutyl group, cyclopentyl group, cyclohexyl group and cycloheptyl group.

In the substituent-bearing C3 to C20 cycloalkyl group, examples of preferred substituents include halogen atoms such as a fluorine atom, chlorine atom, bromine atom and iodine atom; and C1 to C6 alkyl groups such as a methyl group, ethyl group, n-propyl group, i-propyl group, n-butyl group, s-butyl group, i-butyl group, t-butyl group, n-pentyl group and n-hexyl group.

Examples of the unsubstituted or substituent-bearing C4 to C20 cycloalkenyl group include a 2-cyclopentenyl group, 3-cyclohexenyl group and 4-cyclooctenyl group.

Examples of the unsubstituted or substituent-bearing C8 to C20 cycloalkynyl group include a cyclooctynyl group, cyclononynyl group and cyclodecynyl group.

Examples of the unsubstituted or substituent-bearing C6 to C10 aryl group include a phenyl group, naphthyl group, 4-chlorophenyl group, 4-fluorophenyl group and 2,4-dichlorophenyl group.

Examples of the unsubstituted or substituent-bearing 5- to 10-membered heterocyclic group include groups containing 1 to 4 hetero atoms selected from among a nitrogen atom, an oxygen atom and a sulfur atom as the atoms that constitute a ring of the cyclic group. The heterocyclic group may be a heteroaryl group such as a 5-membered heteroaryl group or a 6-membered heteroaryl group, or may be a saturated heterocyclic group. Further, the heterocyclic group may be a monocyclic group or a polycyclic group.

Specific examples of the 5-membered heteroaryl group include pyrrolyl groups such as a pyrrol-1-yl group, pyrrol-2-yl group and pyrrol-3-yl group; furyl groups such as a furan-2-yl group and furan-3-yl group; thienyl groups such as a thiophen-2-yl group and thiophen-3-yl group; imidazolyl groups such as an imidazol-1-yl group, imidazol-2-yl group, imidazol-4-yl group and imidazol-5-yl group; pyrazolyl groups such as a pyrazol-1-yl group, pyrazol-3-yl group, pyrazol-4-yl group and pyrazol-5-yl group; oxazolyl groups such as an oxazol-2-yl group, oxazol-4-yl group and oxazol-5-yl group; isoxazolyl groups such as an isoxazol-3-yl group, isoxazol-4-yl group and isoxazol-5-yl group; thiazolyl groups such as a thiazol-2-yl group, thiazol-4-yl group and thiazol-5-yl group; isothiazolyl groups such as a isothiazol-3-yl group, isothiazol-4-yl group and isothiazol-5-yl group; triazolyl groups such as a 1,2,3-triazol-1-yl group, 1,2,3-triazol-4-yl group, 1,2,3-triazol-5-yl group, 1,2,4-triazol-1-yl group, 1,2,4-triazol-3-yl group and 1,2,4-triazol-5-yl group; oxadiazolyl groups such as a 1,2,4-oxadiazol-3-yl group, 1,2,4-oxadiazol-5-yl group and 1,3,4-oxadiazol-2-yl group; thiadiazolyl groups such as a 1,2,4-thiadiazol-3-yl group, 1,2,4-thiadiazol-5-yl group and 1,3,4-thiadiazol-2-yl group; and tetrazolyl groups such as a tetrazol-1-yl group and tetrazol-2-yl group.

Specific examples of the 6-membered heteroaryl group include pyridyl groups such as a pyridin-2-yl group, pyridin-3-yl group and pyridin-4-yl group; pyrazinyl groups such as a pyrazin-2-yl group and pyrazin-3-yl group; pyrimidinyl groups such as a pyrimidin-2-yl group, pyrimidin-4-yl group and pyrimidin-5-yl group; pyridazinyl groups such as a pyridazin-3-yl group and pyridazin-4-yl group; and a triazinyl group.

Examples of condensed heteroaryl groups include an indol-1-yl group, indol-2-yl group, indol-3-yl group, indol-4-yl group, indol-5-yl group, indol-6-yl group and indol-7-yl group; a benzofuran-2-yl group, benzofuran-3-yl group, benzofuran-4-yl group, benzofuran-5-yl group, benzofuran-6-yl group and benzofuran-7-yl group; a benzothiophen-2-yl group, benzothiophen-3-yl group, benzothiophen-4-yl group, benzothiophen-5-yl group, benzothiophen-6-yl group and benzothiophen-7-yl group; a benzimidazol-l-yl group, benzimidazol-2-yl group, benzimidazol-4-yl group and benzimidazol-5-yl group; a benzoxazol-2-yl group, benzoxazol-4-yl group and benzoxazol-5-yl group; a benzothiazol-2-yl group, benzothiazol-4-yl group and benzothiazol-5-yl group; and a quinolin-2-yl group, quinolin-3-yl group, quinolin-4-yl group, quinolin-5-yl group, quinolin-6-yl group, quinolin-7-yl group and quinolin-8-yl group.

Examples of other heterocyclic groups include an aziridin-1-yl group, aziridin-2-yl group, epoxy group, pyrrolidin-1-yl group, pyrrolidin-2-yl group, pyrrolidin-3-yl group, tetrahydrofuran-2-yl group, tetrahydrofuran-3-yl group, piperidin-1-yl group, piperidin-2-yl group, piperidin-3-yl group, piperidin-4-yl group, piperazin-1-yl group, piperazin-2-yl group, morpholin-2-yl group, morpholin-3-yl group, morpholin-4-yl group, 1,3-benzoxazol-4-yl group, 1,3-benzoxazol-5-yl group, 1,4-benzoxazol-5-yl group, 1,4-benzoxazol-6-yl group, 3,4-dihydro-2H-1,5-benzodioxepin-6-yl group, 3,4-dihydro-2H-1,5-benzodioxepin-7-yl group, 2,3-dihydrobenzofuran-4-yl group, 2,3-dihydrobenzofuran-5-yl group, 2,3-dihydrobenzofuran-6-yl group and 2,3-dihydrobenzofuran-7-yl group.

In the substituent-bearing 5- to 10-membered heterocyclic group, examples of preferred substituents include halogen atoms such as a fluorine atom, chlorine atom, bromine atom and iodine atom; C1 to C6 alkyl groups such as a methyl group, ethyl group, n-propyl group, i-propyl group, n-butyl group, s-butyl group, i-butyl group, t-butyl group, n-pentyl group and n-hexyl group; C3 to C6 cycloalkyl groups such as a cyclopropyl group, cyclobutyl group, cyclopentyl group and cyclohexyl group; C1 to C6 alkoxy groups such as a methoxy group, ethoxy group, n-propoxy group, i-propoxy group, n-butoxy group, s-butoxy group, i-butoxy group and t-butoxy group; a hydroxyl group; C1 to C6 haloalkyl groups such as a chloromethyl group, chloroethyl group and trifluoromethyl group; and C1 to C6 haloalkoxy groups such as a trifluoromethoxy group and 2,2,2-trifluoroethoxy group.

Each of R¹¹, R¹², R¹³, R¹⁴, R¹⁵, R¹⁶, R¹⁷, R¹⁸, R¹⁹, R²⁰ and R²¹ independently represents a hydrogen atom, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing 5- to 10-membered heterocyclic group, or a substituent-bearing carbonyl group.

Specific examples of the unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, and the unsubstituted or substituent-bearing 5- to 10-membered heterocyclic group for R¹¹, R¹², R¹³, R¹⁴, R¹⁵, R¹⁶, R¹⁷, R¹⁸, R¹⁹, R²⁰ and R²¹ include the same groups as those listed above as examples in the description of R¹ and the like.

Examples of the substituent-bearing carbonyl group include a methoxycarbonyl group, ethoxycarbonyl group, n-propoxycarbonyl group, i-propoxycarbonyl group, n-butoxycarbonyl group, and t-butoxycarbonyl group and the like; an aminocarbonyl group, dimethylaminocarbonyl group, phenylaminocarbonyl group, and N-phenyl-N-methylaminocarbonyl group and the like; and a cyclohexylcarbonyl group and the like.

R²² represents a hydrogen atom, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, or unsubstituted or substituent-bearing 5- to 10-membered heterocyclic group.

Specific examples of the unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, and the unsubstituted or substituent-bearing 5- to 10-membered heterocyclic group for R²² include the same groups as those listed above as examples in the description of R¹ and the like.

### [A]

In formula (1), A represents an unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, or unsubstituted or substituent-bearing C5 to C10 heterocyclic group.

However, those cases in which each of X¹, X², R²³ and R²⁴ simultaneously represents a hydroxyl group, glycosyloxy group, -OSO₃H, -OPO₃H₂ or -COR' are excluded, and
when A represents an unsubstituted ethyl group, those cases in which X¹ and X² both represent hydrogen atoms are excluded.
R' represents an unsubstituted or substituent-bearing C1 to C30 alkyl group or an unsubstituted or substituent-bearing C2 to C30 alkenyl group.

Each of R²³ and R²⁴ is, independently, preferably a hydrogen atom, -OR³¹ or-NR³²R³³. R²³ and R²⁴ may be linked to form a 5- or 6-membered ring.

Each of R³¹, R³² and R³³ independently represents a hydrogen atom, unsubstituted or substituent-bearing glycosyl group, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing 5- to 10-membered heterocyclic group, -COR⁴¹, -COOR⁴², -CONR⁴³R⁴⁴, -SO₂R⁴⁵, -PO(OH)OR⁴⁶, or -SO₂NR⁴⁷R⁴⁸. R³² and R³³ may be linked to form a 3- to 6-membered ring.

Each of R⁴¹, R⁴², R⁴³, R⁴⁴, R⁴⁵, R⁴⁶, R⁴⁷ and R⁴⁸ independently represents a hydrogen atom, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing 5- to 10-membered heterocyclic group, or a substituent-bearing carbonyl group.

Specific examples of the unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, and the unsubstituted or substituent-bearing 5- to 10-membered heterocyclic group for A include the same groups as those listed above as examples in the description of R¹ and the like.

### [R⁵¹ and R⁵²]

In formula (2), each of R⁵¹ and R⁵² independently represents a hydrogen atom, hydroxyl group, unsubstituted or substituent-bearing amino group, halogeno group, cyano group, nitro group, unsubstituted or substituent-bearing C1 to C30 alkyl group, unsubstituted or substituent-bearing C2 to C30 alkenyl group, unsubstituted or substituent-bearing C2 to C30 alkynyl group, carboxyl group, formyl group, substituent-bearing carbonyl group, substituent-bearing carbonyloxy group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing C5 to C10 heterocyclic group, unsubstituted or substituent-bearing C1 to C30 alkoxy group, unsubstituted or substituent-bearing C2 to C30 alkenyloxy group, unsubstituted or substituent-bearing C2 to C30 alkynyloxy group, unsubstituted or substituent-bearing C3 to C20 cycloalkyloxy group, unsubstituted or substituent-bearing C4 to C20 cycloalkenyloxy group, unsubstituted or substituent-bearing C8 to C20 cycloalkynyloxy group, unsubstituted or substituent-bearing C6 to C10 aryloxy group, or unsubstituted or substituent-bearing C5 to C10 heterocyclic oxy group. R⁵¹ and R⁵² may be linked to form an unsubstituted or substituent-bearing 5- to 8-membered ring.

Specific examples of the halogeno group for R⁵¹ and R⁵², unsubstituted or substituent-bearing C1 to C30 alkyl group, unsubstituted or substituent-bearing C2 to C30 alkenyl group, unsubstituted or substituent-bearing C2 to C30 alkynyl group, substituent-bearing carbonyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, and unsubstituted or substituent-bearing C5 to C10 heterocyclic group include the same groups as those listed above as examples in the descriptions of R¹ and A and the like.

The unsubstituted or substituent-bearing C1 to C30 alkoxy group is a group in which an alkyl group such as those listed above in relation to R¹ and the like is bonded via an oxy group. Specific examples include a methoxy group, ethoxy group, n-propoxy group, i-propoxy group, n-butoxy group and t-butoxy group.

The unsubstituted or substituent-bearing C2 to C30 alkenyloxy group is a group in which an alkenyl group such as those listed above in relation to R¹ and the like is bonded via an oxy group. Specific examples include an ethenyloxy group, 1-propenyloxy group and allyloxy group.

The unsubstituted or substituent-bearing C2 to C30 alkynyloxy group is a group in which an alkynyl group such as those listed above in relation to R¹ and the like is bonded via an oxy group. Specific examples include an ethynyloxy group, 1-propynyloxy group and 2-propynyloxy group.

The unsubstituted or substituent-bearing C3 to C20 cycloalkyloxy group is a group in which a cycloalkyl group such as those listed above in relation to R¹ and the like is bonded via an oxy group. Specific examples include a cyclopropyloxy group, cyclobutyloxy group, cyclopentyloxy group, cyclohexyloxy group and cycloheptyloxy group.

The unsubstituted or substituent-bearing C4 to C20 cycloalkenyloxy group is a group in which a cycloalkenyl group such as those listed above in relation to R¹ and the like is bonded via an oxy group. Specific examples include a 2-cyclopentenyloxy group, 3-cyclohexenyloxy group and 4-cyclooctenyloxy group.

The unsubstituted or substituent-bearing C8 to C20 cycloalkynyloxy group is a group in which a cycloalkynyl group such as those listed above in relation to R¹ and the like is bonded via an oxy group.

The unsubstituted or substituent-bearing C6 to C10 aryloxy group is a group in which an aryl group such as those listed above in relation to R¹ and the like is bonded via an oxy group. Specific examples include a phenyloxy group and naphthyloxy group.

The unsubstituted or substituent-bearing C5 to C10 heterocyclic oxy group is a group in which a heterocyclic group such as those listed above in relation to R¹ and the like is bonded via an oxy group.

Examples of the substituent-bearing amino group include a methylamino group, dimethylamino group, ethylamino group and diethylamino group.

Examples of the substituent-bearing carbonyloxy group include a methoxycarbonyloxy group, ethoxycarbonyloxy group, n-propoxycarbonyloxy group, i-propoxycarbonyloxy group, n-butoxycarbonyloxy group and t-butoxycarbonyloxy group; an aminocarbonyloxy group, dimethylaminocarbonyloxy group, phenylaminocarbonyloxy group and N-phenyl-N-methylaminocarbonyloxy group; and a cyclohexylcarbonyloxy group.

There are no particular limitations on the salt of the compound of the present invention, provided the salt is permissible in agricultural and horticultural use, and examples include alkali metal salts such as sodium salts and potassium salts, and alkaline earth metal salts such as calcium salts and magnesium salts.

The compound of the present invention and the salt thereof can be obtained using known synthetic mehods. For example, these compounds can be synthesized by subjecting ascorbic acid, an ester thereof, or a modified compound thereof in which a specific hydroxyl group has been modified with a protective group, to a substitution reaction, a reduction reaction or a coupling reaction or the like to introduce the various types of substituents.

The structures of the compound of the present invention and the salt thereof can be identified and confirmed using known analytical methods such as IR spectroscopy, NMR spectroscopy, mass spectroscopy, and elemental analysis and the like. Further, when the synthetic method described above is used to obtain a mixture of a dehydroascorbic acid derivative and a salt thereof, the targeted substance can be isolated by conventional purification methods such as extraction, distillation and chromatography.

The plant antiviral agent of the present invention contains at least one material selected from the group consisting of compounds of the present invention and the salts thereof as an active ingredient.

Further, the plant antiviral agent of the present invention may also include other optional components, provided they do not prevent the plant antiviral activity. Examples of these other optional components include fillers, extenders, binders, humectants, disintegrants, lubricants, diluents, excipients, spreading agents, germicides, fungicides, bactericides, acaricides, insecticides, herbicides, growth regulators and solvents.

From the viewpoint of obtaining a superior plant antiviral agent, the plant antiviral agent of the present invention preferably includes substances which promote the general resistance of plants to viruses. Examples of these substances which promote resistance include germicides such as probenazole and tiadinil, as well as isonicotinic acid and salicylic acid.

Further, the plant antiviral agent of the present invention preferably also includes a surfactant in order that the compound of the present invention or salt thereof can be dispersed or dissolved uniformly in the solvent. Examples of the surfactant include anionic surfactants, cationic surfactants, nonionic surfactants and amphiphilic surfactants.

There are no particular limitations on the formulation of the plant antiviral agent of the present invention. Depending on the plant to which the agent is to be applied, the formulation may be selected appropriately from among wettable powder, emulsible concentrate, water soluble powders, granules, dusting formulations, and tablets and the like. There are no particular limitations on the method used for preparing the plant antiviral agent of the present invention, and an appropriate method may be selected from among known preparation methods in accordance with the desired formulation.

There are no particular limitations on the method used for applying the plant antiviral agent of the present invention, and the application method may be selected appropriately in accordance with the properties of the various components, and the variety of plant that represents the application target. Examples of the application method include foliage treatment, dipping treatments, soil irrigation, seed sterilization and fuming treatments. The plant antiviral agent of the present invention can be used without any restrictions in terms of the form of cultivation, whether it be soil cultivation or hydroponic cultivation or the like. Furthermore, an excellent effect can be achieved even when the plant antiviral agent is used in special environments such as during meristem culture.

The amount of the plant antiviral agent of the present invention that is applied can be determined appropriately in accordance with factors such as the atmospheric conditions, the formulation, the application season, the application method, the application location, the disease being targeted, and the crop being targeted.

There are no particular limitations on the types of plants to which the plant antiviral agent of the present invention can be applied, and both edible plants and non-edible plants may be targeted. Examples include grains such as rice, wheat and maize, beans such as soybeans, azuki beans and peanuts, fruits such as citrus fruits, apples, pears, grapes and peaches, vegetables such as tomatoes, lettuces, cabbages, onions, spring onions and capsicums, cucurbits such as cucumbers, watermelons, melons and pumpkins, root vegetables such as potatoes, sweet potatoes, yams, carrots and turnips, crops for processing such as cotton, sugar beets, hops, sugarcane, rubber, coffee, tobacco and tea, pasture grasses such as rye grass, timothy-grass and orchard grass, and lawn grasses such as bentgrass and Manila grass.

There are no particular limitations on the plant virus targeted by the plant antiviral agent of the present invention. Examples of ideal target viruses include geminiviruses which have single-stranded DNA as the genome, cauliflower mosaic virus which has a double-stranded DNA as the genome, tobacco mosaic virus and tomato bushy stunt virus which have single-stranded RNA as the genome, and rice ragged stunt virus which has a double-stranded RNA as the genome.

### EXAMPLES

The present invention is described below in further detail using a series of examples, but the scope of the present invention is in no way limited by these examples.

### Example 1

### (Synthesis of (R)-3,4-bis(benzyloxy)-5-((S)-2,2-dimethyl-1,3-dioxolan-4-yl)furan-2(5H)-one (Compound 2-31))

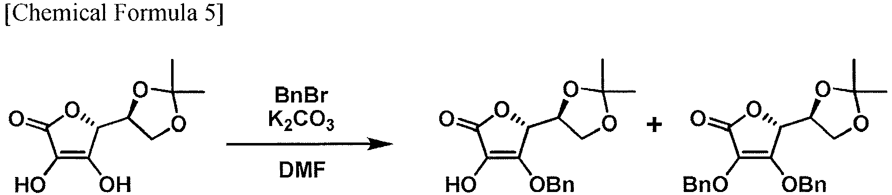

First, (R)-5-((S)-2,2-dimethyl-1,3-dioxolan-4-yl)-3,4-dihydroxyfuran-2(5H)-one (compound 2-21, 10.0 g) was dissolved in N,N-dimethylformamide (200 mL), and following the addition of potassium carbonate (6.40 g), the mixture was cooled to 0°C. Benzyl bromide (7.92 g) was then added gradually in a dropwise manner. Following completion of the dropwise addition, the temperature was gradually raised to room temperature, and the mixture was stirred overnight at room temperature. Subsequently, the reaction mixture was poured into water, and then extracted with diethyl ether. The organic phase was dried over anhydrous magnesium sulfate, filtered, and then concentrated under reduced pressure. The thus obtained concentrate was purified by silica gel column chromatography.

The product (R)-4-(benzyloxy)-5-((S)-2,2-dimethyl-1,3-dioxolan-4-yl)-3-hydroxyfuran-2(5H)-one was obtained in an amount of 8.80 g, a yield of 62%, and (R)-3,4-bis(benzyloxy)-5-((S)-2,2-dimethyl-1,3-dioxolan-4-yl)furan-2(5H)-one (compound 2-31) was obtained in an amount of 0.86 g, a yield of 4.7%.

### Example 2

### (Synthesis of (R)-4-(benzyloxy)-5-((S)-2,2-dimethyl-1,3-dioxolan-4-yl)furan-2(5H)-one (Compound 2-28))

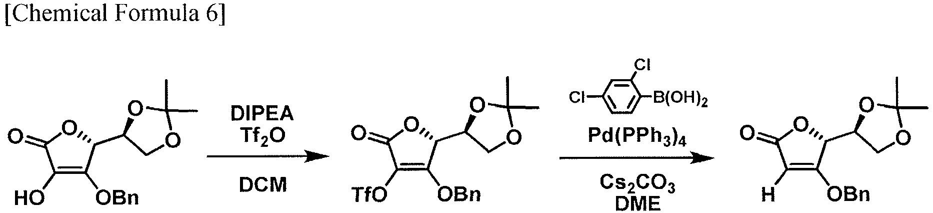

First, (R)-4-(benzyloxy)-5-((S)-2,2-dimethyl-1,3-dioxolan-4-yl)-3-hydroxyfuran-2(5H)-one (4.51 g) was dissolved in dichloromethane (45 mL), and the solution was cooled in an ice bath containing added salt. Diisopropylethylamine (2.85 g) and trifluoromethanesulfonic anhydride (4.99 g) were added to the solution, and the resulting mixture was stirred overnight while the temperature was gradually raised to room temperature. The reaction mixture was then poured into water, and extracted with dichloromethane. The organic phase was dried over anhydrous magnesium sulfate, filtered, and then concentrated under reduced pressure. The thus obtained concentrate was purified by silica gel column chromatography.
(R)-4-(benzyloxy)-5-((S)-2,2-dimethyl-1,3-dioxolan-4-yl)-2-oxo-2,5-dihydrofuran-3-yl trifluoromethanesulfonate was obtained in an amount of 1.10 g, a yield of 17%.

Next, under an atmosphere of nitrogen, (R)-4-(benzyloxy)-5-((S)-2,2-dimethyl-1,3-dioxolan-4-yl)-2-oxo-2,5-dihydrofuran-3-yl trifluoromethanesulfonate (508.7 mg), 2,4-dichlorophenylboric acid (0.29 g), tetrakis(triphenylphosphine)palladium (136.6 mg) and cesium carbonate (1.3 mL, 2 M aqueous solution) were dissolved in 1,2-dimethoxyethane, and the mixture was stirred overnight at 80°C. The reaction mixture was then poured into water, and extracted with ethyl acetate. The organic phase was dried over anhydrous magnesium sulfate, filtered, and then concentrated under reduced pressure. The thus obtained concentrate was purified by silica gel column chromatography.

The product (R)-4-(benzyloxy)-5-((S)-2,2-dimethyl-1,3-dioxolan-4-yl)furan-2(5H)-one (compound 2-28) was obtained in an amount of 181.9 mg, a yield of 54%.

### Example 3

### (Synthesis of (R)-4-(benzyloxy)-5-((S)-2,2-dimethyl-1,3-dioxolan-4-yl)-2-oxo-2,5-dihydrofuran-3-yl 4-toluenesulfonate (Compound 2-66))

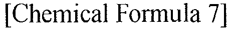

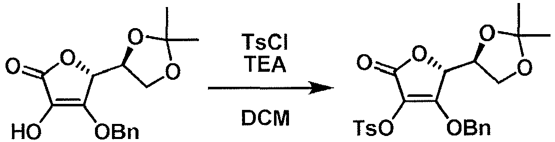

First, (R)-4-(benzyloxy)-5-((S)-2,2-dimethyl-1,3-dioxolan-4-yl)-3-hydroxyfuran-2(5H)-one (2.33 g) was dissolved in dichloromethane (23 mL), and the solution was cooled to 0°C. Triethylamine (1.85 g) and tosyl chloride (1.74 g) were added to the solution, and the resulting mixture was stirred overnight while the temperature was gradually raised to room temperature. The reaction mixture was then poured into water, and extracted with dichloromethane. The organic phase was dried over anhydrous magnesium sulfate, filtered, and then concentrated under reduced pressure. The thus obtained concentrate was purified by silica gel column chromatography.

The product (R)-4-(benzyloxy)-5-((S)-2,2-dimethyl-1,3-dioxolan-4-yl)-2-oxo-2,5-dihydroxyfuran-3-yl 4-toluenesulfonate (compound 2-66) was obtained in an amount of 1.92 g, a yield of 55%.

### Example 4

### (Synthesis of (R)-2-((S)-2,2-dimethyl-1,3-dioxolan-4-yl)-5-oxo-4-(tosyloxy)-2,5-dihydrofuran-3-yl pivalate (Compound 2-67))

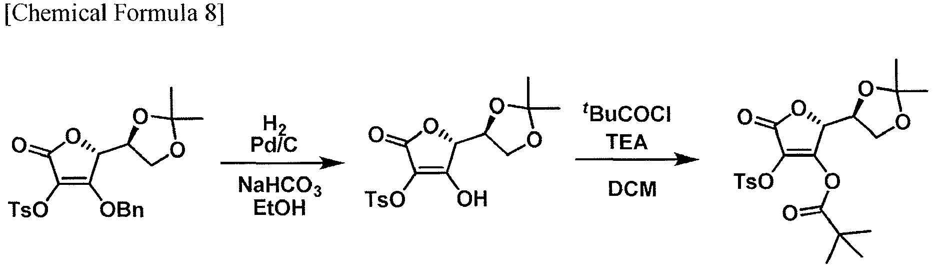

First, (R)-4-(benzyloxy)-5-((S)-2,2-dimethyl-1,3-dioxolan-4-yl)-2-oxo-2,5-dihydrofuran-3-yl 4-toluenesulfonate (1.13 g) was dissolved in ethanol, 10% palladium-carbon (116 mg) and sodium bicarbonate (17 mg) were added, a balloon filled with hydrogen gas was attached to the reaction vessel to substitute the atmosphere inside the system with hydrogen, and the resulting mixture was stirred at room temperature for 6 days. The reaction mixture was then filtered with celite, and the solid material retained on the celite was washed with ethyl acetate. The resulting filtrate was then concentrated under reduced pressure.

### (R)-5-((S)-2,2-dimethyl-1,3-dioxolan-4-yl)-4-hydroxy-2-oxo-2,5-dihydrofuran-3-yl 4-toluenesulfonate was obtained stoichiometrically in an amount of 975.2 mg.

This product (725.0 mg) was dissolved in tetrahydrofuran (20 mL), triethylamine (0.48 g) and pivaloyl chloride (0.28 g) were added to the solution at room temperature, and the resulting mixture was stirred overnight at room temperature. The reaction mixture was then poured into water, and extracted with ethyl acetate. The organic phase was dried over anhydrous magnesium sulfate, filtered, and then concentrated under reduced pressure. The thus obtained concentrate was purified by silica gel column chromatography.

The product (R)-2-((S)-2,2-dimethyl-1,3-dioxolan-4-yl)-5-oxo-4-(tosyloxy)-2,5-dihydrofuran-3-yl pivalate (compound 2-67) was obtained in an amount of 326.8 mg, a yield of 37%.

### Example 5

### (Synthesis of (R)-3-(4-chlorophenyl)-5-((S)-2,2-dimethyl-1,3-dioxolan-4-yl)-4-ethoxyfuran-2(5H)-one (Compound 2-43))

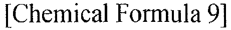

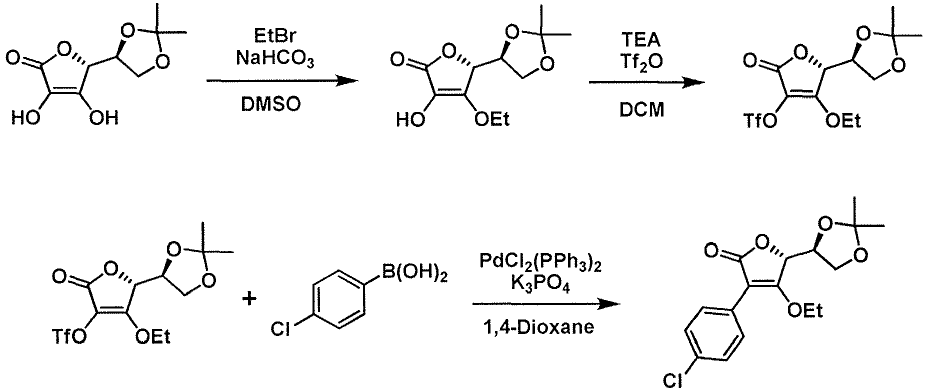

First, (R)-5-((S)-2,2-dimethyl-1,3-dioxolan-4-yl)-3,4-dihydroxyfuran-2(5H)-one (4.32 g) was dissolved in dimethyl sulfoxide (25 mL), sodium bicarbonate (2.52 g) was added to the solution at room temperature, and the resulting mixture was stirred for 20 minutes. Subsequently, ethyl bromide (3.27 g) was added. The temperature of the thus obtained reaction mixture was raised to 50°C, and the mixture was then stirred at that temperature for 17 hours. The reaction mixture was then cooled to room temperature, poured into water, acidified by addition of 1 N hydrochloric acid, and then extracted with ethyl acetate. The organic phase was dried over anhydrous magnesium sulfate, filtered, and then concentrated under reduced pressure. The thus obtained concentrate was purified by silica gel column chromatography.

### (R)-5-((S)-2,2-dimethyl-1,3-dioxolan-4-yl)-4-ethoxy-3-hydroxyfuran-2(5H)-one was obtained in an amount of 3.03 g, a yield of 62%.

Next, the thus obtained compound (1.01 g) was dissolved in dichloromethane (20 mL), and the solution was cooled to 0°C. A dichloromethane solution (1 mL) containing triethylamine (0.79 g) and a dichloromethane solution (4 mL) containing trifluoromethanesulfonic anhydride (1.40 g) were added and stirred for 10 minutes. Subsequently, the mixture was poured into water, and then extracted with dichloromethane. The organic phase was dried over anhydrous magnesium sulfate, filtered, and then concentrated under reduced pressure. The thus obtained concentrate was purified by silica gel column chromatography.

### (R)-5-((S)-2,2-dimethyl-1,3-dioxolan-4-yl)-4-ethoxy-2-oxo-2,5-dihydrofuran-3-yl trifluoromethanesulfonate was obtained in an amount of 1.52 g, a yield of 97%.

Subsequently, this triflate (302.0 mg), 4-chlorophenylboric acid (138.5 mg), dichlorobis(triphenylphosphine)palladium (28.7 mg) and potassium phosphate (272.6 mg) were dissolved in 1,4-dioxane (8 mL), and the resulting solution was heated overnight under reflux conditions and under a nitrogen atmosphere. Subsequently, the reaction mixture was cooled to room temperature, poured into a saturated aqueous solution of ammonium chloride, and then extracted with ethyl acetate. The organic phase was dried over anhydrous magnesium sulfate, filtered, and then concentrated under reduced pressure. The thus obtained concentrate was purified by silica gel column chromatography.

The product (R)-3-(4-chlorophenyl)-5-((S)-2,2-dimethyl-1,3-dioxolan-4-yl)-4-ethoxyfuran-2(5H)-one (compound 2-43) was obtained in an amount of 133.2 mg, a yield of 49%.

### Example 6

### (Synthesis of (R)-3-((4-chlorophenyl)(methyl)amino)-5-((S)-2,2-dimethyl-1,3-dioxolan-4-yl)-4-ethoxyfuran-2(5H)-one (Compound 2-61))

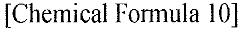

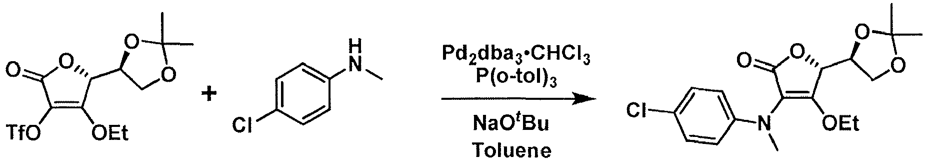

First, (R)-5-((S)-2,2-dimethyl-1,3-dioxolan-4-yl)-4-ethoxy-2-oxo-2,5-dihydrofuran-3-yl trifluoromethanesulfonate (409.0 mg) and 4-chloro-N-methylaniline (170.4 mg) were dissolved in toluene (11 mL), Pd₂bda₃·CHCl₃ (113.0 mg), tri(2-methylphenyl)phosphine (136.9 mg) and sodium tert-butoxide (147.6 mg) were added to the solution, the atmosphere inside the system was replaced with nitrogen, and the reaction mixture was heated overnight under reflux conditions. Subsequently, the reaction mixture was cooled to room temperature, poured into water, and then extracted with ethyl acetate. The organic phase was dried over anhydrous magnesium sulfate, filtered, and then concentrated under reduced pressure. The thus obtained concentrate was purified by silica gel column chromatography.

The product (R)-3-((4-chlorophenyl)(methyl)amino)-5-((S)-2,2-dimethyl-1,3-dioxolan-4-yl)-4-ethoxyfuran-2(5H)-one (compound 2-61) was obtained in an amount of 67.8 mg, a yield of 17%.

### Example 7

### (Synthesis of 4-(4-chlorophenyl)-5-(2,2-dimethyl-1,3-dioxolan-4-yl)-2-oxo-2,5-dihydrofuran-3-yl pivalate (Compound 2-51) and (4R)-4-(4-chlorophenyl)-5-((5S)-2,2-dimethyl-1,3-dioxolan-4-yl)-2-oxo-2,5-dihydrofuran-3-yl pivalate (Compound 2-47))

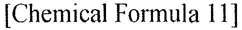

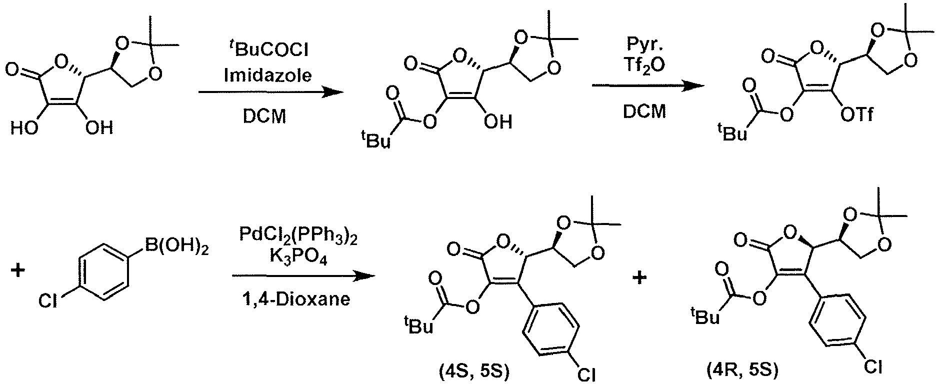

Imidazole was dissolved in dichloromethane (27 mL), and a dichloromethane solution (21 mL) containing pivaloyl chloride (1.37 g) was added dropwise at room temperature. The resulting mixture was then stirred at the same temperature for one hour. Subsequently, a dichloromethane solution (11 mL) containing (R)-5-((S)-2,2-dimethyl-1,3-dioxolan-4-yl)-3,4-dihydroxyfuran-2(5H)-one (2.17 g) was added, and the resulting mixture was stirred overnight at room temperature. Next, the reaction mixture was poured into 1 N hydrochloric acid, and then extracted with dichloromethane. The organic phase was dried over anhydrous magnesium sulfate, filtered, and then concentrated under reduced pressure. The thus obtained concentrate was purified by recrystallization using dichloromethane and hexane.

### (R)-5-((S)-2,2-dimethyl-1,3-dioxolan-4-yl)-4-hydroxy-2-oxo-2,5-dihydrofuran-3-yl pivalate (compound 2-46) was obtained in an amount of 2.84 g, a yield of 95%.

Next, 1.00 g of the thus obtained compound was dissolved in dichloromethane (17 mL), and the solution was cooled to 0°C. A dichloromethane solution (1 mL) containing pyridine (0.64 g) and a dichloromethane solution (4 mL) containing trifluoromethanesulfonic anhydride (1.13 g) were added and stirred for 15 minutes. Subsequently, the reaction mixture was poured into water, and then extracted with dichloromethane. The organic phase was dried over anhydrous magnesium sulfate, filtered, and then concentrated under reduced pressure. The thus obtained concentrate was purified by silica gel column chromatography.

As a result, (R)-5-((S)-2,2-dimethyl-1,3-dioxolan-4-yl)-2-oxo-4-(trifluoromethylsulfonyloxy)-2,5-dihydrofuran-3-yl pivalate was obtained in an amount of 1.36 g, a yield of 95%.

Subsequently, this triflate (473.7 mg), was dissolved in 1,4-dioxane (11 mL), 4-chlorophenylboric acid (190.0 mg), dichlorobis(triphenylphosphine)palladium (38.9 mg) and potassium phosphate (369.9 mg) were added to the solution, and the resulting mixture was heated overnight under reflux conditions and under a nitrogen atmosphere. Subsequently, the reaction mixture was cooled to room temperature, poured into water, and then extracted with ethyl acetate. The organic phase was dried over anhydrous magnesium sulfate, filtered, and then concentrated under reduced pressure. The thus obtained concentrate was purified by silica gel column chromatography.

The product (S)-4-(4-chlorophenyl)-5-((S)-2,2-dimethyl-1,3-dioxolan-4-yl)-2-oxo-2,5-dihydrofuran-3-yl pivalate (compound 2-51) was obtained in an amount of 103.9 mg, a yield of 24%, and (4R)-4-(4-chlorophenyl)-5-((5S)-2,2-dimethyl-1,3-dioxolan-4-yl)-2-oxo-2,5-dihydrofuran-3-yl pivalate (compound 2-47) was obtained in an amount of 115.2 mg, a yield of 27%.

### Example 8

### (Synthesis of 2-(3,4-bis(benzyloxy)-5-oxo-2,5-dihydrofuran-2-yl)ethyl palmitate (Compound 2-12))

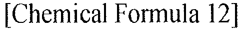

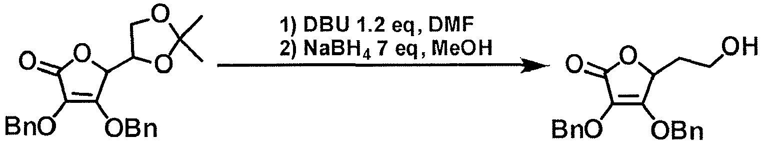

First, (2R)-3,4-dibenzyloxy-2-[(4S)-2,2-dimethyl-1,3-dioxolan-4-yl]-2H-furan-5-one (12.4 g, 31.3 mmol) was dissolved in N,N-dimethylformamide (50 mL). Subsequently, DBU (5.61 mL, 37.5 mmol) was added to the solution, and the resulting mixture was stirred overnight at 40°C. The reaction solution was added to water (500 mL), and 1 N hydrochloric acid was then added to adjust the pH to 2. This aqueous solution was extracted using a mixed solvent of ethyl acetate/methanol = 10/1 (220 mL). The organic phase was washed thoroughly with a saturated saline solution (30 mL), and was then dried over anhydrous magnesium sulfate. Subsequently, the mixture was filtered, and the filtrate was concentrated using a rotary evaporator. The concentrate was purified by flash chromatography, yielding 4.09 g of a crude product. This crude product was dissolved in methanol (40 mL), the solution was cooled to 0°C, and NaBH₄ (2.3 g, 60.8 mmol) was then added in 5 batches. After 2 hours had elapsed, the reaction solution was added to ice water (200 mL), and 1 N hydrochloric acid was then added to adjust the pH to 2. This mixture was extracted with chloroform (200 mL). The organic phase was washed thoroughly with a saturated saline solution (30 mL), and was then dried over anhydrous magnesium sulfate. The mixture was then filtered using a Kiriyama funnel, and the filtrate was concentrated.

As a result, 3,4-bis(benzyloxy)-5-(2-hydroxyethyl)furan-2(5H)-one was obtained in an amount of 2.39 g, a yield of 33%. 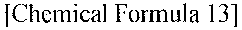 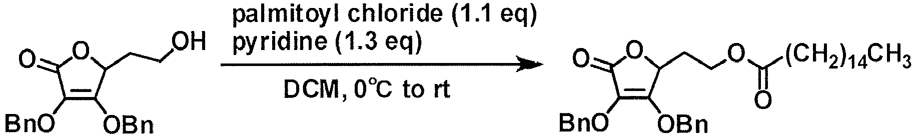 Subsequently, 3,4-bis(benzyloxy)-5-(2-hydroxyethyl)furan-2(5H)-one (1.1 g, 3.23 mmol) was dissolved in dichloromethane (30 mL), pyridine (339 µL, 4.20 mmol) was added, and the resulting mixture was cooled in an ice bath. Palmitoyl chloride (1.08 mL, 3.55 mmol) was then added dropwise, and the reaction mixture was stirred overnight. The reaction mixture was then washed sequentially with a 5% aqueous solution of potassium hydrogen sulfate (30 mL) and a saturated saline solution (30 mL), subsequently dried over anhydrous magnesium sulfate, and then filtered. The filtrate was concentrated, and the concentrate was then purified by flash chromatography.

The product 2-(3,4-bis(benzyloxy)-5-oxo-2,5-dihydrofuran-2-yl)ethyl palmitate (compound 2-12) was obtained in an amount of 1.59 g (yield: 85%).

### Example 9

### (Synthesis of 2-(3,4-dihydroxy-5-oxo-2,5-dihydrofuran-2-yl)ethyl palmitate (Compound 2-10))

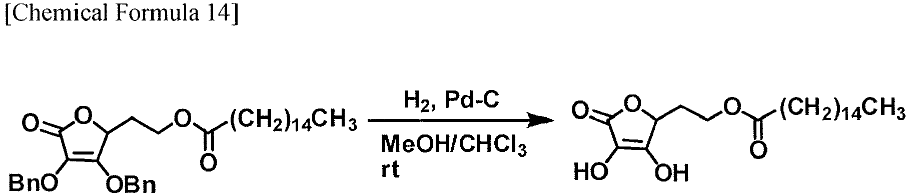

First, 2-(3,4-bis(benzyloxy)-5-oxo-2,5-dihydrofuran-2-yl)ethyl palmitate (1.59 g, 2.75 mmol) was dissolved in a mixed solvent of chloroform/methanol = 10/1 (33 mL), and the solution was then deaerated for 5 minutes. Subsequently, 0.5 g of 10% Pd-C was added to the reaction solution, and hydrogen gas was blown into the system while the solution was stirred at room temperature for 5 hours. The reaction mixture was then filtered with celite, the filtrate was concentrated, and the concentrate was purified by flash chromatography.

The product 2-(3,4-dihydroxy-5-oxo-2,5-dihydrofuran-2-yl)ethyl palmitate (compound 2-10) was obtained in an amount of 0.642 g, a yield of 59%.

### Example 10

### (Synthesis of (2R)-3,4-dibenzyloxy-2-[(1S)-1,2-dihydroxyethyl]-2H-furan-5-one (Compound 1-191))

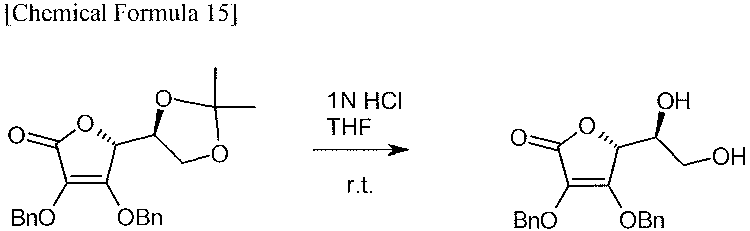

First, (2R)-3,4-dibenzyloxy-2-[(4S)-2,2-dimethyl-l,3-dioxolan-4-yl]-2H-furan-5-one (2.18 g) was dissolved in tetrahydrofuran (4 mL), and 1.5 N hydrochloric acid (4 mL) was then added to the solution at room temperature. The temperature of the reaction mixture was raised to 50°C, and the mixture was then stirred at the same temperature for 4 hours. Subsequently, the reaction mixture was cooled to room temperature, and then concentrated under reduced pressure. Water was added to the thus obtained concentrate, and the resulting mixture was extracted with ethyl acetate. The organic phase was dried over anhydrous magnesium sulfate, filtered, and then concentrated under reduced pressure. The thus obtained concentrate was purified by silica gel column chromatography.

The product (2R)-3,4-dibenzyloxy-2-[(1S)-1,2-dihydroxyethyl]-2H-furan-5-one (compound 1-191) was obtained in an amount of 1.43 g, a yield of 87%.

### Example 11

### (Synthesis of [(2S)-2-[(2R)-3,4-dibenzyloxy-5-oxo-2H-furan-2-yl]-2-hydroxy-ethyl] hexadecanoate (Compound 1-151), and [(2S)-2-[(2R)-3,4-dibenzyloxy-5-oxo-2H-furan-2-yl]-2-hexadecanoyloxy-ethyl] hexadecanoate (Compound 1-159))

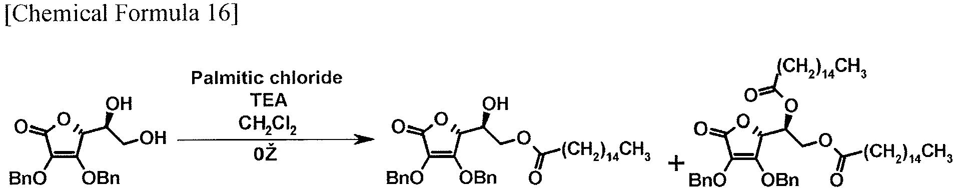

First, (2R)-3,4-dibenzyloxy-2-[(1S)-1,2-dihydroxyethyl]-2H-furan-5-one (1.33 g) was dissolved in dichloromethane (25 mL), and triethylamine (453 mg) and palmitoyl chloride (1.07 g) were then added to the solution at 0°C. The resulting mixture was then stirred at the same temperature for 4 hours. A saturated sodium bicarbonate solution was then added at the same temperature, and the mixture was extracted with chloroform. The organic phase was dried over anhydrous magnesium sulfate, filtered, and then concentrated under reduced pressure. The thus obtained concentrate was purified by silica gel column chromatography.

The product [(2S)-2-[(2R)-3,4-dibenzyloxy-5-oxo-2H-furan-2-yl]-2-hydroxy-ethyl] hexadecanoate (compound 1-151) was obtained in an amount of 1.48 g, a yield of 67%, and [(2S)-2-[(2R)-3,4-dibenzyloxy-5-oxo-2H-furran-2-yl]-2-hexadecanoyloxy-ethyl] hexadecanoate (compound 1-159) was obtained in an amount of 437 mg, a yield of 14%.

### Example 12

### (Synthesis of 4-[(2R)-3,4-dibenzyloxy-5-oxo-2H-furan-2-yl]-1,3-dioxolan-2-one (Compound 2-69))

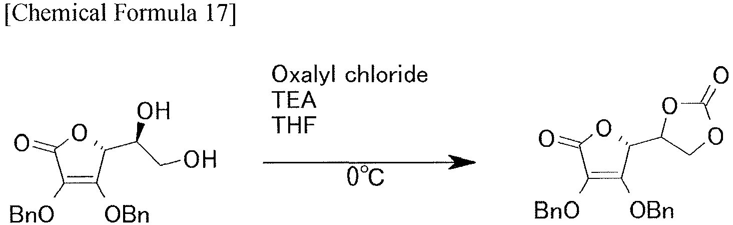

First, (2R)-3,4-dibenzyloxy-2-[(1S)-1,2-dihydroxyethyl]-2H-furan-5-one (100 mg) was dissolved in tetrahydrofuran (24 mL) and triethylamine (85 mL) and oxalyl chloride (40 mL) were then added to the solution at 0°C. The resulting mixture was stirred overnight at the same temperature, a saturated sodium bicarbonate solution was then added, and the mixture was extracted with ethyl acetate. The organic phase was dried over anhydrous magnesium sulfate, filtered, and then concentrated under reduced pressure. The thus obtained concentrate was purified by silica gel column chromatography.

The product 4-[(2R)-3,4-dibenzyloxy-5-oxo-2H-furan-2-yl]-1,3-dioxolan-2-one (compound 2-69) was obtained in an amount of 30 mg, a yield of 28%.

### Example 13

### (Synthesis of (2R)-3,4-dibenzyloxy-2-[(1S)-2-[tert-butyl(dimethyl)silyl]oxy-1-hydroxy-ethyl]-2H-furan-5-one (Compound 1-160))

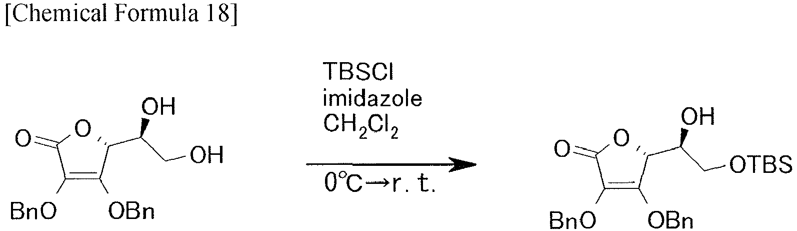

First, (2R)-3,4-dibenzyloxy-2-[(1S)-1,2-dihydroxyethyl]-2H-furan-5-one (2 g) was dissolved in dichloromethane (30 mL), imidazole (763 mg) and TBSCl (888 mg) were added to the solution at 0°C, and the resulting mixture was stirred at the same temperature for one hour. The temperature of the reaction mixture was then gradually raised to room temperature, and the mixture was then stirred at room temperature for 3 hours. Water was then added to the reaction mixture, and the mixture was extracted with chloroform. The organic phase was dried over anhydrous magnesium sulfate, filtered, and then concentrated under reduced pressure. The thus obtained concentrate was purified by silica gel column chromatography.

The product (2R)-3,4-dibenzyloxy-2-[(1S)-2-[tert-butyl(dimethyl)silyl]oxy-1-hydroxy-ethyl]-2H-furan-5-one (compound 1-160) was obtained in an amount of 2.03 g, a yield of 77%.

### Example 14

### (Synthesis of [(2S)-2-[(2R)-3,4-dibenzyloxy-5-oxo-2H-furan-2-yl]-2-ethoxy-ethyl] hexadecanoate (Compound 1-153)

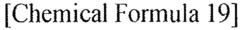

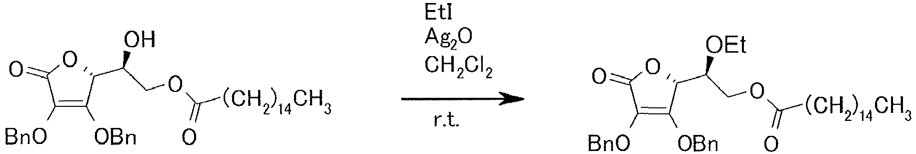

First, [(2S)-2-[(2R)-3,4-dibenzyloxy-5-oxo-2H-furan-2-yl]-2-hydroxy-ethyl] hexadecanoate (150 mg) was dissolved in dichloromethane, silver oxide (117 mg) and ethyl iodide (80 mg) were then added to the solution at room temperature, and the resulting mixture was stirred at the same temperature for one week. Water was then added to the reaction mixture, the mixture was filtered, and the filtrate was extracted with chloroform. The organic phase was dried over anhydrous magnesium sulfate, filtered, and then concentrated under reduced pressure. The thus obtained concentrate was purified by silica gel column chromatography.

The product [(2S)-2-[(2R)-3,4-dibenzyloxy-5-oxo-2H-furan-2-yl]-2-ethoxy-ethyl] hexadecanoate (compound 1-153) was obtained in an amount of 10 mg, a yield of 6.4%.

### Example 15

### (Synthesis of [(1S)-2-[tert-butyl(dimethyl)silyl]oxy-1-[(2R)-3,4-dibenzyloxy-5-oxo-2H-furan-2-yl]ethyl] hexadecanoate (Compound 1-161))

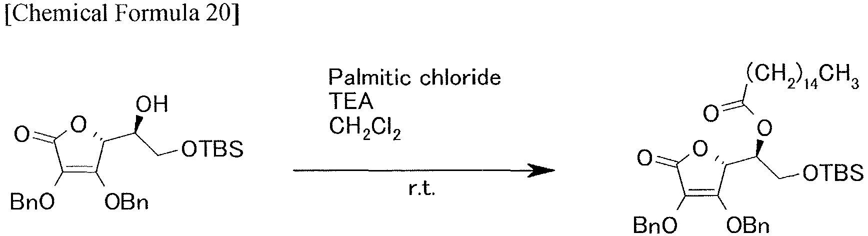

First, (2R)-3,4-dibenzyloxy-2-[(1S)-2-[tert-butyl(dimethyl)silyl]oxy-1-hydroxy-ethyl]-2H-furan-5-one (1 g) was dissolved in dichloromethane (15 mL), and triethylamine (536 mg) and palmitoyl chloride (700 mg) were then added to the solution at 0°C. The temperature of the reaction mixture was then gradually raised to room temperature, and the mixture was stirred overnight at the same temperature. A saturated sodium bicarbonate solution was added at room temperature, and the mixture was extracted with chloroform. The organic phase was dried over anhydrous magnesium sulfate, filtered, and then concentrated under reduced pressure. The thus obtained concentrate was purified by silica gel column chromatography.

The product [(1S)-2-[tert-butyl(dimethyl)silyl]oxy-1-[(2R)-3,4-dibenzyloxy-5-oxo-2H-furan-2-yl]ethyl] hexadecanoate (compound 1-161) was obtained in an amount of 1.16 g, a yield of 77%.

### Example 16

### (Synthesis of (2S)-3,4-dibenzyloxy-2-[(1R)-2-[tert-butyl(dimethyl)silyl]oxy-1-iodo-ethyl]-2H-furan-5-one (Compound 1-162))

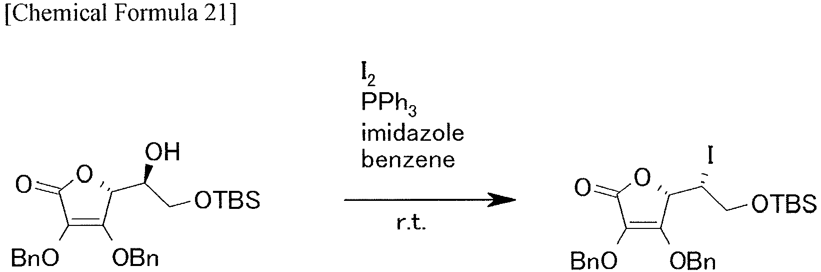

First, (2R)-3,4-dibenzyloxy-2-[(1S)-2-[tert-butyl(dimethyl)silyl]oxy-1-hydroxy-ethyl]-2H-furan-5-one (100 mg) was dissolved in benzene (3 mL), triphenylphosphine (139 mg), imidazole (36 mg) and iodine (107 mg) were then added to the solution at room temperature, and the resulting mixture was stirred at the same temperature for 4 hours. Subsequently, water was added to the reaction mixture, and the resulting mixture was extracted with ethyl acetate. The organic phase was dried over anhydrous magnesium sulfate, filtered, and then concentrated under reduced pressure. The thus obtained concentrate was purified by silica gel column chromatography.

The product (2S)-3,4-dibenzyloxy-2-[(1R)-2-[tert-butyl(dimethyl)silyl]oxy-1-iodo-ethyl]-2H-furan-5-one (compound 1-162) was obtained in an amount of 63 mg, a yield of 51%.

### Example 17

### (Synthesis of (5Z)-3,4-dibenzyloxy-5-[2-[tert-butyl(dimethyl)silyl]oxyethylidene]furan-2-one (Compound 3-19))

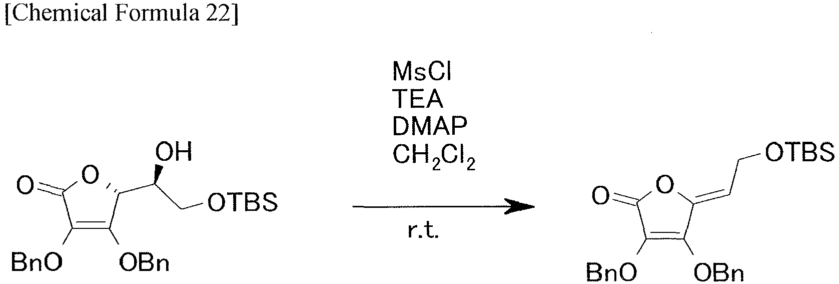

First, (2R)-3,4-dibenzyloxy-2-[(1S)-2-[tert-butyl(dimethyl)silyl]oxy-1-hydroxy-ethyl]-2H-furan-5-one (940 mg) was dissolved in dichloromethane (10 mL), and triethylamine (1 g), mesyl chloride (342 mg) and N,N-dimethyl-4-aminopyridine (24 mg) were then added to the solution at 0°C. The temperature of the reaction mixture was then gradually raised to room temperature, and the mixture was stirred overnight at the same temperature. A saturated sodium bicarbonate solution was added at room temperature, and the mixture was extracted with chloroform. The organic phase was dried over anhydrous magnesium sulfate, filtered, and then concentrated under reduced pressure. The thus obtained concentrate was purified by silica gel column chromatography.

The product (5Z)-3,4-dibenzyloxy-5-[2-[tert-butyl(dimethyl)silyl]oxyethylidene]-furan-2-one (compound 3-19) was obtained in an amount of 700 mg, a yield of 78%.

### Example 18

### (Synthesis of [(2R)-3-benzyloxy-2-[(4S)-2,2-dimethyl-1,3-dioxolan-4-yl]-5-oxo-2H-furan-4-yl] 2,2-dimethylpropanoate (Compound 2-65))

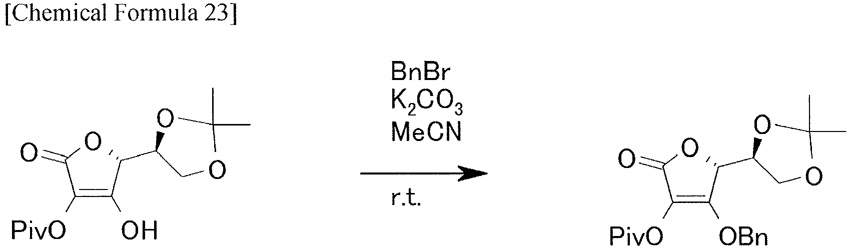

First, [(2R)-2-[(4S)-2,2-dimethyl-1,3-dioxolan-4-yl]-3-hydroxy-5-oxo-2H-furan-4-yl] 2,2-dimethylpropanoate (1.87 g) was dissolved in acetonitrile (20 mL), and potassium carbonate (2.58 g) and benzyl bromide (1.6 g) were added to the solution at room temperature. The reaction mixture was then stirred overnight at the same temperature. Subsequently, water was added to the reaction mixture, and the mixture was then extracted with ethyl acetate. The organic phase was dried over anhydrous magnesium sulfate, filtered, and then concentrated under reduced pressure. The thus obtained concentrate was purified by silica gel column chromatography.

The product [(2R)-3-benzyloxy-2-[(4S)-2,2-dimethyl-1,3-dioxolan-4-yl]-5-oxo-2H-furan-4-yl] 2,2-dimethylpropanoate (compound 2-65) was obtained in an amount of 1.902 g, a yield of 77%.

### Example 19

### (Synthesis of [(2R)-3-benzyloxy-2-[(1S)-1,2-dihydroxyethyl]-5-oxo-2H-furan-4-yl] 2,2-dimethylpropanoate (Compound 1-42))

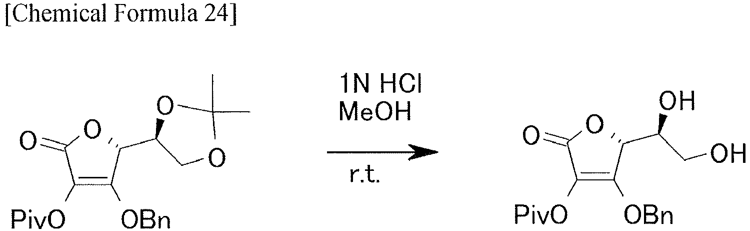

First, [(2R)-3-benzyloxy-2-[(4S)-2,2-dimethyl-1,3-dioxolan-4-yl]-5-oxo-2H-furan-4-yl] 2,2-dimethylpropanoate (1.92 g) was dissolved in methanol (40 mL), IN hydrochloric acid (4 mL) was then added to the solution at room temperature, and the mixture was stirred overnight at the same temperature. The reaction mixture was then concentrated under reduced pressure, water was added to the resulting concentrate, and the mixture was extracted with chloroform. The organic phase was dried over anhydrous magnesium sulfate, filtered, and then concentrated under reduced pressure. The thus obtained concentrate was purified by silica gel column chromatography.

The product [(2R)-3-benzyloxy-2-[(1S)-1,2-dihydroxyethyl]-5-oxo-2H-furan-4-yl] 2,2-dimethylpropanoate (compound 1-42) was obtained in an amount of 1.46 g, a yield of 86%.

### Example 20

### (Synthesis of [(2S)-2-[(2R}-3-benzyloxy-4-(2,2-dimethylpropanoyloxy)-5-oxo-2H-furan-2-yl]-2-hydroxy-ethyl] hexadecanoate (Compound 1-109), and [(2S)-2-[(2R)-3-benzyloxy-4-(2,2-dimethylpropanoyloxy)-5-oxo-2H-furan-2-yl]-2-hexadecanoyloxy-ethyl] hexadecanoate (Compound 1-158))

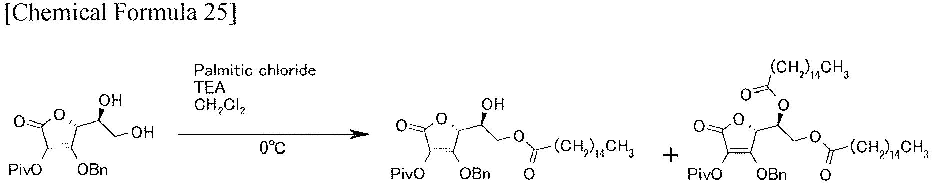

First, [(2R)-3-benzyloxy-2-[(1S)-1,2-dihydroxyethyl]-5-oxo-2H-furan-4-yl] 2,2-dimethylpropanoate (1.46 g) was dissolved in dichloromethane (30 mL), and triethylamine (508 mg) and palmitoyl chloride (1.2 g) were then added to the solution at 0°C. The resulting mixture was then stirred at the same temperature for 4 hours. A saturated sodium bicarbonate solution was then added at the same temperature, and the mixture was extracted with chloroform. The organic phase was dried over anhydrous magnesium sulfate, filtered, and then concentrated under reduced pressure. The thus obtained concentrate was purified by silica gel column chromatography.

The product [(2S)-2-[(2R)-3-benzyloxy-4-(2,2-dimethylpropanoyloxy)-5-oxo-2H-furan-2-yl]-2-hydroxy-ethyl] hexadecanoate (compound 1-109) was obtained in an amount of 1.39 g, a yield of 57%, and [(2S)-2-[(2R)-3-benzyloxy-4-(2,2-dimethylpropanoyloxy)-5-oxo-2H-furan-2-yl]-2-hexadecanoyloxy-ethyl] hexadecanoate (compound 1-158) was obtained in an amount of 512 mg, a yield of 15%.

### Example 21

### (Synthesis of [(2S)-2-acetoxy-2-[(2R)-3-benzyloxy-4-(2,2-dimethylpropanoyloxy)-5-oxo-2H-furan-2-yl]ethyl] hexadecanoate (Compound 1-157))

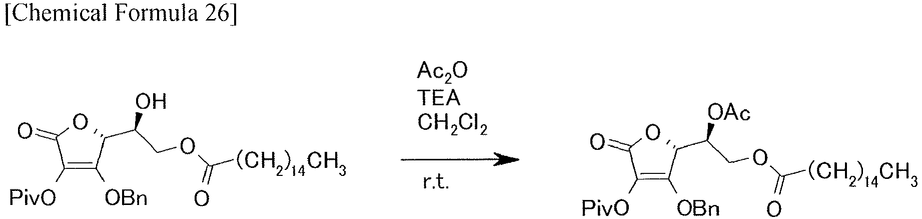

First, [(2S)-2-[(2R)-3-benzyloxy-4-(2,2-dimethylpropanoyloxy)-5-oxo-2H-furan-2-yl]-2-hydroxy-ethyl] hexadecanoate (200 mg) was dissolved in dichloromethane (4 mL), and triethylamine (103 mg) and acetic anhydride (342 mg) were added to the solution at 0°C. The temperature of the reaction mixture was then gradually raised to room temperature, and the mixture was then stirred overnight at the same temperature. A saturated sodium bicarbonate solution was then added at the same temperature, and the mixture was extracted with chloroform. The organic phase was dried over anhydrous magnesium sulfate, filtered, and then concentrated under reduced pressure. The thus obtained concentrate was purified by silica gel column chromatography.

The product [(2S)-2-acetoxy-2-[(2R)-3-benzyloxy-4-(2,2-dimethylpropanoyloxy)-5-oxo-2H-furan-2-yl]ethyl] hexadecanoate (compound 1-157) was obtained in an amount of 66 mg, a yield of 31%.

### Example 22

### (Synthesis of [(2S)-2-[(2R)-4-(2,2-dimethylpropanoyloxy)-3-hydroxy-5-oxo-2H-furan-2-yl]-2-hydroxy-ethyl] hexadecanoate (Compound 1-148))

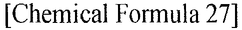

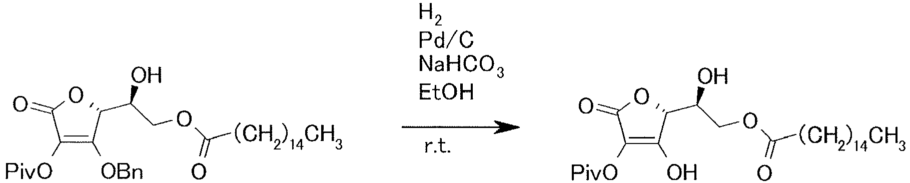

First, [(2S)-2-[(2R)-3-benzyloxy-4-(2,2-dimethylpropanoyloxy)-5-oxo-2H-furan-2-yl]-2-hydroxy-ethyl] hexadecanoate (1 g) was dissolved in ethanol (15 mL), palladium-carbon (100 mg) and sodium bicarbonate (11 mg) were added, a balloon filled with hydrogen gas was attached to the reaction vessel to substitute the atmosphere inside the system with hydrogen, and the resulting mixture was stirred at room temperature for 6 hours. The reaction mixture was then filtered with celite, and the solid material retained on the celite was washed with ethyl acetate. The resulting filtrate was then concentrated under reduced pressure.

The product [(2S)-2-[(2R)-4-(2,2-dimethylpropanoyloxy)-3-hydroxy-5-oxo-2H-furan-2-yl]-2-hydroxy-ethyl] hexadecanoate (compound 1-148) was obtained in an amount of 540 mg, a yield of 64%.

### Example 23

### (Synthesis of [(2S)-2-[(2R)-4-(2,2-dimethylpropanoyloxy)-3-hydroxy-5-oxo-2H-furan-2-yl]-2-hexadecanoyloxy-ethyl] hexadecanoate (Compound 1-155))

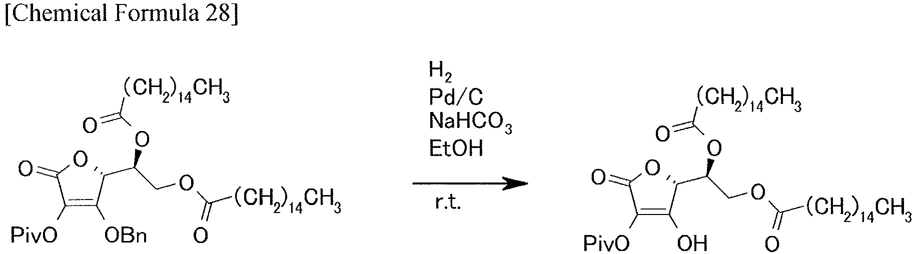

First, [(2S)-2-[(2R)-3-benzyloxy-4-(2,2-dimethylpropanoyloxy)-5-oxo-2H-furan-2-yl]-2-hexadecanoyloxy-ethyl] hexadecanoate (512 mg) was dissolved in ethanol (8 mL), palladium-carbon (51 mg) and sodium bicarbonate (1 mg) were added, a balloon filled with hydrogen gas was attached to the reaction vessel to substitute the atmosphere inside the system with hydrogen, and the resulting mixture was stirred at room temperature for 6 hours. The reaction mixture was then filtered with celite, and the solid material retained on the celite was washed with ethyl acetate. The resulting filtrate was then concentrated under reduced pressure.

The product [(2S)-2-[(2R)-4-(2,2-dimethylpropanoyloxy)-3-hydroxy-5-oxo-2H-furan-2-yl]-2-hexadecanoyloxy-ethyl] hexadecanoate (compound 1-155) was obtained in an amount of 450 mg, a yield of 99%.

### Example 24

### (Synthesis of [(2S)-2-acetoxy-2-[(2R)-4-(2,2-dimethylpropanoyloxy)-3-hydroxy-5-oxo-2H-furan-2-yl]ethyl] hexadecanoate (Compound 1-149))

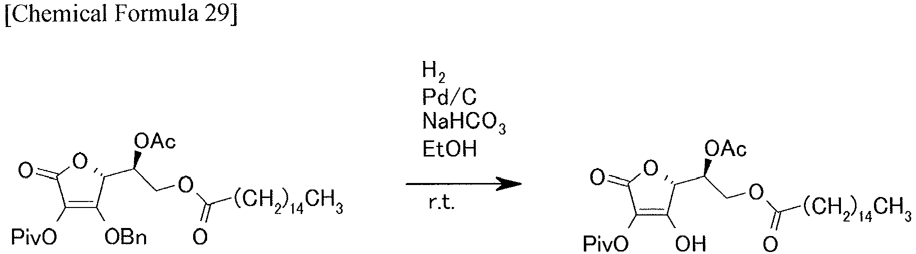

First, [(2S)-2-acetoxy-2-[(2R)-3-benzyloxy-4-(2,2-dimethylpropanoyloxy)-5-oxo-2H-furan-2-yl]ethyl] hexadecanoate (60 mg) was dissolved in ethanol (3 mL), palladium-carbon (6 mg) and sodium bicarbonate (1 mg) were added, a balloon filled with hydrogen gas was attached to the reaction vessel to substitute the atmosphere inside the system with hydrogen, and the resulting mixture was stirred at room temperature for 6 hours. The reaction mixture was then filtered with celite, and the solid material retained on the celite was washed with ethyl acetate. The resulting filtrate was then concentrated under reduced pressure.

The product [(2S)-2-acetoxy-2-[(2R)-4-(2,2-dimethylpropanoyloxy)-3-hydroxy-5-oxo-2H-furan-2-yl]ethyl] hexadecanoate (compound 1-149) was obtained in an amount of 50 mg, a yield of 98%.

### Example 25

### (Synthesis of [(2S)-2-acetoxy-2-[(2R)-3,4-dibenzyloxy-5-oxo-2H-furan-2-yl]ethyl] hexadecanoate (Compound 1-152))

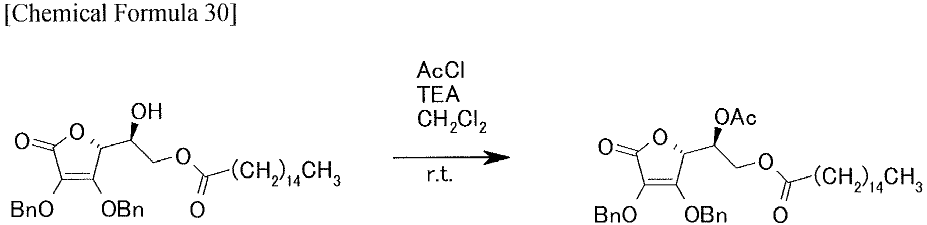

First, [(2S)-2-[(2R)-3,4-dibenzyloxy-5-oxo-2H-furan-2-yl]-2-hydroxy-ethyl] hexadecanoate (850 mg) was dissolved in dichloromethane (10 mL), and triethylamine (434 mg) and acetyl chloride (168 mg) were added to the solution at 0°C. The temperature of the reaction mixture was then gradually raised to room temperature, and the mixture was then stirred overnight at the same temperature. A saturated sodium bicarbonate solution was then added to the reaction mixture at room temperature, and the mixture was extracted with chloroform. The organic phase was dried over anhydrous magnesium sulfate, filtered, and then concentrated under reduced pressure. The thus obtained concentrate was purified by silica gel column chromatography.

The product [(2S)-2-acetoxy-2-[(2R)-3,4-dibenzyloxy-5-oxo-2H-furan-2-yl]ethyl] hexadecanoate (compound 1-152) was obtained in an amount of 504 mg, a yield of 55%.

### Example 26

### (Synthesis of [(1S)-1-[(2R)-3,4-dibenzyloxy-5-oxo-2H-furan-2-yl]-2-hydroxyethyl] hexadecanoate (Compound 1-150))

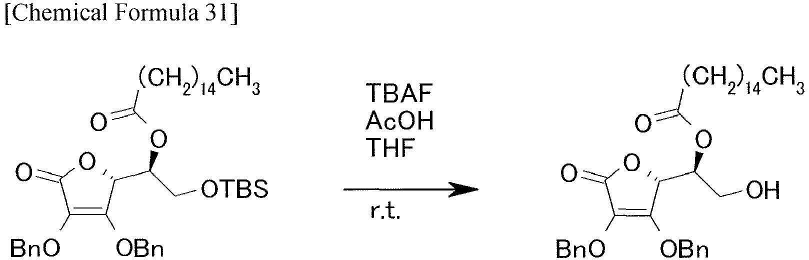

First, [(1S)-2-[tert-butyl(dimethyl)silyl]oxy-1-[(2R)-3,4-dibenzyloxy-5-oxo-2H-furan-2-yl]ethyl] hexadecanoate (300 mg) was dissolved in tetrahydrofuran (5 mL), and a tetrahydrofuran solution (635 µL) containing acetic acid (38 mg) and TBAF was then added at 0°C. The temperature of the reaction mixture was then gradually raised to room temperature, and the mixture was then stirred at the same temperature for 5 hours. Water was then added to the reaction mixture at room temperature, and the mixture was extracted with ethyl acetate. The organic phase was dried over anhydrous magnesium sulfate, filtered, and then concentrated under reduced pressure. The thus obtained concentrate was purified by silica gel column chromatography.

The product [(1S)-1-[(2R)-3,4-dibenzyloxy-5-oxo-2H-furan-2-yl]-2-hydroxyethyl] hexadecanoate (compound 1-150) was obtained in an amount of 154 mg, a yield of 61%.

### Example 27

### (Synthesis of [(2S)-2-[(2R)-3,4-dibenzyloxy-5-oxo-2H-furan-2-yl]-2-(2,2-dimethylpropanoyloxy)ethyl] hexadecanoate (Compound 1-154))

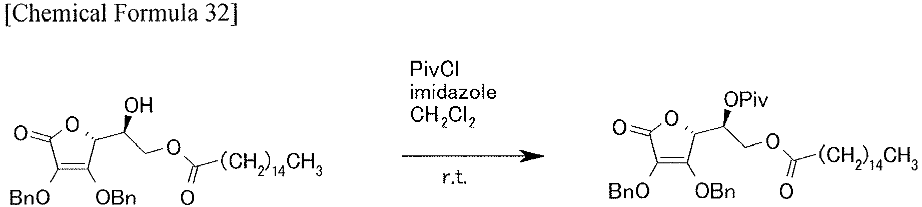

Imidazole was dissolved in dichloromethane (3.5 mL), and a dichloromethane solution (1.5 mL) of pivaloyl chloride (529 mg) was then added dropwise at room temperature. The resulting mixture was then stirred at the same temperature for one hour. Subsequently, a dichloromethane solution (5 mL) containing [(2S)-2-[(2R)-3,4-dibenzyloxy-5-oxo-2H-furan-2-yl]-2-hydroxy-ethyl] hexadecanoate (870 mg) was added, and the mixture was stirred at room temperature for 4 hours. Water was then added to the reaction mixture at room temperature, and the mixture was extracted with chloroform. The organic phase was dried over anhydrous magnesium sulfate, filtered, and then concentrated under reduced pressure. The thus obtained concentrate was purified by silica gel column chromatography.

The product [(2S)-2-[(2R)-3,4-dibenzyloxy-5-oxo-2H-furan-2-yl]-2-(2,2-dimethylpropanoyloxy)ethyl] hexadecanoate (compound 1-154) was obtained in an amount of 580 mg, a yield of 59%.

### Example 28

### (Synthesis of [(2S)-2-acetoxy-2-[(2R)-3,4-dihydroxy-5-oxo-2H-furan-2-yl]ethyl] hexadecanoate (Compound 1-144))

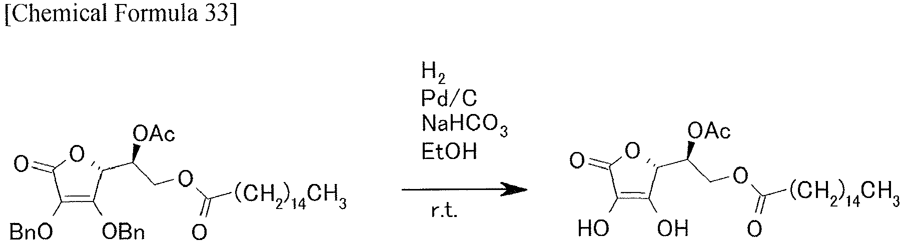

First, [(2S)-2-acetoxy-2-[(2R)-3,4-dibenzyloxy-5-oxo-2H-furan-2-yl]ethyl] hexadecanoate (compound 1-152, 505 mg) was dissolved in ethanol (10 mL), palladium-carbon (50 mg) and sodium bicarbonate (6 mg) were added, a balloon filled with hydrogen gas was attached to the reaction vessel to substitute the atmosphere inside the system with hydrogen, and the resulting mixture was stirred overnight at room temperature. The reaction mixture was then filtered with celite, and the solid material retained on the celite was washed with ethyl acetate. The resulting filtrate was then concentrated under reduced pressure.

The product [(2S)-2-acetoxy-2-[(2R)-3,4-dihydroxy-5-oxo-2H-furan-2-yl]ethyl] hexadecanoate (compound 1-144) was obtained stoichiometrically in an amount of 362 mg.

### Example 29

### (Synthesis of [(2S)-2-[(2R)-3,4-dihydroxy-5-oxo-2H-furan-2-yl]-2-(2,2-dimethylpropanoyloxy)ethyl] hexadecanoate (Compound 1-145))

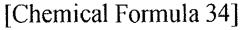

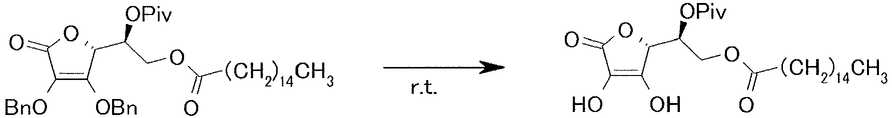

First, [(2S)-2-[(2R)-3,4-dibenzyloxy-5-oxo-2H-furan-2-yl]-2-(2,2-dimethylpropanoyloxy)ethyl] hexadecanoate (580 mg) was dissolved in ethanol (15 mL), palladium-carbon (58 mg) and sodium bicarbonate (7 mg) were added, a balloon filled with hydrogen gas was attached to the reaction vessel to substitute the atmosphere inside the system with hydrogen, and the resulting mixture was stirred overnight at room temperature. The reaction mixture was then filtered with celite, and the solid material retained on the celite was washed with ethyl acetate. The resulting filtrate was then concentrated under reduced pressure.

The product [(2S)-2-[(2R)-3,4-dihydroxy-5-oxo-2H-furan-2-yl]-2-(2,2-dimethylpropanoyloxy)ethyl] hexadecanoate (compound 1-145) was obtained in an amount of 42 mg, a yield of 9.9%.

### Example 30

### (Synthesis of [(2R)-3-benzyloxy-2-[(4S)-2,2-dimethyl-1,3-dioxolan-4-yl]-5-oxo-2H-furan-4-yl] hexadecanoate (Compound 2-27))

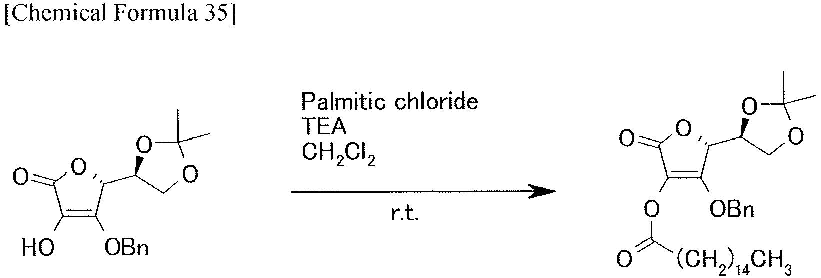

First, (R)-4-(benzyloxy)-5-((S)-2,2-dimethyl-1,3-dioxolan-4-yl)-3-hydroxyfuran-2(5H)-one (compound 2-26, 1.5 g) was dissolved in dichloromethane (20 mL), and triethylamine (1.49 g) and palmitoyl chloride (2.02 g) were then added to the solution at 0°C. The temperature of the reaction mixture was then gradually raised to room temperature, and the mixture was then stirred for 4 hours. A saturated sodium bicarbonate solution was then added at the same temperature, and the mixture was extracted with chloroform. The organic phase was dried over anhydrous magnesium sulfate, filtered, and then concentrated under reduced pressure. The thus obtained concentrate was purified by silica gel column chromatography.

The product [(2R)-3-benzyloxy-2-[(4S)-2,2-dimethyl-1,3-dioxolan-4-yl]-5-oxo-2H-furan-4-yl] hexadecanoate (compound 2-27) was obtained in an amount of 2.23 g, a yield of 84%.

### Example 31

### (Synthesis of [(2R)-3-benzyloxy-2-[(4S)-2,2-dimethyl-1,3-dioxolan-4-yl]-5-oxo-2H-furan-4-yl] acetate (Compound 2-23))

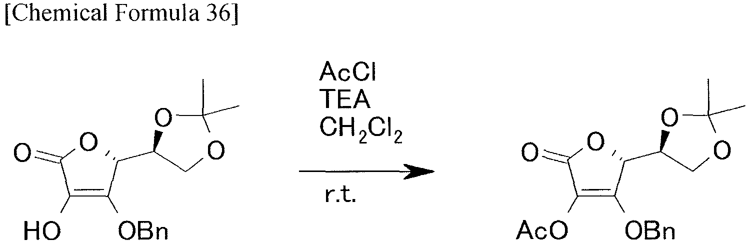

First, (R)-4-(benzyloxy)-5-((S)-2,2-dimethyl-1,3-dioxolan-4-yl)-3-hydroxyfuran-2(5H)-one (1.5 g) was dissolved in dichloromethane (25 mL), and triethylamine (1.49 g) and acetyl chloride (577 mg) were then added to the solution at 0°C. The temperature of the reaction mixture was then gradually raised to room temperature, and the mixture was then stirred for 4 hours. A saturated sodium bicarbonate solution was then added at the same temperature, and the mixture was extracted with chloroform. The organic phase was dried over anhydrous magnesium sulfate, filtered, and then concentrated under reduced pressure. The thus obtained concentrate was purified by silica gel column chromatography.

The product [(2R)-3-benzyloxy-2-[(4S)-2,2-dimethyl-1,3-dioxolan-4-yl]-5-oxo-2H-furan-4-yl] acetate (compound 2-23) was obtained in an amount of 887 mg, a yield of 52%.

### Example 32

### (Synthesis of [(2R)-3-benzyloxy-2-[(1S)-1,2-dihydroxyethyl]-5-oxo-2H-furan-4-yl] hexadecanoate (Compound 1-105) and (2R)-3-benzyloxy-2-[(1S)-1,2-dihydroxyethyl]-4-hydroxy-2H-furan-5-one (Compound 1-2))

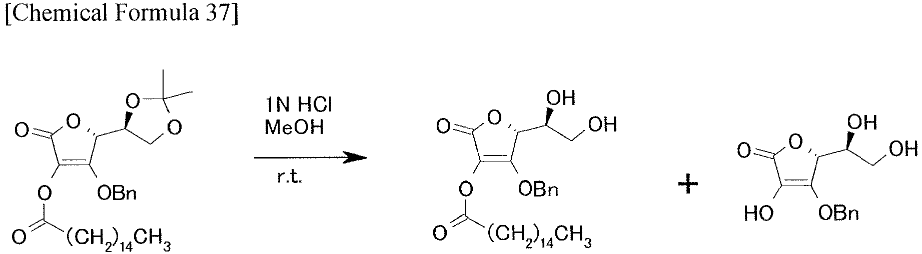

First, [(2R)-3-benzyloxy-2-[(4S)-2,2-dimethyl-1,3-dioxolan-4-yl]-5-oxo-2H-furan-4-yl] hexadecanoate (2.23 g) was dissolved in methanol (40 mL), 1 N hydrochloric acid (4 mL) was added to the solution at room temperature, and the resulting mixture was stirred overnight at the same temperature. The reaction mixture was then concentrated under reduced pressure, water was added to the concentrate, and the resulting mixture was extracted with ethyl acetate. The organic phase was dried over anhydrous magnesium sulfate, filtered, and then concentrated under reduced pressure. The thus obtained concentrate was purified by silica gel column chromatography.

The product [(2R)-3-benzyloxy-2-[(IS)-1,2-dihydroxyethyl]-5-oxo-2H-furan-4-yl] hexadecanoate (compound 1-105) was obtained in an amount of 826 mg, a yield of 40%, and (2R)-3-benzyloxy-2-[(1S)-1,2-dihydroxyethyl]-4-hydroxy-2H-furan-5-one (compound 1-2) was obtained in an amount of 268 mg, a yield of 25%.

### Example 33

### (Synthesis of [(2R)-2-[(4S)-2,2-dimethyl-1,3-dioxolan-4-yl]-3 -hydroxy-5-oxo-2H-furan-4-yl] acetate (Compound 2-22))

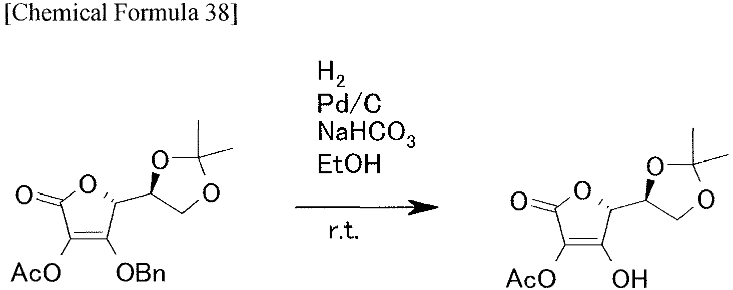

First, [(2R)-3-benzyloxy-2-[(4S)-2,2-dimethyl-1,3-dioxolan-4-yl]-5-oxo-2H-furan-4-yl] acetate (887 mg) was dissolved in ethanol (15 mL), palladium-carbon (90 mg) and sodium bicarbonate (21 mg) were added, a balloon filled with hydrogen gas was attached to the reaction vessel to substitute the atmosphere inside the system with hydrogen, and the resulting mixture was stirred at room temperature for 6 hours. The reaction mixture was then filtered with celite, and the solid material retained on the celite was washed with ethyl acetate. The resulting filtrate was then concentrated under reduced pressure.

The product [(2R)-2-[(4S)-2,2-dimethyl-1,3-dioxolan-4-yl]-3 -hydroxy-5-oxo-2H-furan-4-yl] acetate (compound 2-22) was obtained in an amount of 650 mg, a yield of 99%.

### Example 34

### (Synthesis of (2R)-3-benzyloxy-4-[tert-butyl(dimethyl)silyl]oxy-2-[(4S)-2,2-dimethyl-1,3-dioxolan-4-yl]-2H-furan-5-one (Compound 2-33))

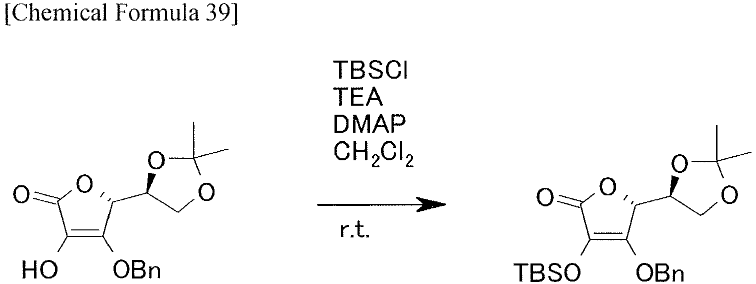

First, (R)-4-(benzyloxy)-5-((S)-2,2-dimethyl-1,3-dioxolan-4-yl)-3-hydroxyfuran-2(5H)-one (1 g) was dissolved in dichloromethane (10 mL), and triethylamine (990 mg), tert-butyldimethylsilyl chloride (738 mg) and N,N-dimethyl-4-aminopyridine (40 mg) were then added to the solution at 0°C. The temperature of the reaction mixture was then gradually raised to room temperature, and the mixture was then stirred overnight at the same temperature. A saturated sodium bicarbonate solution was then added at room temperature, and the mixture was extracted with chloroform. The organic phase was dried over anhydrous magnesium sulfate, filtered, and then concentrated under reduced pressure. The thus obtained concentrate was purified by silica gel column chromatography.

The product (2R)-3-benzyloxy-4-[tert-butyl(dimethyl)silyl]oxy-2-[(4S)-2,2-dimethyl-1,3-dioxolan-4-yl]-2H-furan-5-one (compound 2-33) was obtained in an amount of 826 mg, a yield of 60%.

### Example 35

### (Synthesis of (2R)-4-[tert-butyl(dimethyl)silyl]oxy-2-[(4S)-2,2-dimethyl-1,3-dioxolan-4-yl]-3-hydroxy-2H-furan-5-one (Compound 2-32))

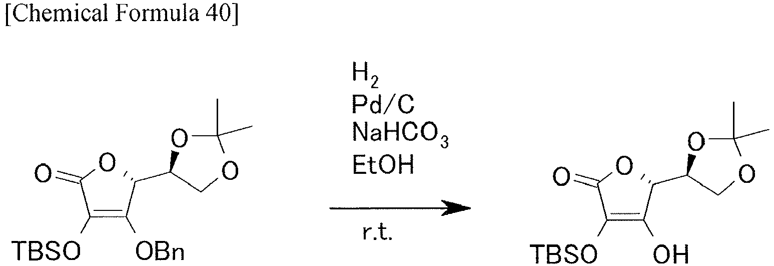

First, (2R)-3-benzyloxy-4-[tert-butyl(dimethyl)silyl]oxy-2-[(4S)-2,2-dimethyl-1,3-dioxolan-4-yl]-2H-furan-5-one (827 mg) was dissolved in ethanol (20 mL), palladium-carbon (83 mg) and sodium bicarbonate (17 mg) were added, a balloon filled with hydrogen gas was attached to the reaction vessel to substitute the atmosphere inside the system with hydrogen, and the resulting mixture was stirred at room temperature for 6 hours. The reaction mixture was then filtered with celite, and the solid material retained on the celite was washed with ethyl acetate. The resulting filtrate was then concentrated under reduced pressure.

The product (2R)-4-[tert-butyl(dimethyl)silyl]oxy-2-[(4S)-2,2-dimethyl-1,3-dioxolan-4-yl]-3-hydroxy-2H-furan-5-one (compound 2-32) was obtained stoichiometrically in an amount of 649 mg.

### Example 36

### (Synthesis of [(2R)-2-[(1S)-1,2-dihydroxyethyl]-3-hydroxy-5-oxo-2H-furan-4-yl] hexadecanoate (Compound 1-214))

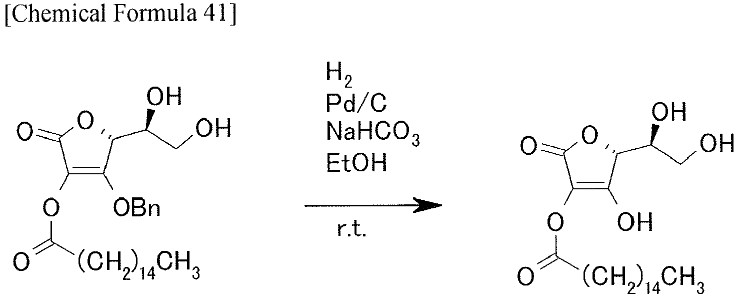

First, [(2R)-3-benzyloxy-2-[(1S)-1,2-dihydroxyethyl]-5-oxo-2H-furan-4-yl] hexadecanoate (470 mg) was dissolved in ethanol (10 mL), palladium-carbon (50 mg) and sodium bicarbonate (7 mg) were added, a balloon filled with hydrogen gas was attached to the reaction vessel to substitute the atmosphere inside the system with hydrogen, and the resulting mixture was stirred at room temperature for 6 hours. The reaction mixture was then filtered with celite, and the solid material retained on the celite was washed with ethyl acetate. The resulting filtrate was then concentrated under reduced pressure.

The product [(2R)-2-[(1S)-1,2-dihydroxyethyl]-3-hydroxy-5-oxo-2H-furan-4-yl] hexadecanoate (compound 1-214) was obtained stoichiometrically in an amount of 410 mg.

### Example 37

### (Synthesis of [(2R)-4-[tert-butyl(dimethyl)silyl]oxy-2-[(4S)-2,2-dimethyl-1,3-dioxolan-4-yl]-5-oxo-2H-furan-3-yl] hexadecanoate (Compound 2-34))

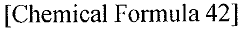

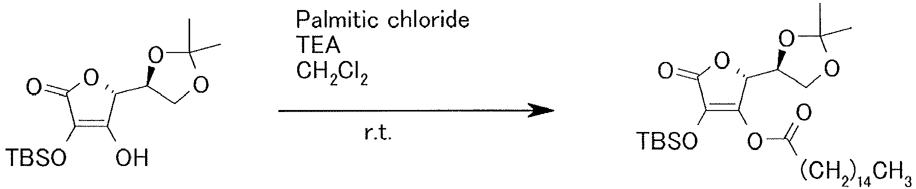

First, (2R)-4-[tert-butyl(dimethyl)silyl]oxy-2-[(4S)-2,2-dimethyl-1,3-dioxolan-4-yl]-3-hydroxy-2H-furan-5-one (649 mg) was dissolved in dichloromethane (10 mL), and triethylamine (595 mg) and palmitoyl chloride (810 mg) were then added to the solution at 0°C. The temperature of the reaction mixture was then gradually raised to room temperature, and the mixture was then stirred for 4 hours. A saturated sodium bicarbonate solution was then added at room temperature, and the mixture was extracted with chloroform. The organic phase was dried over anhydrous magnesium sulfate, filtered, and then concentrated under reduced pressure. The thus obtained concentrate was purified by silica gel column chromatography.

The product [(2R)-4-[tert-butyl(dimethyl)silyl]oxy-2-[(4S)-2,2-dimethyl-1,3-dioxolan-4-yl]-5-oxo-2H-furan-3-yl] hexadecanoate (compound 2-34) was obtained in an amount of 640 mg, a yield of 58%.

### Example 38

### (Synthesis of (S)-3-(benzyloxy)-5-((R)-1,2-dihydroxyethyl)furan-2(5H)-one (Compound 1-15))

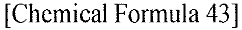

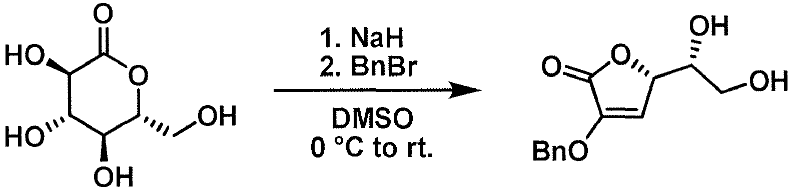

The synthesis of (S)-3-(benzyloxy)-5-((R)-1,2-dihydroxyethyl)furan-2(5H)-one was performed in accordance with a known method from the literature (Carbohydrate Research, 1997, vol. 303, p. 185). In other words, D-(+)-glucono-1,5-lactone (2.00 g, 11.2 mmol) was dissolved in dimethyl sulfoxide (60 mL), and the solution was cooled to 0°C. Sodium hydride (60%, 0.94 g) was then added to the solution, and after raising the temperature to room temperature, the temperature was once again cooled to 0°C, and benzyl bromide (3.83 g, 22.4 mmol) was added. The reaction mixture was then stirred overnight while the temperature was gradually raised to room temperature. Subsequently, the reaction mixture was poured into water, and then extracted with diethyl ether. Table salt was added to the water phase, the water phase was extracted with ethyl acetate, and the obtained organic phases were then combined, dried over anhydrous magnesium sulfate, filtered, and concentrated under reduced pressure. The thus obtained concentrate was purified by silica gel column chromatography.

The product (S)-3-(benzyloxy)-5-((R)-1,2-dihydroxyethyl)furan-2(5H)-one (compound 1-15) was obtained in an amount of 0.72 g, a yield of 26%.

### Example 39

### (Synthesis of (R)-2-((S)-4-(benzyloxy)-5-oxo-2,5-dihydrofuran-2-yl)-2-hydroxyethyl palmitate (Compound 1-107))

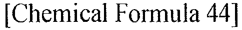

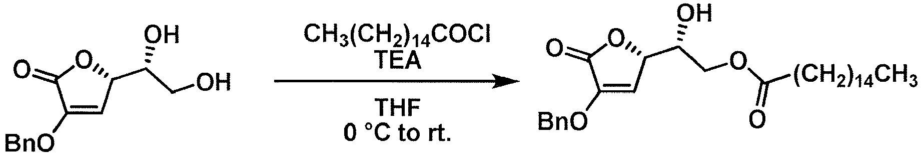

First, (S)-3-(benzyloxy)-5-((R)-1,2-dihydroxyethyl)furan-2(5H)-one (compound 1-15) was dissolved in tetrahydrofuran, triethylamine (0.32 g, 3.16 mmol) was added to the solution, and the resulting mixture was cooled to 0°C. A tetrahydrofuran solution of palmitoyl chloride (0.86 g, 3.13 mmol) was then added, and the reaction mixture was stirred overnight at room temperature. Subsequently, the reaction mixture was poured into water, and then extracted with ethyl acetate. The organic phase was dried over anhydrous magnesium sulfate, filtered, and then concentrated under reduced pressure. The thus obtained concentrate was purified by silica gel column chromatography.

The product (R)-2-((S)-4-(benzyloxy)-5-oxo-2,5-dihydrofuran-2-yl)-2-hydroxyethyl palmitate (compound 1-107) was obtained in an amount of 0.59 g, a yield of 43%.

### Example 40

### (Synthesis of 3,4-dihydroxy-5-(2-phenoxyethyl)furan-2(5H)-one (Compound 2-4))

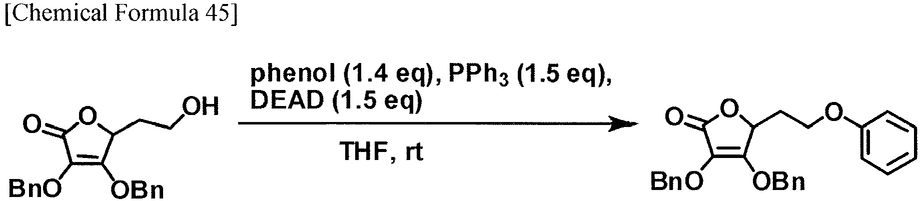

First, 3,4-bis(benzyloxy)-5-(2-hydroxyethyl)furan-2(5H)-one (1.2 g, 3.53 mmol) was dissolved in anhydrous tetrahydrofuran (20 mL), and triphenylphosphine (1.39 g, 5.29 mmol) and phenol (0.465 g, 4.94 mmol) were than added to the solution. Subsequently, diethyl azodicarboxylate (2.08 mL, 5.29 mmol) was added gradually in a dropwise manner over a period of at least one hour, and the resulting mixture was then stirred overnight at room temperature. The reaction mixture was then concentrated, and the concentrate was purified by flash chromatography.

As a result, 0.76 g of 3,4-bis(benzyloxy)-5-(2-phenoxyethyl)furan-2(5H)-one (compound 2-9) was obtained. A ¹H-NMR analysis confirmed that the product contained 0.3 g of phenol (crude yield: 52%). 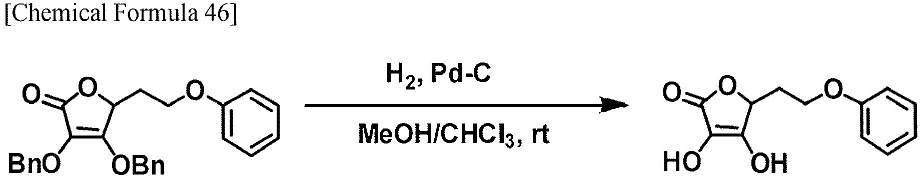 The 3,4-bis(benzyloxy)-5-(2-phenoxyethyl)furan-2(5H)-one (0.75 g, 1.80 mmol) containing 0.3 g of phenol was dissolved in a mixed solvent of chloroform/methanol = 10/1 (33 mL), and the solution was then deaerated for 5 minutes. Subsequently, 0.3 g of 10% Pd-C was added to the reaction solution, and hydrogen gas was blown into the system while the solution was stirred overnight at room temperature. The reaction mixture was then filtered with celite, the filtrate was concentrated, and the concentrate was purified by flash chromatography.

The product 3,4-dihydroxy-5-(2-phenoxyethyl)furan-2(5H)-one (compound 2-4) was obtained in an amount of 0.262 g (yield: 62%).

### Example 41

### (Synthesis of [(2R)-2-[(1S)-1,2-dihydroxyethyl]-4-hydroxy-5-oxo-2H-furan-3-yl] hexadecanoate (Compound 1-215))

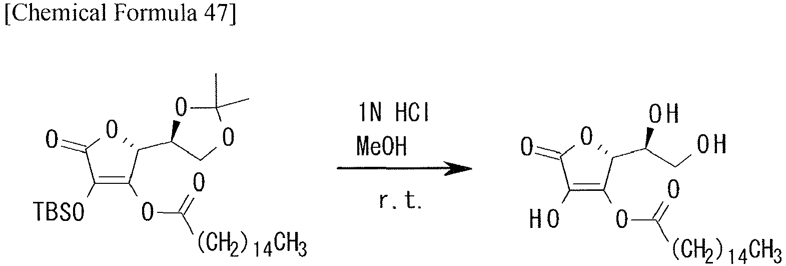

First, [(2R)-4-[tert-butyl(dimethyl)silyl]oxy-2-[(4S)-2,2-dimethyl-1,3-dioxolan-4-yl]-5-oxo-2H-furan-3-yl] hexadecanoate (640 mg) was dissolved in methanol (13 mL), 1 N hydrochloric acid (1.5 mL) was added to the solution at room temperature, and the resulting mixture was stirred overnight at the same temperature. The reaction mixture was then concentrated under reduced pressure, water was added to the concentrate, and the resulting mixture was extracted with ethyl acetate. The organic phase was dried over anhydrous magnesium sulfate, filtered, and then concentrated under reduced pressure. The thus obtained concentrate was purified by silica gel column chromatography.

The product [(2R)-2-[(1S)-1,2-dihydroxyethyl]-4-hydroxy-5-oxo-2H-furan-3-yl] hexadecanoate (compound 1-215) was obtained in an amount of 424 mg, a yield of 38%.

### Example 42

### (Synthesis of (S)-5-((S)-2,2-dimethyl-1,3-dioxolan-4-yl)-3-hydroxy-4-(propylamino)furan-2(5H)-one (Compound 2-116))

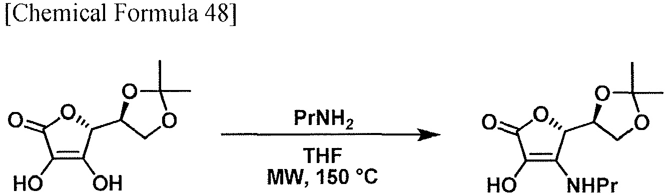

First, (+)-5,6-O-isopropylidene-L-ascorbic acid (1.60 g, 7.40 mmol) was dissolved in THF (15 mL), and propylamine (0.48g, 8.12 mmol) was then added to the solution. Subsequently, the solution was reacted at 150°C for 30 minutes under microwave irradiation. Following the reaction, the reaction mixture was cooled to room temperature and concentrated under reduced pressure, and the residue was purified by silica gel column chromatography yielding (S)-5-((S)-2,2-dimethyl-1,3-dioxolan-4-yl)-3-hydroxy-4-(propylamino)furan-2(5H)-one (compound 2-116) in an amount of 0.73 g, a yield of 38%.

### Example 43

### (Synthesis of (S)-3-(benzyloxy)-5-((S)-2,2-dimethyl-1,3-dioxolan-4-yl)-4-(propylamino)furan-2(5H)-one (Compound 2-120))

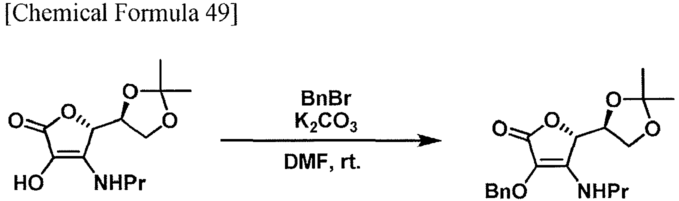

The compound 2-116 (0.99 g, 3.85 mmol) was dissolved in DMF (20 mL), potassium carbonate (0.64 g, 4.63 mmol) and benzyl bromide (0.79 g, 4.62 mmol) were added to the solution, and the resulting mixture was stirred overnight at room temperature. Water was then added to the reaction mixture, and the mixture was extracted with ethyl acetate. The organic phase was dried over anhydrous magnesium sulfate and filtered. The filtrate was then concentrated under reduced pressure, and the residue was purified by silica gel column chromatography, yielding (S)-3-(benzyloxy)-5-((S)-2,2-dimethyl-1,3-dioxolan-4-yl)-4-(propylamino)furan-2(5H)-one (compound 2-120) in an amount of 0.40 g, a yield of 30%.

### Example 44

### (Synthesis of (S)-3-(benzyloxy)-5-((S)-1,2-dihydroxyethyl)-4-(propylamino)furan-2(5H)-one (Compound 1-259))

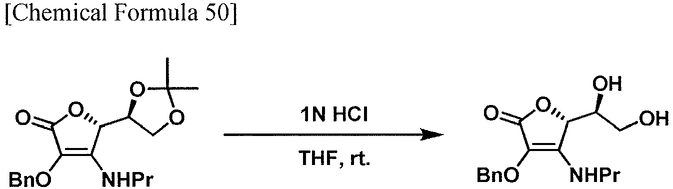

The compound 2-120 (0.40 g, 1.15 mmol) was dissolved in THF (20 mL), a 1 N aqueous solution of HCl (1 mL) was added to the solution at room temperature, and the resulting mixture was stirred overnight. The next day, additional 1 N HCl solution (1 mL) was added, and following stirring for a full day, the reaction mixture was poured into a saturated aqueous solution of sodium bicarbonate, and the resulting mixture was extracted with ethyl acetate. The organic phase was dried over anhydrous magnesium sulfate and filtered. The filtrate was then concentrated under reduced pressure, and the residue was purified by silica gel column chromatography, yielding (S)-3-(benzyloxy)-5-((S)-1,2-dihydroxyethyl)-4-(propylamino)furan-2(5H)-one (compound 1-259) in an amount of 0.21 g, a yield of 59%.

### Example 45

### (Synthesis of (S)-2-((R)-4-(diethylcarbamoyloxy)-3-hydroxy-5-oxo-2,5-dihydrofuran-2-yl)-2-hydroxyethyl palmitate (Compound 1-244) and (S)-2-((R)-3,4-bis(diethylcarbamoyloxy)-5-oxo-2,5-dihydrofuran-2-yl)-2-hydroxyethyl palmitate (Compound 1-246))

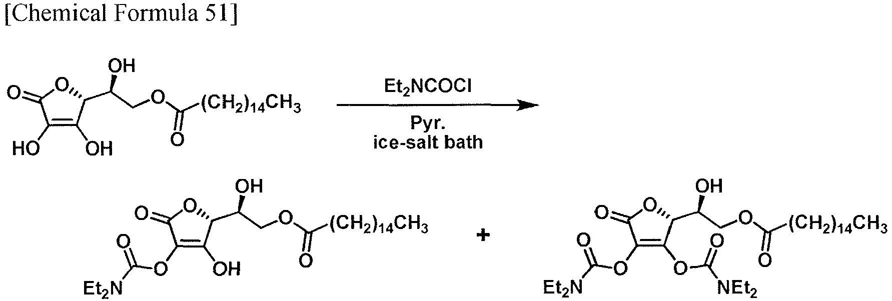

First, 6-O-palmitoyl-L-ascorbic acid (2.00 g, 4.82 mmol) was dissolved in pyridine (15 mL), and the resulting solution was cooled in an ice bath containing added salt. A toluene solution (5 mL) containing diethylcarbamoyl chloride (0.70 g, 5.16 mmol) was then added dropwise. The temperature of the reaction solution was then gradually raised to room temperature, and the reaction was allowed to proceed at the same temperature for two days. Subsequently, the insoluble material was removed by filtration and washed with toluene. The filtrate was then concentrated, and the residue was purified by silica gel column chromatography, yielding (S)-2-((R)-4-(diethylcarbamoyloxy)-3-hydroxy-5-oxo-2,5-dihydrofuran-2-yl)-2-hydroxyethyl palmitate (compound 1-244) in an amount of 0.50 g, a yield of 37%, and (S)-2-((R)-3,4-bis(diethylcarbamoyloxy)-5-oxo-2,5-dihydrofuran-2-yl)-2-hydroxyethyl palmitate (compound 1-246) in an amount of 0.12 g, a yield of 20%.

### Example 46

### (Synthesis of (S)-2-((S)-4-(benzyloxy)-5-oxo-3-(propylamino)-2,5-dihydrofuran-2-yl)-2-hydroxyethyl palmitate (Compound 1-261))

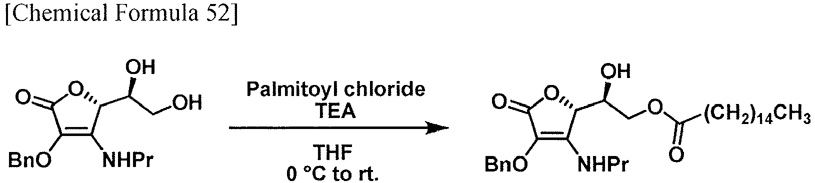

The compound 1-259 (0.21 g, 0.683 mmol) was dissolved in THF, triethylamine (80 mg, 0.79 mmol) was added to the solution at room temperature, the reaction mixture was cooled to 0°C, and then palmitoyl chloride (0.21 g, 0.764 mmol) was added at the same temperature. The temperature of the reaction mixture was then gradually raised to room temperature, and the mixture was then stirred overnight at room temperature. The reaction mixture was then concentrated under reduced pressure, and the residue was purified by silica gel column chromatography, yielding (S)-2-((S)-4-(benzyloxy)-5-oxo-3-(propylamino)-2,5-dihydrofuran-2-yl)-2-hydroxyethyl palmitate (compound 1-261) in an amount of 0.17 g, a yield of 46%.

### Example 47

### (Synthesis of (S)-2-hydroxy-2-((S)-4-hydroxy-5-oxo-3-(propylamino)-2,5-dihydrofuran-2-yl)ethyl palmitate (Compound 1-262))

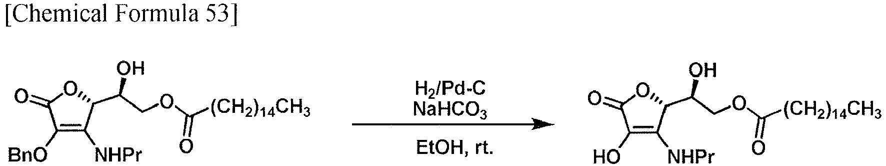

The compound 1-261 (0.17 g, 0.311 mmol) was dissolved in ethanol (15 mL), and sodium bicarbonate (29 mg, 0.345 mmol) and 10% palladium-carbon (0.20 g) were added to the solution. The reaction mixture was stirred overnight under a hydrogen atmosphere, and then filtered with celite. The filtrate was concentrated under reduced pressure, and the residue was purified by silica gel column chromatography, yielding (S)-2-hydroxy-2-((S)-4-hydroxy-5-oxo-3-(propylamino)-2,5-dihydrofuran-2-yl)ethyl palmitate (compound 1-262) in an amount of 50 mg, a yield of 35%.

### Example 48

### (Synthesis of (S)-4-((6-chloropyridin-3-yl)methylamino)-5-((S)-2,2-dimethyl-1,3-dioxolan-4-yl)-3-hydroxyfuran-2(5H)-one (Compound 2-122))

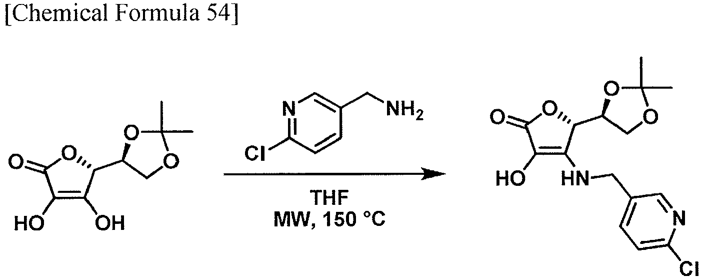

First, (+)-5,6-O-isopropylidene-L-ascorbic acid (1.60 g, 7.40 mmol) was dissolved in THF (15 mL), and (6-chloropyridin-3-yl)methaneamine (1.16 g, 8.13 mmol) was then added to the solution. Subsequently, the solution was reacted at 150°C for 30 minutes under microwave irradiation. Following the reaction, the reaction mixture was cooled to room temperature and concentrated under reduced pressure. The residue was then purified by silica gel column chromatography yielding (S)-4-((6-chloropyridin-3-yl)methylamino)-5-((S)-2,2-dimethyl-1,3-dioxolan-4-yl)-3-hydroxyfuran-2(5H)-one (compound 2-122) in an amount of 0.25 g, a yield of 9.9%.

### Example 49

### (Synthesis of (S)-2-((R)-4-(tert-butoxycarbonyloxy)-3-hydroxy-5-oxo-2,5-dihydrofuran-2-yl)-2-hydroxyethyl palmitate (Compound 1-247))

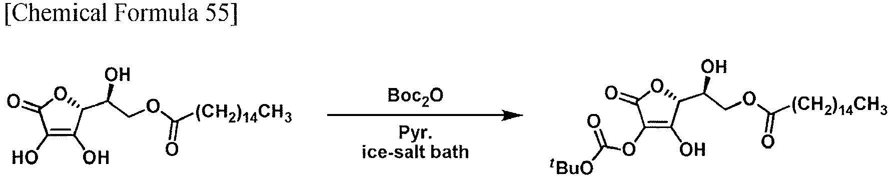

First, 6-O-palmitoyl-L-ascorbic acid (2.00 g, 4.82 mmol) was dissolved in pyridine (15 mL), and the resulting solution was cooled in an ice bath containing added salt. A toluene solution (5 mL) containing BoC₂O (1.13 g, 5.18 mmol) was then added dropwise. The temperature of the reaction solution was then gradually raised to room temperature, and the reaction was allowed to proceed overnight at the same temperature. Subsequently, the insoluble material was removed by filtration and washed with toluene. The filtrate was then concentrated, and the residue was purified by silica gel column chromatography, yielding (S)-2-((R)-4-(tert-butoxycarbonyloxy)-3-hydroxy-5-oxo-2,5-dihydrofuran-2-yl)-2-hydroxyethyl palmitate (compound 1-247) in an amount of 1.00 g, a yield of 40%.

### Example 50

### (Synthesis of (S)-2-hydroxy-2-((R)-3-hydroxy-5-oxo-4-(tosyloxy)-2,5-dihydrofuran-2-yl)ethyl palmitate (Compound 1-249))

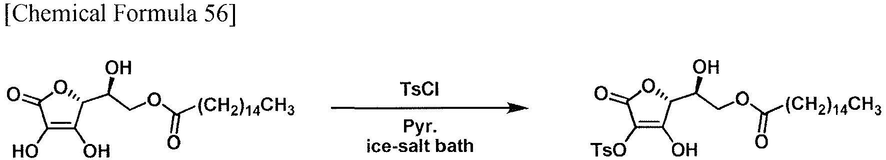

First, 6-O-palmitoyl-L-ascorbic acid (2.00 g, 4.82 mmol) was dissolved in pyridine (15 mL), and the resulting solution was cooled in an ice bath containing added salt. A toluene solution (5 mL) containing tosyl chloride (0.98 g, 5.14 mmol) was then added dropwise. The temperature of the reaction solution was then gradually raised to room temperature, and the reaction was allowed to proceed overnight at the same temperature. Subsequently, the insoluble material was removed by filtration and washed with toluene. The filtrate was then concentrated, and the residue was purified by silica gel column chromatography, yielding (S)-2-hydroxy-2-((R)-3-hydroxy-5-oxo-4-(tosyloxy)-2,5-dihydrofuran-2-yl)ethyl palmitate (compound 1-249) in an amount of 0.82 g, a yield of 30%.

### Example 51

### (Synthesis of 5-(furan-2-yl)-3,4-dimethoxyfuran-2(5H)-one (Compound 2-125))

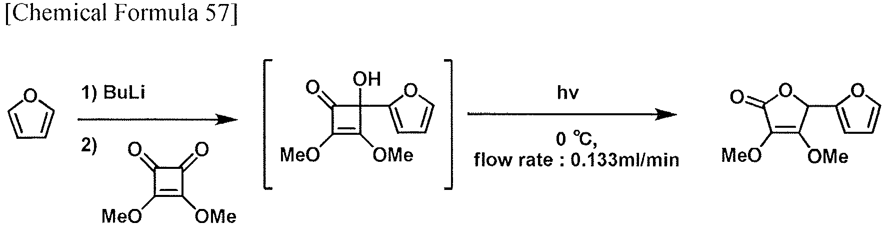

Synthesis was performed with reference to Angewandte Chemie, International Edition, 2012, 51, 4405 to 4408. Furan (1.2 g, 17.6 mmol) was dissolved in THF (20 mL), and the resulting solution was cooled to -20°C in a salt ice bath. Butyllithium (1.59 M, 4.87 mL, 7.74 mmol) was then added gradually in a dropwise manner over a period of at least one hour, and the resulting mixture was then stirred for 3 hours while the temperature was gradually raised from -20°C to room temperature. After 3 hours, the reaction mixture was cooled to -78°C, and a solution of 3,4-dimethoxy-3-cyclobutene-1,2-dione (1.1 g, 7.74 mmol) dissolved in THF (20 mL) was added gradually in a dropwise manner over a period of at least one hour. The reaction mixture was then stirred overnight while the temperature was gradually raised from -78°C to room temperature, a 5% aqueous solution of ammonium chloride (40 mL) was then added to the reaction mixture, and the resulting mixture was extracted with diethyl ether. Subsequently, the organic layer was washed with a saturated saline solution, and dried over anhydrous magnesium sulfate. The mixture was filtered, the filtrate was concentrated under reduced pressure, and the residue was then purified by flash chromatography, yielding 1.05 g of an intermediate (yield: 64%). This intermediate was unstable, and therefore following purification, was immediately used in the next reaction. The intermediate (0.25 g, 1.19 mmol) was dissolved in acetonitrile (10 mL), and the solution was deaerated for 10 minutes using an aspirator. This solution was then subjected to ultraviolet irradiation using a flow-type photoreaction system manufactured by YMC Co., Ltd. (reaction temperature: 0°C, flow rate: 0.133 mL/min). Subsequently, the reaction mixture was concentrated using a rotary evaporator, and the residue was purified by flash chromatography, yielding 5-(furan-2-yl)-3,4-dimethoxyfuran-2(5H)-one (compound 2-125) in an amount of 0.055 g, a yield of 22%.

### Example 52

### (Synthesis of 4-(4-chlorophenyl)-4-hydroxy-2,3-dimethoxycyclobut-2-enone (Compound 4-1))

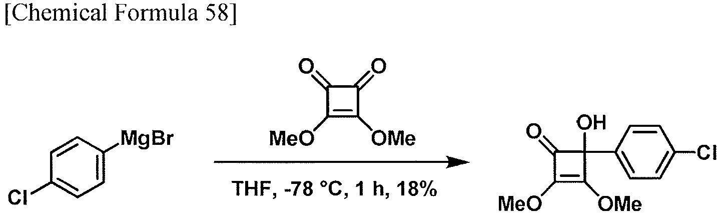

Synthesis was performed in a similar manner to that described for the compound 2-125. First, 3,4-dimethoxy-3-cyclobutene-1,2-dione (2.42 g, 17.0 mmol) was dissolved in THF (90 mL), and the resulting solution was cooled to -78°C. Subsequently, 4-chlorophenylmagnesium bromide (4.56 mL, 18.8 mmol) was added gradually in a dropwise manner over a period of at least one hour. The reaction was then continued at the same temperature for a further one hour, a 5% aqueous solution of ammonium chloride (40 mL) was then added to the reaction mixture, and the temperature of the mixture was then gradually raised from -78°C to room temperature. Subsequently, the reaction mixture was extracted with methylene chloride, and the organic phase was washed with a saturated saline solution and then dried over magnesium sulfate. The resulting mixture was filtered, the filtrate was concentrated under reduced pressure, and the resulting residue was then purified by flash chromatography, yielding 4-(4-chlorophenyl)-4-hydroxy-2,3-dimethoxycyclobut-2-enone (compound 4-1) in an amount of 0.684 g, a yield of 18%.

### Example 53

### (Synthesis of 5-(4-chlorophenyl)-3,4-dimethoxyfuran-2(5H)-one (Compound 2-128))

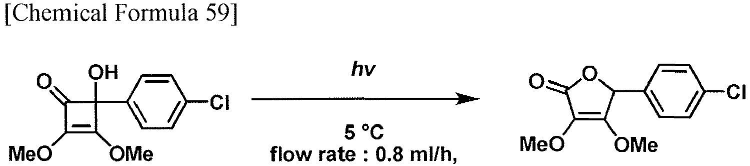

Synthesis was performed in a similar manner to that described for the compound 2-125. First, the compound 4-1 (0.33 g, 1.30 mmol) was dissolved in acetonitrile (10 mL), and the solution was deaerated for 10 minutes using an aspirator and then subjected to ultraviolet irradiation reaction using a flow-type photoreaction system manufactured by YMC Co., Ltd. (reaction temperature: 5°C, flow rate: 0.8 mL/h). Subsequently, the reaction mixture was concentrated using a rotary evaporator, and the residue was purified by flash chromatography, yielding 5-(4-chlorophenyl)-3,4-dimethoxyfuran-2(5H)-one (compound 2-128) in an amount of 0.08 g, a yield of 24%.

### Example 54

### (Synthesis of 2-(4-hydroxy-3-methoxy-5-oxofuran-2(5H)-ylidene) acetate (Compound 3-55))

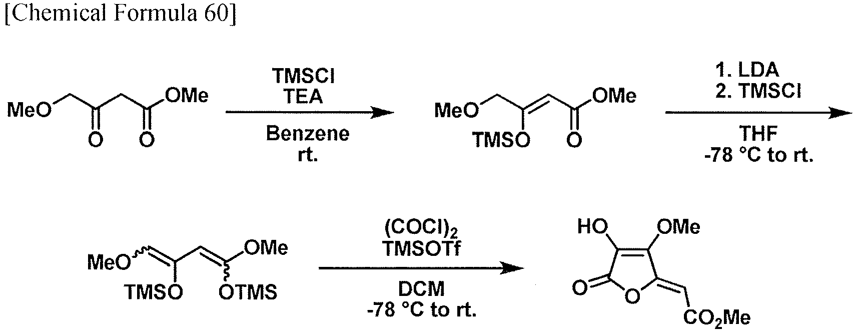

Methyl 4-methoxyacetoacetate (25 g, 171.1 mmol) was dissolved in benzene (350 mL), and triethylamine (22.61 g, 223.4 mmol) and trimethylsilyl chloride (25 g, 230.1 mmol) were then added sequantially to the solution. Following stirring for a full day at room temperature, the reaction mixture was filtered with celite and washed with hexane. The filtrate was concentrated using an evaporator, and by subsequently distilling the residue under reduced pressure, methyl 4-methoxy-3-(trimethylsilyloxy)but-2-enoate (77 to 78°C at 5 mmHg) was obtained in an amount of 28.14 g, a yield of 71%. Next, a THF solution (120 mL) of diisopropylamine (17.3 mL, 126.9 mmol) was cooled to -78°C, and butylllithium (1.59 M, 87 mL, 138.3 mmol) was added in a dropwise manner. The resulting mixture was stirred at 0°C for 30 minutes and then once again cooled to -78°C, and a THF solution (30 mL) containing the methyl 4-methoxy-3-(trimethylsilyloxy)but-2-enoate (28.14 g, 115.2 mmol) was then added in a dropwise manner. Following stirring for a short period, a THF solution (30 mL) of trimethylsilyl chloride (13.77 g, 126.7 mmol) was added dropwise to the reaction mixture. The reaction mixture was then stirred overnight as the temperature was gradually raised to room temperature. The THF was then removed using an evaporator, hexane (120 mL) was added to the residue, and the solution was filtered using celite under a nitrogen atmosphere. The filtrate was then distilled under reduced pressure, yielding 4-methoxy-6-(methoxymethylene)-2,2,8,8-tetramethyl-3,7-dioxa-2,8-disilanon-4-ene (96 to 97°C at 1 mmHg) in an amount of 16.68 g, a yield of 46%. Subsequently, this intermediate (16.68 g, 53.0 mmol) was dissolved in methylene chloride (795 mL), the resulting solution was cooled to -78°C, and oxalyl chloride (4.6 mL, 53.6 mmol) and a methylene chloride solution (30 mL) of trimethylsilyl trifluoromethanesulfonate (3.53 g, 15.9 mmol) were added sequntially to the solution. The reaction solution was stirred overnight while the temperature was gradually raised to room temperature, and was then poured into a saturated saline solution. The organic phase was separated, and the water phase was extracted with ethyl acetate. All the organic phases were then combined, dried over anhydrous magnesium sulfate, and filtered. The filtrate was concetrated under reduced pressure, and the residue was purified by silica gel column chromatography, yielding 2-(4-hydroxy-3-methoxy-5-oxofuran-2(5H)-ylidene) acetate (compound 3-55) in an amount of 8.23 g, a yield of 78%.

### Example 55

### (Synthesis of methyl 2-(4-hydroxy-3-methoxy-5-oxo-2,5-dihydrofuran-2-yl)acetate (Compound 2-130))

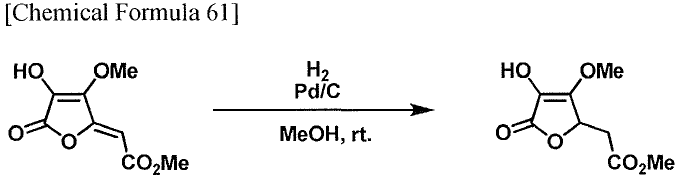

The compound 3-55 (1.20 g, 6.00 mmol) was dissolved in methanol (30 mL), and 10% palladium-carbon (0.24 g) was added. The reaction mixture was stirred at room temperature under a hydrogen atmosphere for 3 days, and then filtered with celite. The filtrate was concetrated under reduced pressure, and the thus obtained residue was purified by silica gel column chromatography, yielding 2-(4-hydroxy-3-methoxy-5-oxo-2,5-dihydrofuran-2-yl)acetate (compound 2-130) in an amount of 0.67 g, a yield of 55%.

### Example 56

### (Synthesis of 4-methoxy-5-(2-methoxy-2-oxoethylidene)-2-oxo-2,5-dihydrofuran-3-yl palmitate (Compound 3-59))

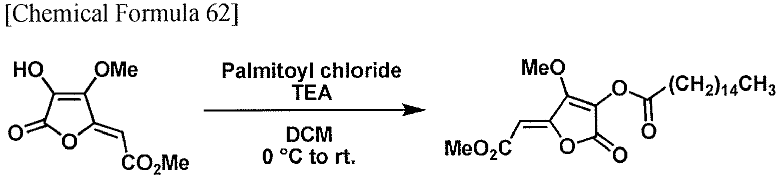

The compound 3-55 (1.20 g, 6.00 mmol) was dissolved in methylene chloride (30 mL), and following the addition of triethylamine (0.73 g, 7.21 mmol), palmitoyl chloride (1.98 g, 7.20 mmol) was added at 0°C. The reaction mixture was then stirred at room temperature for 3 days, and then filtered using celite. The filtrate was concetrated under reduced pressure, and the residue was purified by silica gel column chromatography, yielding 4-methoxy-5-(2-methoxy-2-oxoethylidene)-2-oxo-2,5-dihydrofuran-3-yl palmitate (compound 3-59) in an amount of 2.25 g, a yield of 86%.

### Example 57

### (Synthesis of 4-methoxy-5-(2-methoxy-2-oxoethyl)-2-oxo-2,5-dihydrofuran-3-yl palmitate (Compound 2-131))

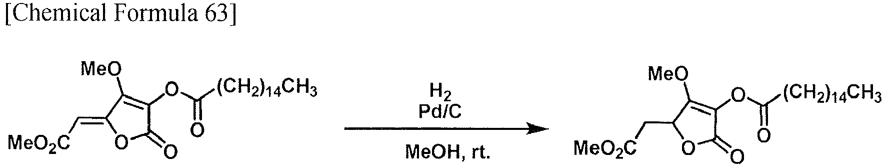

The compound 3-55 (1.48 g, 3.37 mmol) was dissolved in methanol, and 10% palladium-carbon (0.36 g) was added. The reaction mixture was stirred under a hydrogen atmosphere for 2 days, and then filtered with celite. The filtrate was concetrated under reduced pressure, and the thus obtained residue was purified by silica gel column chromatography, yielding 4-methoxy-5-(2-methoxy-2-oxoethyl)-2-oxo-2,5-dihydrofuran-3-yl palmitate (compound 2-131) in an amount of 1.25 g, a yield of 84%.

### Example 58

### (Synthesis of (S)-2-hydroxy-2-((R)-3-hydroxy-5-oxo-4-((2S,3R,4S,5S,6R)-3,4,5-trihydroxy-6-(hydroxymethyl)tetrahydro-2H-pyran-2-yloxy)-2,5-dihydrofuran-2-yl)ethyl palmitate (Compound 1-251) and ((2R,3S,4S,5R,6S)-6-((R)-5-((S)-1,2-dihydroxyethyl)-4-hydroxy-2-oxo-2,5-dihydrofuran-3-yloxy)-3,4,5-trihydroxytetrahydro-2H-pyran-2-yl)methyl palmitate (Compound 1-252))

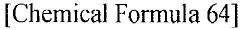

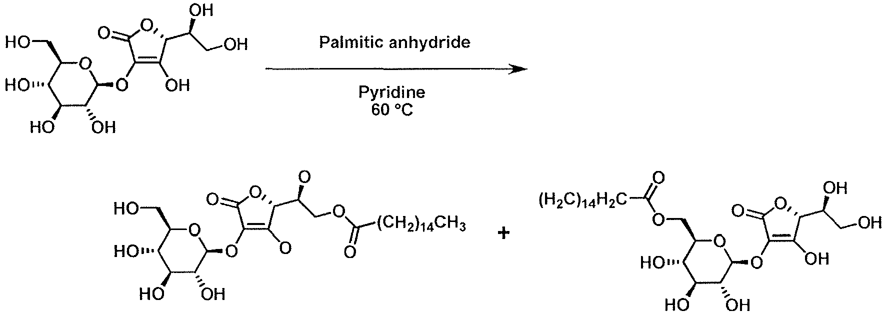

First, 2-O-α-D-glucopyranosyl-L-ascorbic acid (11.9 g) was dissolved in pyridine (100 mL), and plamitic anhydride (19.5 g) was then added to the solution at room temperature. The temperature of the reaction mixture was raised to 60°C, and the mixture was then stirred overnight at the same temperature. The reaction mixture was then cooled to room temperature and concentrated under reduced pressure, and the resulting residue was purified by silica gel column chromatography, yielding (S)-2-hydroxy-2-((R)-3-hydroxy-5-oxo-4-((2S,3R,4S,5S,6R)-3,4,5-trihydroxy-6-(hydroxymethyl)tetrahydro-2H-pyran-2-yloxy)-2,5-dihydrofuran-2-yl)ethyl palmitate (compound 1-251) in an amount of 691 mg, a yield of 3.9%, and ((2R,3S,4S,5R,6S)-6-((R)-5-((S)-1,2-dihydroxyethyl)-4-hydroxy-2-oxo-2,5-dihydrofuran-3-yloxy)-3,4,5-trihydroxytetrahydro-2H-pyran-2-yl)methyl palmitate (compound 1-252) in an amount of 316 mg, a yield of 1.9%.

### (Examples of the Compound of the Present Invention)

The compounds of the present invention obtained in the examples described above, and other compounds of the present invention synthesized using the same techniques as the above examples are shown in Tables 1 to 3. In Table 1 and Table 2, X¹, X² and A correspond with X¹, X² and A in formula (1). In Table 3, X¹, X², R⁵¹ and R⁵² correspond with X¹, X², R⁵¹ and R⁵² in formula (1) and formula (2). Further, a line to which an * is appended in any of the following chemical formulas indicates the bonding position to the 5-membered ring.

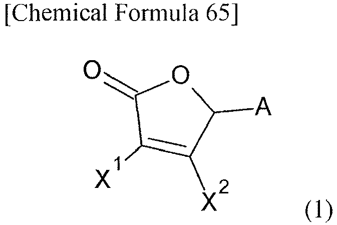

[Table 1]

**Table 1-1**

| Number | X¹ | X² | A |
|---|---|---|---|
| 1-1 | H | OH | 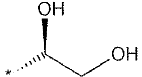 |
| 1-2 | OH | OBn | 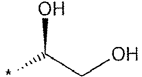 |
| 1-3 | OMe | OH | 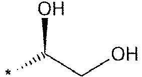 |
| 1-4 | OEt | OH | 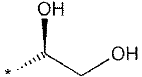 |
| 1-5 | OBn | OH | 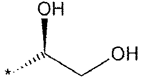 |
| 1-6 | OCH₂COPh-4-F | OH | 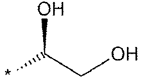 |
| 1-7 | Ph | OH | 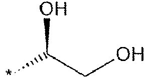 |
| 1-8 | OSO₂Ph | OH | 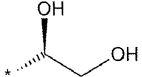 |
| 1-9 | OSO₂N(Me)₂ | OH | 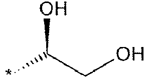 |
| 1-10 | OP(=O)(OH)OCH₂NH₂ | OH | 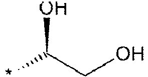 |
| 1-11 | 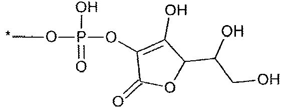 | OH | 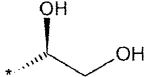 |
| 1-12 | 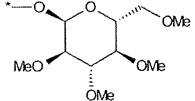 | OH | 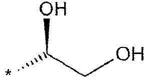 |

[Table 2]

**Table 1-2**

| Number | X¹ | X² | A |
|---|---|---|---|
| 1-13 | OMe | H | 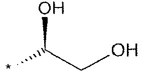 |
| 1-14 | OEt | H | 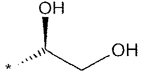 |
| 1-15 | OBn | H | 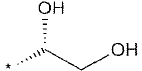 |
| 1-16 | OCH₂COPh-4-F | H | 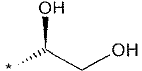 |
| 1-17 | Ph | H | 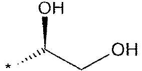 |
| 1-18 | OSO₂Ph | H | 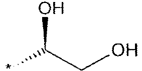 |
| 1-19 | OSO₂N(Me)₂ | H | 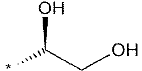 |
| 1-20 | OP(=O)(OH)OCH₂NH₂ | H | 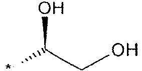 |
| 1-21 | OSi(Me)₂^{t}Bu | H | 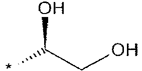 |
| 1-22 | 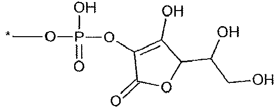 | H | 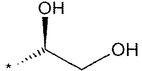 |
| 1-23 | 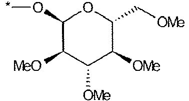 | H | 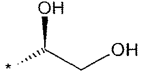 |
| 1-24 | OPO₃H₂ | H | 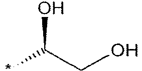 |

[Table 3]

**Table 1-3**

| Number | X¹ | X² | A |
|---|---|---|---|
| 1-25 | OSO₃H | H | 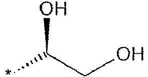 |
| 1-26 | glucosyl | H | 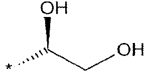 |
| 1-27 | galactosyl | H | 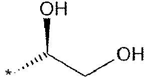 |
| 1-28 | mannosyl | H | 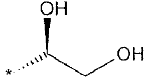 |
| 1-29 | OCOMe | H | 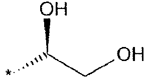 |
| 1-30 | OCOEt | H | 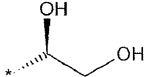 |
| 1-31 | OCOⁿPr | H | 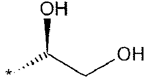 |
| 1-32 | OCO'Pr | H | 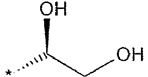 |
| 1-33 | OCO(CH₂)₁₄CH₃ | H | 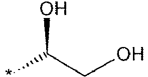 |
| 1-34 | OCO(CH₂)₁₅CH₃ | H | 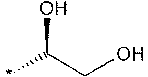 |
| 1-35 | OCO(CH)₁₆CH₃ | H | 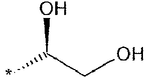 |
| 1-36 | OCO(CH)₁₇CH₃ | H | 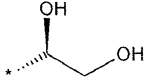 |

[Table 4]

**Table 1-4**

| Number | X¹ | X² | A |
|---|---|---|---|
| 1-37 | OCOCH=CH₂ | H | 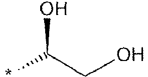 |
| 1-38 | OCOCH₂CH=CH₂ | H | 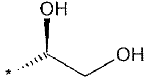 |
| 1-39 | OCO(CH₂)₇CH=CH(CH₂)₅CH₃ | H | 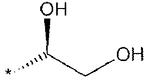 |
| 1-40 | OCOCH=C(CH₃)CH₂CH₂CH=C(CH₃)₂ | H | 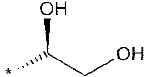 |
| 1-41 | OCOCH₂CH (CH)CH₂CH₂CH=C(CH)₂ | H | 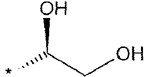 |
| 1-42 | OCO^{t}Bu | OBn | 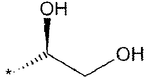 |
| 1-43 | OBn | OBn | 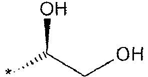 |
| 1-44 | H | OMe | 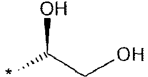 |
| 1-45 | H | OEt | 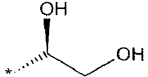 |
| 1-46 | H | OBn | 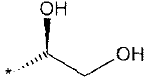 |
| 1-47 | H | OCH₂COPh-4-F | 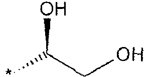 |
| 1-48 | H | Ph | 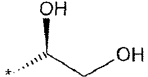 |

[Table 5]

**Table 1-5**

| Number | X¹ | X² | A |
|---|---|---|---|
| 1-49 | H | OSO₂Ph | 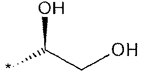 |
| 1-50 | H | OSO₂N(Me)₂ | 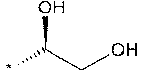 |
| 1-51 | H | OP(=O)(OH)OCH₂NH₂ | 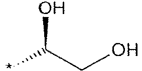 |
| 1-52 | H | OSi(Me)₂^{t}Bu | 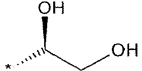 |
| 1-53 | H | 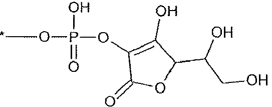 | 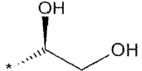 |
| 1-54 | H | 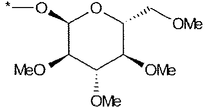 | 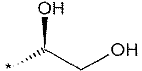 |
| 1-55 | H | H | 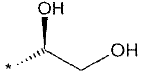 |
| 1-56 | H | OMe | 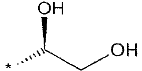 |
| 1-57 | H | OEt | 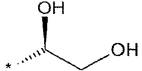 |
| 1-58 | H | OBn | 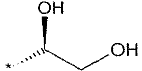 |
| 1-59 | H | OCH₂COPh-4F | 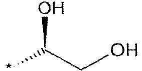 |
| 1-60 | H | Ph | 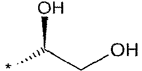 |

[Table 6]

**Table 1-6**

| Number | X¹ | X² | A |
|---|---|---|---|
| 1-61 | H | OSO₂Ph | 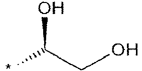 |
| 1-62 | H | OSO₂N(Me)₂ | 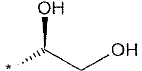 |
| 1-63 | H | OP(=O)(OH)OCH₂NH₂ | 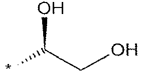 |
| 1-64 | H | 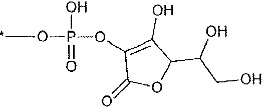 | 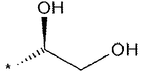 |
| 1-65 | H | 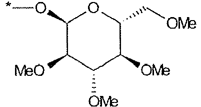 | 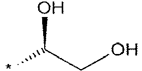 |
| 1-66 | H | OPO₃H₂ | 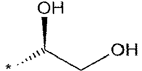 |
| 1-67 | H | OSO₃H | 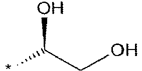 |
| 1-68 | H | glucosyl | 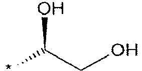 |
| 1-69 | H | galactosyl | 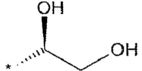 |
| 1-70 | H | mannosyl | 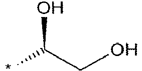 |
| 1-71 | H | OCOMe | 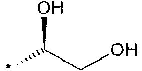 |
| 1-72 | H | OCOEt | 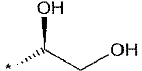 |

[Table 7]

**Table 1-7**

| Number | X¹ | X² | A |
|---|---|---|---|
| 1-73 | H | OCOⁿPr | 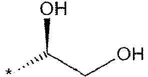 |
| 1-74 | H | OCOⁱPr | 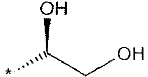 |
| 1-75 | H | OCO^{t}Bu | 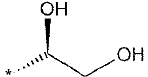 |
| 1-76 | H | OCO(CH₂)₁₄CH₃ | 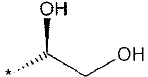 |
| 1-77 | H | OCO(CH₂)₁₅CH₃ | 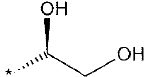 |
| 1-78 | H | OCO(CH₂)₁₆CH₃ | 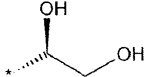 |
| 1-79 | H | OCO(CH₂)₁₇CH₃ | 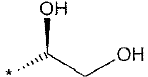 |
| 1-80 | H | OCOCH=CH₂ | 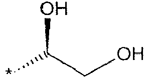 |
| 1-81 | H | OCOCH₂CH=CH₂ | 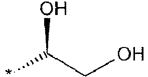 |
| 1-82 | H | OCO(CH₂)₇CH=CH(CH₂)₅CH₃ | 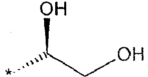 |
| 1-83 | H | OCOCH=C(CH₃)CH₂CH₂CH=C(CH₃)₂ | 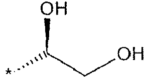 |
| 1-84 | H | OCOCH₂CH(CH₃)CH₂CH₂CH=C(CH₃)₂ | 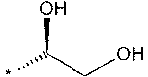 |

[Table 8]

**Table 1-8**

| Number | X¹ | X² | A |
|---|---|---|---|
| 1-85 | F | OH | 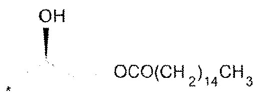 |
| 1-86 | Cl | OH | 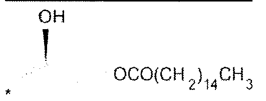 |
| 1-87 | Br | OH | 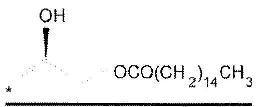 |
| 1-88 | I | OH | 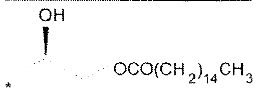 |
| 1-89 | OH | F | 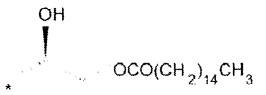 |
| 1-90 | OH | Cl | 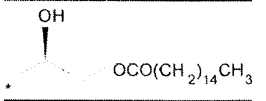 |
| 1-91 | OH | Br | 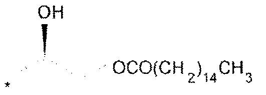 |
| 1-92 | OH | I | 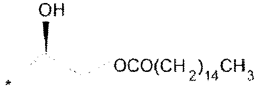 |
| 1-93 | OH | OH | 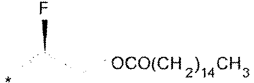 |
| 1-94 | OH | OH | 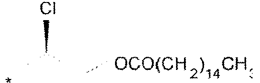 |
| 1-95 | OH | OH | 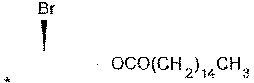 |
| 1-96 | OH | OH | 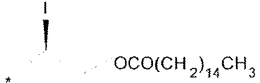 |

[Table 9]

**Table 1-9**

| Number | X¹ | X² | A |
|---|---|---|---|
| 1-97 | OCO(CH₂)₁₄CH₃ | OH | 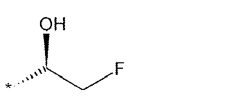 |
| 1-98 | OCO(CH₂)₁₄CH₃ | OH | 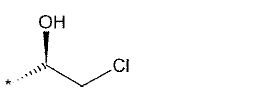 |
| 1-99 | OCO(CH₂)₁₄CH₃ | OH | 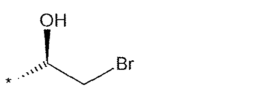 |
| 1-100 | OCO(CH₂)₁₄CH₃ | OH | 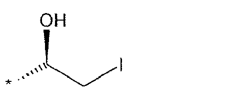 |
| 1-101 | OH | OCO(CH₂)₁₄CH₃ | 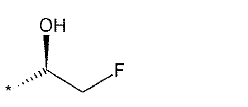 |
| 1-102 | OH | OCO(CH₂)₁₄CH₃ | 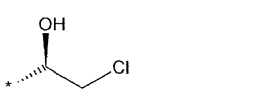 |
| 1-103 | OH | OCO(CH₂)₁₄CH₃ | 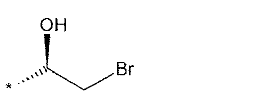 |
| 1-104 | OH | OCO(CH₂)₁₄CH₃ | 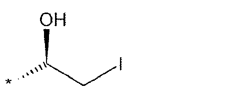 |
| 1-105 | OCO(CH₂)₁₄CH₃ | OBn | 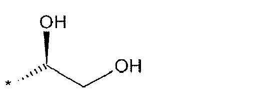 |
| 1-106 | OP(=O)(OH)OCH₂NH₂ | OH | 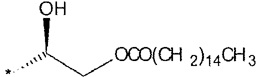 |
| 1-107 | OBn | H | 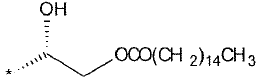 |
| 1-108 | OBn | OBn | 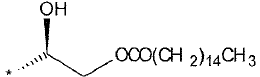 |

[Table 10]

**Table 1-10**

| Number | X¹ | X² | A |
|---|---|---|---|
| 1-109 | OCO^{t}Bu | OBn | 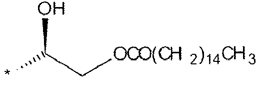 |
| 1-110 | OCO^{t}Bu | OH | 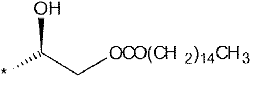 |
| 1-111 | OCO^{t}Bu | OBn | 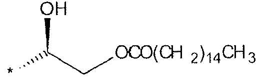 |
| 1-112 | OCO^{t}Bu | OBn | 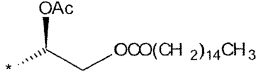 |
| 1-113 | OCO^{t}Bu | OBn | 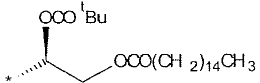 |
| 1-114 | OH | OP(=O)(OH)OCH₂NH₂ | 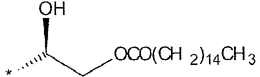 |
| 1-115 | OH | OH | 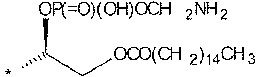 |
| 1-116 | OMe | OH | 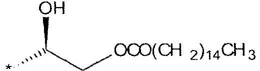 |
| 1-117 | OH | OMe | 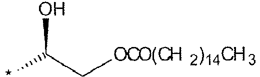 |
| 1-118 | OH | OH | 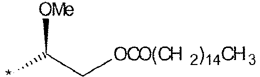 |
| 1-119 | OEt | OH | 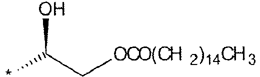 |
| 1-120 | OH | OEt | 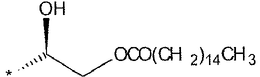 |

[Table 11]

**Table 1-11**

| Number | X¹ | X² | A |
|---|---|---|---|
| 1-121 | OH | OH | 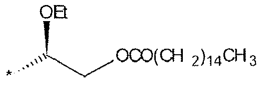 |
| 1-122 | OSO₂Ph | OH | 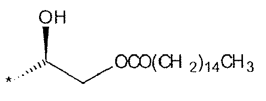 |
| 1-123 | OH | OSO₂Ph | 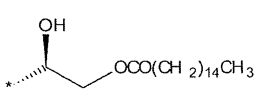 |
| 1-124 | OH | OH | 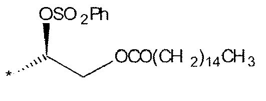 |
| 1-125 | OSO₂N(Me)₂ | OH | 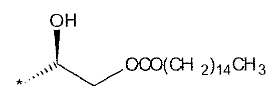 |
| 1-126 | OH | OSO₂N(Me)₂ | 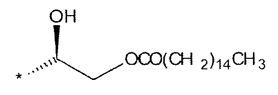 |
| 1-127 | OH | OH | 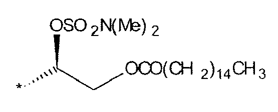 |
| 1-128 | OP(=O)(OH)OCH₂NH₂ | OH | 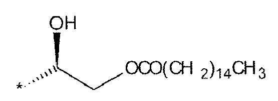 |
| 1-129 | OH | OP(=O)(OH)OCH₂NH₂ | 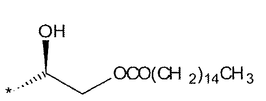 |
| 1-130 | OH | OH | 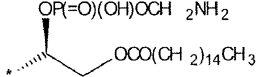 |
| 1-131 | OH | H | 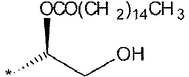 |
| 1-132 | OH | H | 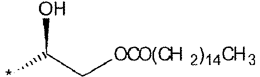 |

[Table 12]

**Table 1-12**

| Number | X¹ | X² | A |
|---|---|---|---|
| 1-133 | OH | OH | 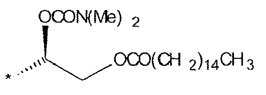 |
| 1-134 | OH | OH | 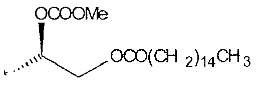 |
| 1-135 | OH | OH | 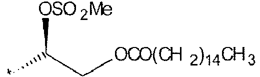 |
| 1-136 | OH | OCON(Me)₂ | 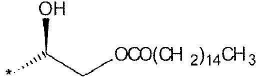 |
| 1-137 | OH | OCOOMe | 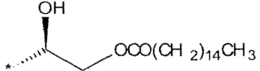 |
| 1-138 | OH | OSO₂Me | 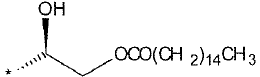 |
| 1-139 | OCON(Me)₂ | OH | 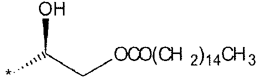 |
| 1-140 | OCOOMe | OH | 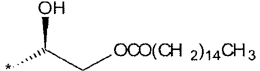 |
| 1-141 | OSO₂Me | OH | 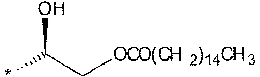 |
| 1-142 | OCOPh | OH | 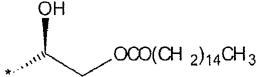 |
| 1-143 | OH | OCOPh | 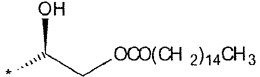 |
| 1-144 | OH | OH | 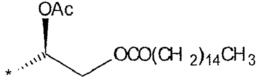 |

[Table 13]

**Table 1-13**

| Number | X¹ | X² | A |
|---|---|---|---|
| 1-145 | OH | OH | 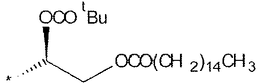 |
| 1-146 | OH | OH | 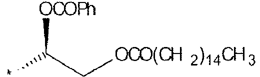 |
| 1-147 | OBn | OBn | 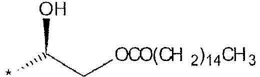 |
| 1-148 | OCO^{t}Bu | OH | 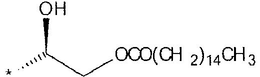 |
| 1-149 | OCO^{t}Bu | OH | 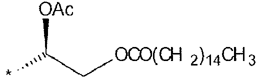 |
| 1-150 | OBn | OBn | 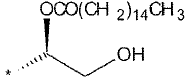 |
| 1-151 | OBn | OBn | 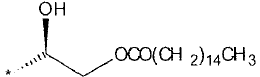 |
| 1-152 | OBn | OBn | 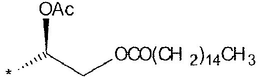 |
| 1-153 | OBn | OBn | 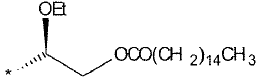 |
| 1-154 | OBn | OBn | 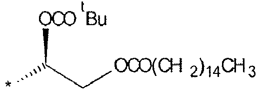 |
| 1-155 | OCO^{t}Bu | OH | 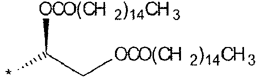 |
| 1-156 | OCO^{t}Bu | OBn | 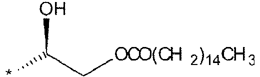 |

[Table 14]

**Table 1-14**

| Number | X¹ | X² | A |
|---|---|---|---|
| 1-157 | OCO^{t}Bu | OBn | 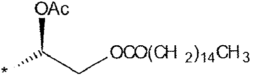 |
| 1-158 | OCO^{t}Bu | OBn | 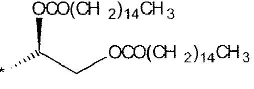 |
| 1-159 | OBn | OBn | 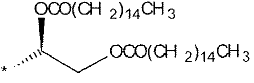 |
| 1-160 | OBn | OBn | 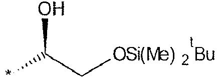 |
| 1-161 | OBn | OBn | 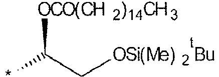 |
| 1-162 | OBn | OBn | 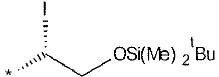 |
| 1-168 | OPO₃H₂ | OH | 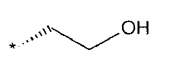 |
| 1-164 | OSO₃H | OH | 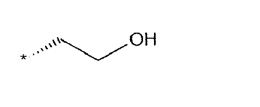 |
| 1-165 | glucosyl | OH | 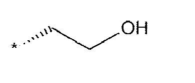 |
| 1-166 | galactosyl | OH | 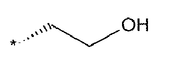 |
| 1-167 | mannosyl | OH | 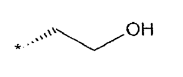 |
| 1-168 | OCOMe | OH | 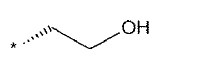 |

[Table 15]

**Table 1-15**

| Number | X¹ | X² | A |
|---|---|---|---|
| 1-169 | OCOEt | OH | 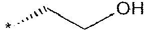 |
| 1-170 | OCOⁿPr | OH | 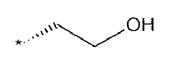 |
| 1-171 | OCOⁱPr | OH | 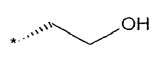 |
| 1-172 | OCO^{t}Bu | OH | 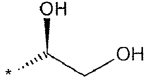 |
| 1-173 | OCO(CH₂)₁₄CH₃ | OH | 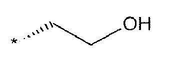 |
| 1-174 | OCO(CH₂)₁₅CH₃ | OH | 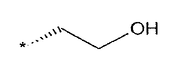 |
| 1-175 | OCO(CH₂)₁₆CH₃ | OH | 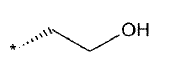 |
| 1-176 | OCO(CH)₁₇CH₃ | OH | 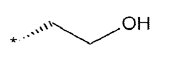 |
| 1-177 | OCOCH=CH₂ | OH | 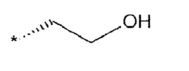 |
| 1-178 | OCOCH₂CH=CH₂ | OH | 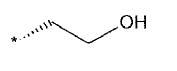 |
| 1-179 | OCO(CH₂)₇CH=CH(CH₂)₅CH₃ | OH | 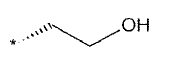 |
| 1-180 | OSO₃H | H | 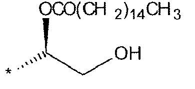 |

[Table 16]

**Table 1-16**

| Number | X¹ | X² | A |
|---|---|---|---|
| 1-181 | OSO₃H | OH | 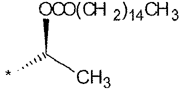 |
| 1-182 | OP(=O)(OH)OCH₂NH₂ | OH | 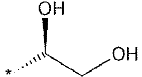 |
| 1-183 | OP(=O)(OH)OCH₂NH₂ | OH | 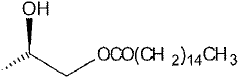 |
| 1-184 | OH | OP(=O)(OH)OCH₂NH₂ | 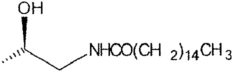 |
| 1-185 | OH | OH | 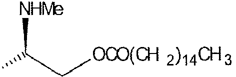 |
| 1-186 | OH | OH | 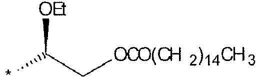 |
| 1-187 | 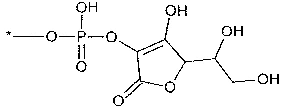 | OH | 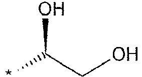 |
| 1-188 | OEt | Ph | 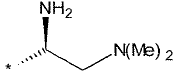 |
| 1-189 | OEt | Ph | 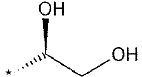 |
| 1-190 | OSO₂Ph | OBn | 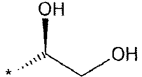 |
| 1-191 | OBn | OBn | 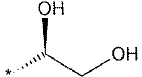 |
| 1-192 | H | OBn | 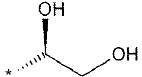 |

[Table 17]

**Table 1-17**

| Number | X¹ | X² | A |
|---|---|---|---|
| 1-193 | OMe | OMe | 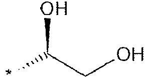 |
| 1-194 | OSi(Me)₂^{t}Bu | OH | 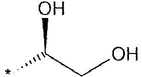 |
| 1-195 | OH | OSi(Me)₂^{t}Bu | 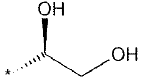 |
| 1-196 | OH | OH | 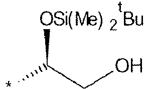 |
| 1-197 | OH | OH | 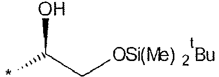 |
| 1-198 | OSi(Me)₂^{t}Bu | OH | 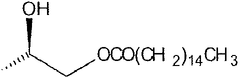 |
| 1-199 | OH | OSi(Me)₂^{t}Bu | 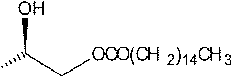 |
| 1-200 | OH | OH | 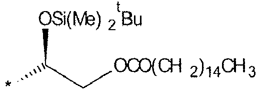 |
| 1-201 | OH | OH | 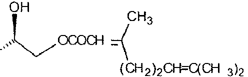 |
| 1-202 | OH | OH | 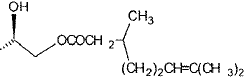 |
| 1-203 | OH | OH | 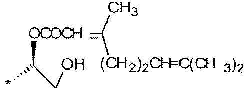 |
| 1-204 | OH | OH | 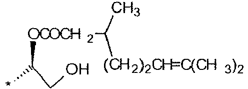 |

[Table 18]

**Table 1-18**

| Number | X¹ | X² | A |
|---|---|---|---|
| 1-205 | OH | OCOCH=C(CH₃)CH₂CH₂CH=C(CH₃)₂ | 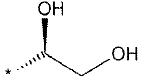 |
| 1-206 | OH | OCOCH₂CH(CH₃)CH₂CH₂CH₂C(CH₃)₂ | 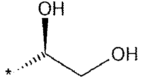 |
| 1-207 | OCOCH=C(CH₃)CH₂CH₂CH=C(CH₃)₂ | OH | 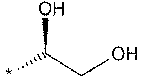 |
| 1-208 | OCOCH₂CH(CH₃)CH₂CH₂CH=C(CH₃)₂ | OH | 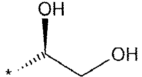 |
| 1-209 | OH | OH | 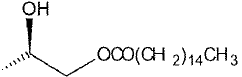 |
| 1-210 | OCO^{t}Bu | OH | 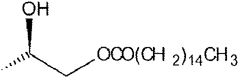 |
| 1-211 | OCO^{t}Bu | OH | 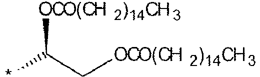 |
| 1-212 | OCO^{t}Bu | OH | 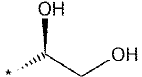 |
| 1-213 | OH | OCO^{t}Bu | 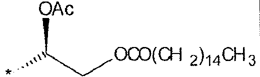 |
| 1-214 | OCO(CH₂)₁₄CH₃ | OH | 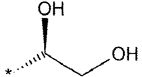 |
| 1-215 | OH | OCO(CH₂)₁₄CH₃ | 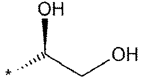 |
| 1-216 | OCO(CH₂)₁₄CH₃ | OH | 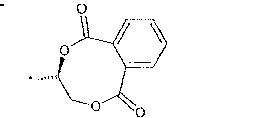 |

[Table 19]

**Table 1-19**

| Number | X¹ | X² | A |
|---|---|---|---|
| 1-217 | OH | OCO(CH₂)₁₄CH₃ | 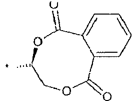 |
| 1-218 | OH | OH | 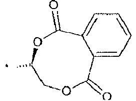 |
| 1-219 | OCO(CH₂)₁₄CH₃ | OH | 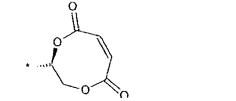 |
| 1-220 | OH | OCO(CH₂)₁₄CH₃ | 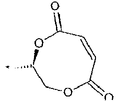 |
| 1-221 | OPO₃H₂ | OH | 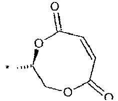 |
| 1-222 | OCO(CH₂)₁₄CH₃ | OH | 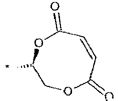 |
| 1-223 | OH | OCO(CH₂)₁₄CH₃ | 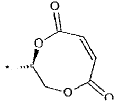 |
| 1-224 | OH | OSO₃H | 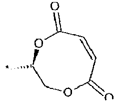 |
| 1-225 | 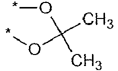 | | 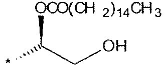 |
| 1-226 | 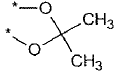 | | 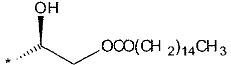 |
| 1-227 | 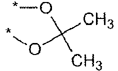 | | 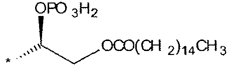 |
| 1-228 | 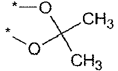 | | 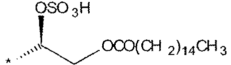 |
| 1-229 | 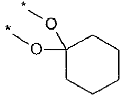 | | 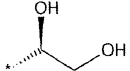 |

[Table 20]

**Table 1-20**

| Number | X¹ | X² | A |
|---|---|---|---|
| 1-230 | 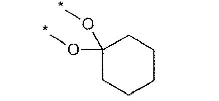 | | 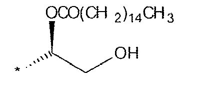 |
| 1-231 | 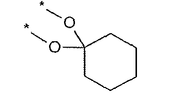 | | 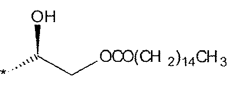 |
| 1-232 | 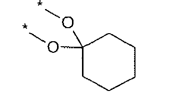 | | 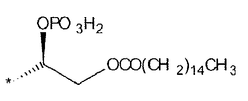 |
| 1-233 | 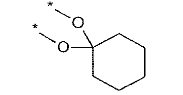 | | 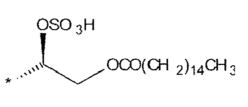 |
| 1-234 | 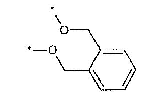 | | 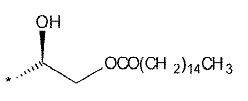 |
| 1-235 | 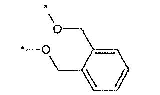 | | 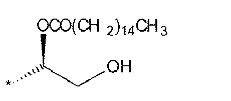 |
| 1-236 | 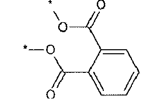 | | 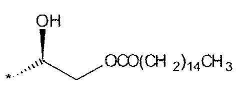 |
| 1-237 | 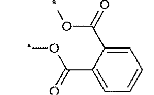 | | 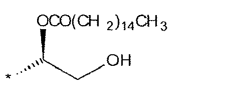 |
| 1-238 | 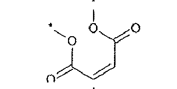 | | 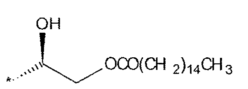 |
| 1-239 | 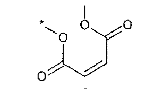 | | 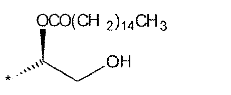 |
| 1-240 | 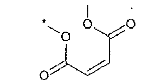 | | 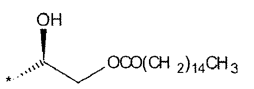 |
| 1-241 | 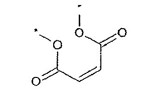 | | 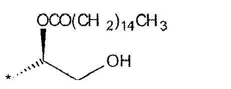 |
| 1-242 | 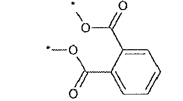 | | 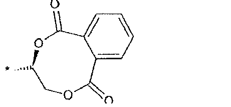 |

[Table 21]

**Table 1-21**

| Number | X¹ | X² | A |
|---|---|---|---|
| 1-243 | OCON(Et)₂ | OH | 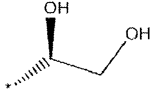 |
| 1244 | OCON(Et)₂ | OH | 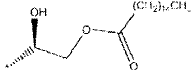 |
| 1-245 | OH | OCON(Et)₂ | 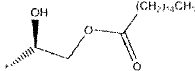 |
| 1-246 1-247 | OCON(Et)₂ OCOO^{t}Bu | OCON(Et)₂ OH | 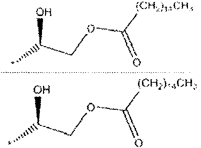 |
| 1-248 | OH | COO^{t}Bu | 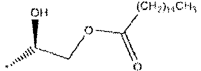 |
| 1-249 | OTs | OH | 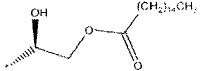 |
| 1-250 | OH | OTs | |
| 1-251 | 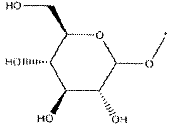 | OH | 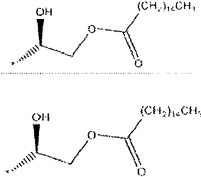 |
| 1-252 | 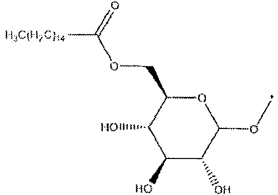 | OH | 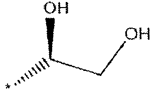 |
| 1-253 | OH | 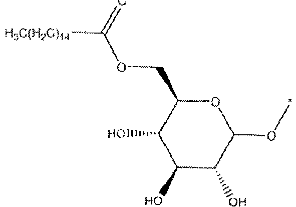 | 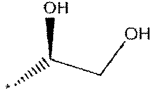 |

[Table 22]

**Table 1-22**

| Number | X¹ | X² | A |
|---|---|---|---|
| 1-254 | 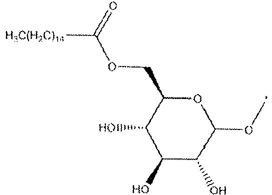 | OH | 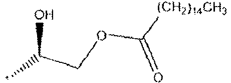 |
| 1-255 | OH | 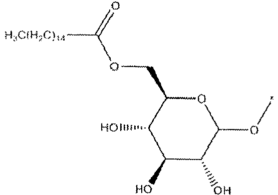 | 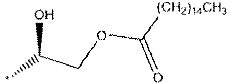 |
| 1-256 | OH | OH | 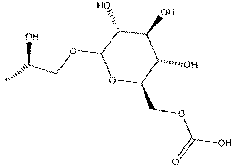 |
| 1-257 | OCO(CH₂)₁₄CH₃ | OH | 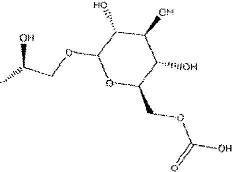 |
| 1-258 | OMe | NHⁿPr | 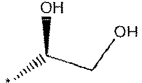 |
| 1-259 | OBn | NHⁿPr | 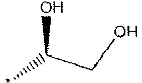 |
| 1-260 | NHⁿPr | OBn | 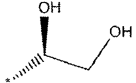 |
| 1-261 | OBn | NHⁿPr | 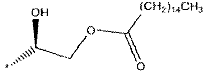 |
| 1-262 | OH | NHⁿPr | 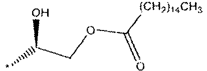 |

[Table 23]

**Table 2-1**

| Number | X¹ | X² | A |
|---|---|---|---|
| 2-1 | OCO^{t}Bu | OCOPh | (CH₂)₂OCO(CH₂)₁₄CH₃ |
| 2-2 | OCO^{t}Bu | Ph | CH(OH)(CH)₂CO(CH₂)₁₄CH₃ |
| 2-3 | N(Me)Ph | Ph | CHMe(CH₂)₂CO(CH₂)₁₄CH₃ |
| 2-4 | OH | OH | (CH₂)₂OPh |
| 2-5 | OEt | Ph | 4-Cl-Ph |
| 2-6 | OEt | Ph | 2-Furanyl |
| 2-7 | OSO₂Ph | OBn | CH₂COOMe |
| 2-8 | OBn | OBn | (CH₂)₂OH |
| 2-9 | OBn | OBn | (CH₂)₂OPh |
| 2-10 | OH | OH | (CH₂)₂OCO(CH₂)₁₄CH₃ |
| 2-11 | H | OBn | (CH₂)₂OCO(CH₂)₁₄CH₃ |
| 2-12 | OBn | OBn | (CH₂)₂OCO(CH₂)₁₄CH₃ |
| 2-13 | OMe | OMe | CH(OH)(CH₂)₂CO(CH₂)₁₄CH₃ |
| 2-14 | OH | H | 4-Cl-Ph |
| 2-15 | 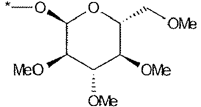 | OH | 4-Cl-Ph |
| 2-16 | 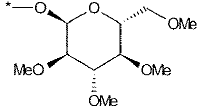 | OBn | (CH₂)₂OCO(CH₂)₁₄CH₃ |
| 2-17 | OCO^{t}Bu | OH | Ph-4-Cl |
| 2-18 | OMe | OMe | 2-Furanyl |
| 2-19 | OH | OMe | CH₂COOMe |
| 2-20 | OH | OH | 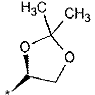 |
| 2-21 | OH | OH | 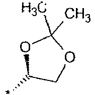 |
| 2-22 | OAc | OH | 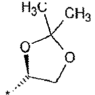 |
| 2-23 | OAc | OBn | 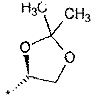 |
| 2-24 | OH | OEt | 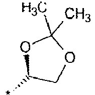 |

[Table 24]

**Table 2-2**

| Number | X¹ | X² | A |
|---|---|---|---|
| 2-25 | OH | OBn | 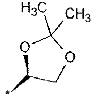 |
| 2-26 | OH | OBn | 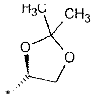 |
| 2-27 | OCO(CH₂)₁₄CH₃ | OBn | 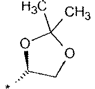 |
| 2-28 | H | OBn | 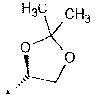 |
| 2-29 | OBn | OH | 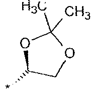 |
| 2-30 | OBn | H | 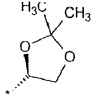 |
| 2-31 | OBn | OBn | 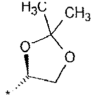 |
| 2-32 | OSi(Me)₂^{t}Bu | OH | 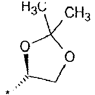 |
| 2-33 | OSi(Me)₂^{t}Bu | OBn | 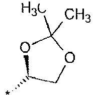 |
| 2-34 | OSiMe)₂^{t}Bu | OCO(CH₂)₁₄CH₃ | 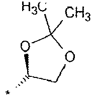 |
| 2-35 | OSO₂Ph-4-Me | OBn | 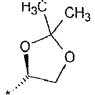 |
| 2-36 | OBn | OSO₂Ph-4-Me | 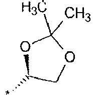 |

[Table 25]

**Table 2-3**

| Number | X¹ | X² | A |
|---|---|---|---|
| 2-37 | OSO₂Ph-4-Me | OCO^{t}Bu | 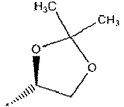 |
| 2-38 | OSO₂Ph-4-Me | OCO^{t}Bu | 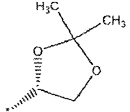 |
| 2-39 | OCO^{t}Bu | OSO₂Ph-4-Me | 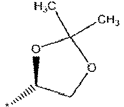 |
| 2-40 | OCH₂Ph-4-Br | OH | 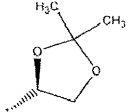 |
| 2-41 | OH | OCH₂Ph-4-Br | 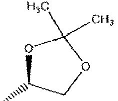 |
| 2-42 | OCH₂COPh-4-F | OCH₂Ph-4-F | 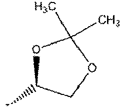 |
| 2-43 | Ph-4-Cl | OEt | 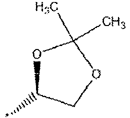 |
| 2-44 | OEt | Ph-4-Cl | 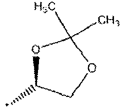 |
| 2-45 | OCO^{t}Bu | OH | 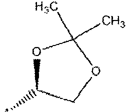 |
| 2-46 | OCO^{t}Bu | Ph | 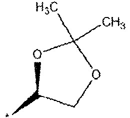 |

[Table 26]

**Table 2-4**

| Number | X1 | X2 | A |
|---|---|---|---|
| 2-47 | OCO^{t}Bu | Ph-4-Cl | 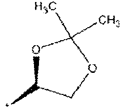 |
| 2-48 | OCO^{t}Bu | Ph | 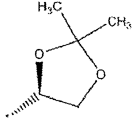 |

[Table 27]

**Table 2-5**

| Number | X¹ | X² | A |
|---|---|---|---|
| 2-49 | OCO^{t}Bu | OBn | 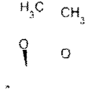 |
| 2-50 | OCO^{t}Bu | Ph-4-Cl | 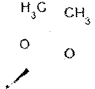 |
| 2-51 | OCO^{t}Bu | Ph-4-Cl | 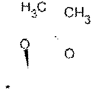 |
| 2-52 | Ph-2.4·Cl | OEt | 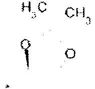 |
| 2-53 | OEt | Ph-2.4·Cl | 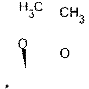 |
| 2-54 | Ph-24·Cl | OCO^{t}Bu | 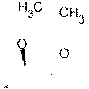 |
| 2-55 | OCO^{t}Bu | Ph-2.4-Cl | 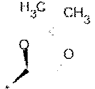 |
| 2-56 | OCO^{t}Bu | Ph-2.4-Cl | 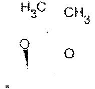 |
| 2-57 | OCO^{t}Bu | OCOPh-4-F | 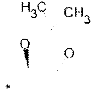 |
| 2-58 | OCO^{t}Bu | OCOPh-4·Cl | 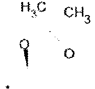 |
| 2-59 | OCO^{t}Bu | OCOPh-2.4·Cl | 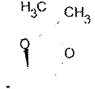 |
| 2-60 | OH | OCH₂COPh-4-OMe | 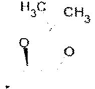 |

[Table 28]

**Table 2-6**

| Number | X¹ | X² | A |
|---|---|---|---|
| 2-61 | N(Me)Ph-4-Cl | OEt | 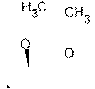 |
| 2-62 | OCO^{t}Bu | OH | 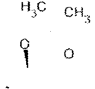 |
| 2-63 | OAc | OBn | 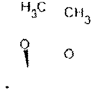 |
| 2-64 | OCO^{t}Bu | OH | 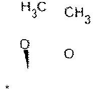 |
| 2-65 | OCO^{t}Bu | OBn | 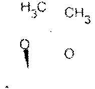 |
| 2-66 | OTs | OBn | 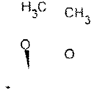 |
| 2-67 | OTs | OCO^{t}Bu | 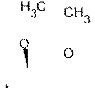 |
| 2-68 | OCO(CH₂)₁₄ CH₃ | OBn | 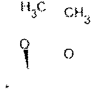 |
| 2-69) | OBn | OBn | 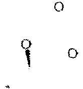 |
| 2-70 | OH | OCO(CH₂)₁₄CH₃ | 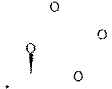 |
| 2-71 | OCO(CH₂)₁₄CH₃ | OH | 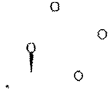 |
| 2-72 | OCO^{t}Bu | OCOPh | 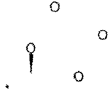 |

[Table 29]

**Table 2-7**

| Number | X¹ | X² | A |
|---|---|---|---|
| 2-73 | H | OBn | 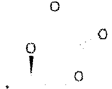 |
| 2-74 | OSO Ph | OBn | 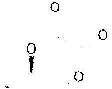 |
| 2-75 | OEt | Ph-4-Cl | 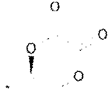 |
| 2-76 | Ph-2.4·Cl | OCOtBu | 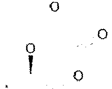 |
| 2-77 | OCOtBu | Ph-2.4-Cl | 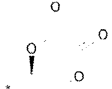 |
| 2-78 | N(Me)Ph-4-Cl | OEt | 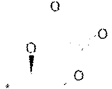 |
| 2-79 | OCO^{t}Bu | OCOPh | 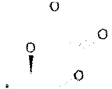 |
| 2-80 | Ph-2.4-Cl | OCO^{t}Bu | 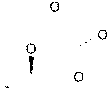 |
| 2-81 | N(Me)Ph | Ph | 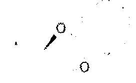 |
| 2-82 | N(Me)Ph-4-Cl | Ph | 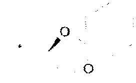 |
| 2-83 | N(Me)Ph-4-Cl | OEt | 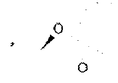 |
| 2-84 | Ph-4-Cl | OEt | 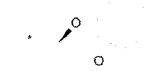 |

[Table 30]

**Table 2-8**

| Number | X¹ | X² | A |
|---|---|---|---|
| 2-85 | OCO^{t}Bu | OCOPh-4-F | 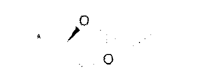 |
| 2-86 | OCO^{t}Bu | OCOph-4-F | 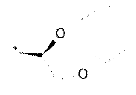 |
| 2-87 | OCO^{t}Bu | OCOPh-4-Cl | 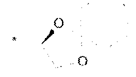 |
| 2-88 | OCO^{t}Bu | OCOPh-2.4-Cl | 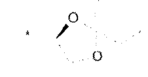 |
| 2-89 | OEt | Ph-4-Cl | 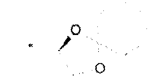 |
| 2-90 | 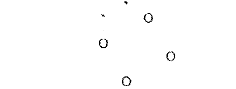 | | 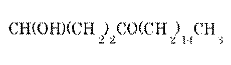 |
| 2-91 | 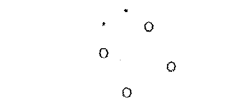 | | 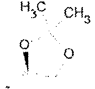 |
| 2-92 | 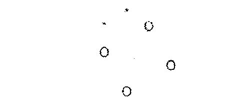 | | 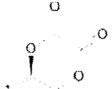 |
| 2-93 | 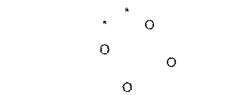 | | 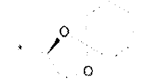 |
| 2-94 | 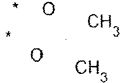 | | 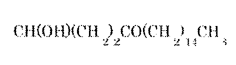 |
| 2-95 | 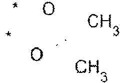 | | 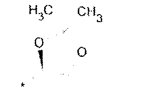 |
| 2-96 | 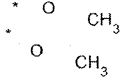 | | 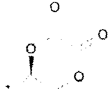 |

[Table 31]

**Table 2-9**

| Number | X¹ | X² | A |
|---|---|---|---|
| 2-97 | 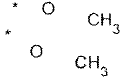 | | 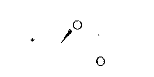 |
| 2-98 | 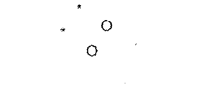 | | 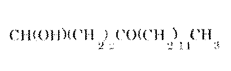 |
| 2-99 | 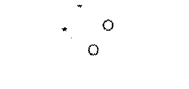 | | 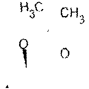 |
| 2-100 | 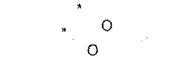 | | 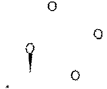 |
| 2-101 | 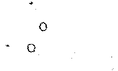 | | 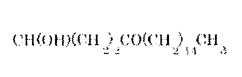 |
| 2-102 | 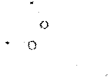 | | 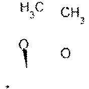 |
| 2-103 | 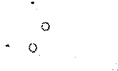 | | 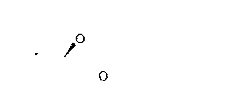 |
| 2-104 | 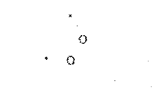 | | 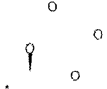 |
| 2-105 | 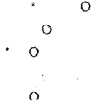 | | 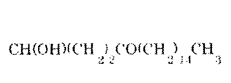 |
| 2-106 | 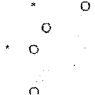 | | 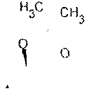 |
| 2-107 | 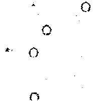 | | 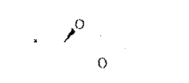 |
| 2-108 | 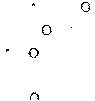 | | 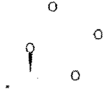 |

[Table 32]

**Table 2-10**

| Number | X¹ | X² | A |
|---|---|---|---|
| 2-109 | 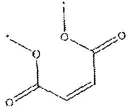 | | CH(OH)(CH₂)₂CO(CH₂)₁₄CH₃ |
| 2-110 | 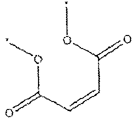 | | 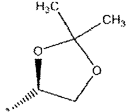 |
| 2-111 | 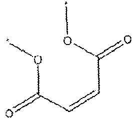 | | 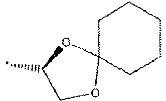 |
| 2-112 | 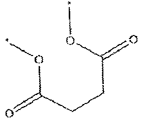 | | 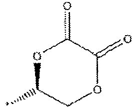 |
| 2-113 | 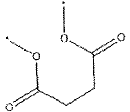 | | 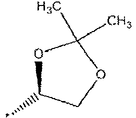 |
| 2-114 | 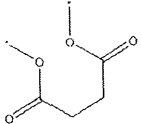 | | 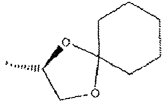 |
| 2-115 | 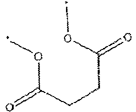 | | 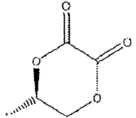 |

[Table 33]

**Table 2-11**

| Number | X1 | X2 | A |
|---|---|---|---|
| 2-116 | OH | NHⁿPr | 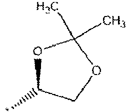 |
| 2-117 | OCO(CH₂)₁₄CH₃ | NHⁿPr | 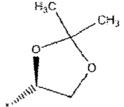 |
| 2-118 | NHⁿPr | OH | 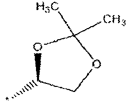 |
| 2-119 | OMe | NHⁿPr | 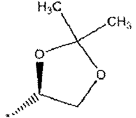 |
| 2-120 | OBn | NHⁿPr | 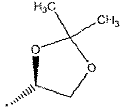 |
| 2-121 | NHⁿPr | OBn | 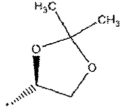 |
| 2-122 | OH | NHCH₂-3-(6-Cl-Py) | 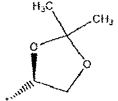 |
| 2-123 | NHCH₂-3-(6-Cl-Py) | OH | 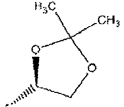 |

[Table 34]

**Table 2-12**

| Number | X¹ | X² | A |
|---|---|---|---|
| 2-124 | OMe | OH | 2-Furanyl |
| 2-125 | OMe | OMe | 2-Furanyl |
| 2-126 | OMe | NHⁿPr | 2-Furanyl |
| 2-127 | OCO(CH₂)₁₄CH₃ | OMe | 4-Cl-Ph |
| 2-128 | OMe | OMe | 4-Cl-Ph |
| 2-129 | OMe | NHⁿPr | 4-Cl-Ph |
| 2-130 | OH | OMe | CH₂COOMe |
| 2-131 | OCO(CH₂)₁₄CH₃ | OMe | CH₂COOMe |
| 2-132 | OMe | OCO(CH₂)₁₄CH₃ | CH₂COOMe |
| 2-133 | OCO(CH₂)₁₄CH₃ | OMe | CH₂COOMe |
| 2-134 | OCO(CH₂)₁₄CH₃ | OBn | CH₂COOMe |

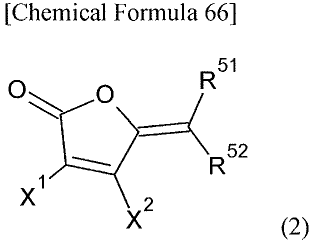

[Table 35]

**Table 3-1**

| Number | X¹ | X² | R⁵¹ | R⁵² |
|---|---|---|---|---|
| 3-1 | OH | OH | H | H |
| 3-2 | OH | OH | H | OH |
| 3-3 | OH | OH | Me | H |
| 3-4 | OH | OH | H | Me |
| 3-5 | OH | OH | Me | Me |
| 3-6 | OBn | OH | H | H |
| 3-7 | OH | OBn | H | H |
| 3-8 | OBn | OBn | H | H |
| 3-9 | H | OH | Me | Me |
| 3-10 | OH | H | Me | Me |
| 3-11 | OBn | OBn | Me | Me |
| 3-12 | OCO(CH₂)₁₄CH₃ | OH | H | H |
| 3-13 | OH | OCO(CH₂)₁₄CH₃ | H | H |
| 3-14 | OH | OH | CH₂OH | H |
| 3-15 | OH | OH | H | CH₂OH |
| 3-16 | OH | OBn | CH₂OH | H |
| 3-17 | OH | OBn | H | CH₂OH |
| 3-18 | OH | OH | CH₂OSi(Me)₂^{t}Bu | H |
| 3-19 | OBn | OBn | CH₂OSi(Me)₂^{t}Bu | H |
| 3-20 | OH | OH | H | CH₂OSi(Me)₂^{t}Bu |
| 3-21 | OBn | OBn | H | CH₂OSi(Me)₂^{t}Bu |
| 3-22 | OH | OH | CH₂OCO(CH₂)₁₄CH₃ | H |
| 3-23 | OH | OBn | CH₂OCO(CH₂)₁₄CH₃ | H |
| 3-24 | OBn | OH | CH₂OCO(CH₂)₁₄CH₃ | H |
| 3-25 | OH | OH | H | CH₂OCO(CH₂)₁₄CH₃ |
| 3-26 | OH | OBn | H | CH₂OCO(CH₂)₁₄CH₃ |
| 3-27 | OBn | OH | Me | CH₂OCO(CH₂)₁₄CH₃ |
| 3-28 | Cl | OH | H | CH₂OH |
| 3-29 | Cl | OH | CH₂OH | H |
| 3-30 | OH | Cl | Me | CH₂OH |
| 3-31 | OH | Cl | CH₂OH | H |
| 3-32 | Cl | OH | H | CH₂OH |
| 3-33 | OH | Cl | CH₂OH | Me |
| 3-34 | OH | Ph | CH₂OH | H |
| 3-35 | OH | Ph | H | CH₂OH |
| 3-36 | OH | Ph-4-Cl | CH₂OH | H |
| 3-37 | OH | Ph-4-Cl | H | CH₂OH |
| 3-38 | Ph-4-Cl | OH | CH₂OH | H |
| 3-39 | Ph-4-Cl | OH | H | CH₂OH |
| 3-40 | OH | Ph-4-Cl | CH₂OH | H |
| 3-41 | OH | Ph-4-Cl | H | CH₂OH |
| 3-42 | OCO^{t}Bu | OH | CH₂OH | H |
| 3-43 | OCO^{t}Bu | OH | H | CH₂OH |
| 3-44 | OH | OCO^{l}Bu | CH₂OH | H |
| 3-45 | OH | OCO^{t}Bu | H | CH₂OH |
| 3-46 | N(Me)Ph-4-Cl | OH | CH₂OH | H |

[Table 36]

**Table 3-2**

| Number | X¹ | X² | R⁵¹ | R⁵² |
|---|---|---|---|---|
| 3-47 | 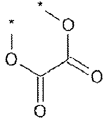 | | CH₂OH | H |
| 3-48 | 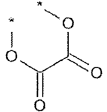 | | H | CH₂OH |
| 3-49 | 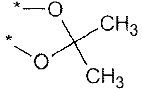 | | CH₂OH | H |
| 3-50 | 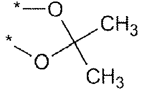 | | H | CH₂OH |
| 3-51 | 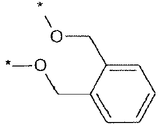 | | CH₂OH | H |
| 3-52 | 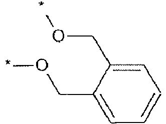 | | H | CH₂OH |
| 3-53 | 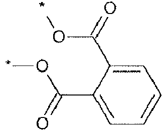 | | CH₂OH | H |
| 3-54 | 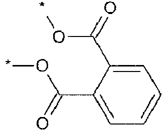 | | H | CH₂OH |

[Table 37]

**Table 3-3**

| Number | X¹ | X² | R⁵¹ | R⁵² |
|---|---|---|---|---|
| 3-55 | OH | OMe | COOMe | H |
| 3-56 | OH | OMe | H | COOMe |
| 3-57 | OMe | OH | COOMe | H |
| 3-58 | OMe | OH | H | COOMe |
| 3-59 | OCO(CH₂)₁₄CH₃ | OMe | COOMe | H |
| 3-60 | OMe | OCO(CH₂)₁₄CH₃ | COOMe | H |
| 3-61 | NHⁿPr | OCO(CH₂)₁₄CH₃ | COOMe | H |
| 3-62 | OCO(CH₂)₁₄CH₃ | OH | CH₂OH | H |
| 3-63 | OCO(CH₂)₁₄CH₃ | OMe | CH₂OH | H |
| 3-64 | OMe | NHⁿPr | COOMe | H |
| 3-65 | NHⁿPr | OMe | COOMe | H |
| 3-66 | OMe | NHⁿPr | CH₂OH | H |
| 3-67 | OMe | NHⁿPr | H | CH₂OH |
| 3-68 | OBn | NHⁿPr | COOMe | H |
| 3-69 | NHⁿPr | OBn | COOMe | H |
| 3-70 | NHⁿPr | OBn | H | COOMe |

The melting point and the refractive index were measured for some of the compounds shown above in Table 1-1 to Table 3-3. These physical properties are shown below in Tables 4-1 and 4-2.

[Table 38]

**Table 4-1**

| Number | Melting point (°C) | Refractive index (nD) |
|---|---|---|
| 1-2 | | 1.5420 (21.1°C) |
| 1-105 | 95-97 | |
| 1-107 | 95-96 | |
| 1-144 | 43-45 | |
| 1-148 | 88-90 | |
| 1-149 | 36-38 | |
| 1-159 | 52-54 | |
| 1-214 | 83-85 | |
| 1-247 | 128-132 | |
| 1-249 | 96-100 | |
| 1-251 | 146-148 | |
| 1-252 | 120-123 | |
| 1-262 | 95-96 | |

[Table 39]

**Table 4-2**

| Number | Melting point (°C) | Refractive index (nD) |
|---|---|---|
| 2-4 | 128-131 | |
| 2-10 | 90-93 | |
| 2-28 | | 1.4955 (20.3°C) |
| 2-31 | 90-93 | |
| 2-35 | 75-77 | |
| 2-37 | 86-88 | |
| 2-43 | 105-106 | |
| 2-47 | 81-83 | |
| 2-51 | 149-151 | |
| 2-61 | 188-189 | |
| 2-69 | 108-110 | |
| 2-122 | 155-158 | |
| 2-125 | | 1.5102 (20.7°C) |
| 3-19 | | 1.5829 (21.9°C) |
| 4-1 | 96-98 | |

¹H-NMR spectra were measured for some of the compounds shown above in Table 1-1 to Table 3-3. The data from these measurements are shown below in Tables 5-1 to 5-4.

[Table 40]

**Table 5-1**

| Number | ¹H-NMR (δ, ppm) |
|---|---|
| 1-15 | 1.87 (br.t, 1H. OH), 2.51 (br.d, 1H, OH), 3.72-3.87 (m, 3H), 4.91 (dd, 1H), 5.04 (dd, 2H), 6.29 (d, 1H), 7.30-7.40 (m, 5H) |
| 1-42 | 1.33(s, 9H), 2.09-2.12(m, 1H), 2.49(d, 1H), 3.79-3.82(m, 1H), 3.83-3.90(m, 1H), 4.02-4.05(m, 1H), 4.88(d, 1H), 5.29(s, 2H), 7.34-7.36(m, 2H), 7.40-7.42(m, 3H) |
| 1-43 | 3.75-3.81(m, 2H), 3.91-3.92(m, 1H), 4.69(d, 1H), 5.08-5.23(m, 4H), 7.22-7.27(m, 2H), 7.35-7.39(m, 8H) |
| 1-108 | 0.88(t, 3H), 1.25-1.32(m, 26H), 2.27(d, 2H), 4.23(dd, 1H), 4.32(dd, 1H), 4.80(d, 1H), 5.10(s, 2H), 5.15(s, 2H), 5.38(ddd, 1H), 7.22-7.26(m, 2H), 7.34-7.38(m, 8H) |
| 1-145 | 0.88(t, 3H), 1.13(s, 9H), 1.20-1.25(m, 26H), 1.57-1.60(m, 2H), 2.30(t, 2H), 4.32(dd, 1H), 4.39(dd, 1H), 4.83(d, 1H), 5.38-5.39(m, 1H) |
| 1-150 | 0.88(t, 3H), 1.23-1.30(m, 26H), 2.21(t, 2H), 3.83-3.86(m, 2H), 4.90(d, 1H), 5.08-5.12(m, 2H), 5.14-5.20(m, 3H), 7.22-7.25(m, 2H), 7.33-7.40(m, 8H) |
| 1-152 | 0.88(t, 3H), 1.25-1.28(m, 26H), 1.95(s, 3H), 2.28(t, 2H), 4.23(dd, 1H), 4.30(dd, 1H), 4.80(d, 1H), 5.08-5.17(m, 4H), 5.32-5.36(m, 1H), 7.23-7.24(m, 2H), 7.26-7.40(m, 8H) |
| 1-153 | 0.88(t, 3H), 1.08(t, 3H), 1.30-1.20(m, 26H), 2.29(t, 2H), 3.42(qd, 1H), 3.55(qd, 1H), 3.70(ddd, 1H), 4.17(dd, 1H), 4.30(dd, 1H), 4.68(d, 1H), 5.10-5.22(m, 4H), 7.20-7.23(m, 2H), 7.35-7.41(m, 8H) |
| 1-154 | 0.88(t, 3H), 1.16(s, 9H), 1.25-1.30(m, 26H), 2.27(t, 2H), 4.26(dd, 1H), 4.33(dd, 1H), 4.80(d, 1H), 5.04-5.15(m, 4H), 5.39(ddd, 1H), 7.22-7.24(m, 2H), 7.26-7.39(m, 8H) |
| 1-155 | 0.88(t, 6H), 1.24-1.26(m, 52H), 1.31(s, 9H), 2.30(t, 4H), 4.31-4.34(m, 1H), 4.38-4.41 (m, 1H), 4.68(d, 1H), 5.45-5.47(m, 1H) |
| 1-156 | 0.88(t, 3H), 1.25-1.59(m, 35H), 2.33(t, 2H), 4.11-4.15(m, 1H), 4.20-4.24(m, 1H), 4.37(dd, 1H), 4.84(d, 1H), 5.27(d, 1H), 5.32(d, 1H), 7.33-7.36(m, 2H), 7.39-7.42(m, 3H) |

[Table 41]

**Table 5-2**

| Number | 1H-NMR (δ, ppm) |
|---|---|
| 1-157 | 0.88(t, 3H), 1.25-1.33(m,26H), 1.55(s, 9H), 2.13(s, 3H), 2.30(t, 2H), 4.31-4.34(m, 2H), 4.90(d, 1H), 5.10(d, 1H), 5.24(d, 1H), 5.42-5.46(m, 1H), 7.37-7.42(m, 5H) |
| 1-158 | 0.88(t, 3H), 1.25-1.36(m, 61 H), 2.29(t, 2H), 2.37(t, 2H), 4.28-4.38(m, 2H), 4.91 (d, 1H), 5.10(d, 1H), 5.22(d, 1H), 5.45-5.48(m, 1H), 7.30-7.42(m, 5H) |
| 1-160 | 0.62(s, 6H), 0.88(s, 9H), 3.90(dd, 1H), 3.76(dd, 1H), 3.95(ddd, 1H), 4.74(d, 1H), 5.10(s, 2H), 5.15(d, 1H), 5.22(d, 1H), 7.22-7.24(m, 2H), 7.34-7.40(m, 8H) |
| 1-161 | 0.06(s, 6H), 0.80-0.83(m, 12H), 1.17-1.24(m, 26H), 2.1 (t, 2H), 3.69-3.71 (m, 2H), 4.88(d, 1H), 5.03(s, 2H), 5.09-5.13(m, 3H), 7.17-7.20(m, 2H), 7.28-7.33(m, 8H) |
| 1-162 | 0.01(s, 6H), 0.81(s, 9H), 3.85-3.95(m, 2H), 4.43-4.46(m, 1H), 4.69(d, 1H), 5.13-5.26(m, 4H), 7.24-7.26(m, 2H), 7.34-7.44(m, 8H) |
| 1-215 | 0.88(t, 3H), 1.24-1.28(m, 26H), 2.59(t, 2H), 3.81-3.87(m, 2H), 3.88-3.95(m, 1H), 4.92(d, 1H) |
| 1-244 | 0.88(t, 3H), 1.16-1.29(m, 30H), 1.61-1.65(m, 4H), 2.36(t, 2H), 3.33-3.42(m, 4H), 4.04-4.09(m, 1H), 4.27(dd, 1H), 4.36(dd, 1H), 4.82(d, 1H) |
| 1-246 | 0.88(t, 3H), 1.14-1.29(m, 39H), 1.59-1.65(m, 2H), 2.34(t, 3H), 3.26-3.44(m, 8H), 4.18-4.25(m, 2H), 4.39(dd, 1H), 5.29(d, 1H) |
| 1-259 | 0.86 (t, 3H), 1.47(tq, 2H), 2.13(br.s, 1H, OH), 2.82(br.s, 1H, OH), 3.18-3.24(m, 2H), 3.34-3.58(m, 1H), 3.72-3.74(m, 1H), 3.97(br.s, 1H), 4.75(d, 1H), 4.84(br.s, 1H, NH), 4.97(d, 1H), 5.08(d, 1H), 7.33-7.41(m, 5H) |
| 1-261 | 0.84(t, 3H), 0.88(t, 3H), 1.25-1.32(m, 24H), 1.45(tq, 2H), 1.57-1.63(m, 2H), 2.34(t, 2H), 3.16-3.29(m, 2H), 4.02(dd, 1H), 4.09(dd, 1H), 4.19-4.22(m, 1H), 4.72(d, 1H), 4.97(d, 1H), 5.04(d, 1H), 7.31-7.40(m, 5H) |

[Table 42])

**Table 5-3**

| Number | ¹H-NMR (δ, ppm) |
|---|---|
| 2-4 | 7.26 (t, 2H, J = 8.2 Hz), 6.93-6.90 (m, 3H), 4.91 (dd, 1H, J = 3.2 and 8.4 Hz), 4.13 and 4.12 (d×2, 1H, J = 7.2 Hz)), 2.45-2.39 (m, 1H), 1.96-1.88 (m, 1H) |
| 2-10 | 4.90-4.80 (m, 1H), 4.35-4.15 (m, 1H), 4.25-4,15 (m, 1H), 2.32 (t, 2H, J = 7.6 Hz), 2.35-2.20 (m, 1H), 2.05-1.95 (m, 1H), 1.25 (s, 26H), 0.88 (t, 3H, J = 7.0 Hz) |
| 2-12 | 7.38-7.20 (m, 10H), 5.20, 5.16, 5.13 and 5.09 (d×4, 4H, J = 11 Hz), 4.72 (dd, 1H, J = 3.4 and 8.6 Hz), 4.25-4.15 (m, 2H), 4.13 and 4.12 (dx2, 1H, J = 7.2 Hz), 2.30-2.18 (m, 1H), 2.26 (t, 2H, J = 7.6 Hz), 1.85-1.73 (m. 1H), 1.26 (s, 26H), 0.88 (t, 3H, J = 7.0 Hz) |
| 2-22 | 1.35(s, 3H), 1.38(s, 3H), 2.36(s, 3H), 3.72(dd, 1H), 4.08-4.12(m, 1H), 4.13-4.23(m, 1H), 4.42-4.43 (m, 1H), 4.69(d, 1H) |
| 2-23 | 1.37(s, 3H), 1.40(s, 3H), 2.22(s, 3H), 4.08(dd, 1H), 4.14(dd, 1H), 4.40(ddd, 1H), 5.29(d, 1H), 5.31 (d, 1H), 5.37(d, 1H), 7.34-7.41 (m, 5H) |
| 2-27 | 0.88(t, 3H), 1.24-1.28(m, 26H), 1.37(s, 3H), 1.40(s, 3H), 2.49(t, 2H), 4.08-4.13(m, 2H), 4.38(ddd, 1H), 4.71 (d, 1H), 5.27(d, 1H), 5.35(d, 1H), 7.33-7.41 (m, 5H) |
| 2-32 | 0.24(s, 6H), 0.97(s, 9H), 1.37(s, 3H), 1.43(s, 3H), 3.96(dd, 1H), 4.12(dd, 1H), 4.45-4.47(m, 1H), 4.72(d, 1H) |
| 2-33 | 0.32(s, 6H), 0.98(s, 9H), 1.24(s, 3H), 1.28(s, 3H), 4.01 (dd, 1H), 4.06-4.13(m, 1H), 4.50(d, 1H), 5.45(d, 1H), 5.49(d, 1H), 7.36-7.40(m, 5H) |
| 2-34 | 0.25(s, 6H), 0.88(t, 3H), 0.93(s, 9H), 1.26-1.37(m, 32H), 2.53(t, 2H), 4.03(dd, 1H), 4.14(dd, 1H), 4.24-4.27(m, 1H), 5.19(d, 1H) |
| 2-45 | 1.34(s, 9H), 1.38(s, 3H), 1.40(s, 3H), 4.11(dd, 1H), 4.21(dd, 1H), 4.42-4.46(m, 1H), 4.68(d, 1H) |
| 2-49 | 1.33(s, 9H), 1.36(s, 3H), 1.41 (s, 3H), 4.06-4.15(m, 2H), 4.36-4.40(m, 1H), 4.70(d, 1H), 5.25(d, 1H), 5.24(d, 1H), 7.33-7.42(m, 5H) |

[Table 43]

**Table 5-4**

| Number | 1H-NMR (δ, ppm) |
|---|---|
| 2-116 | 0.96(t, 3H), 1.36(s, 3H), 1.45(s, 3H), 1.61(dq, 2H), 3.45(dt, 2H), 3.75(dd, 1H), 4.03(dd, 1H), 4.54-4.58(m, 2H), 4.84(d, 1H) |
| 2-120 | 0,86(t, 3H), 1.35(s, 3H), 1.40(s, 3H), 1.46(tq, 2H), 3.19-3.26(m, 2H), 3.59(dd, 2H), 3.98(dd, 1H), 4.51 (dd, 1H), 4.63(br.s, 1H, NH), 4.81 (d, 1H), 4.99(d, 1H), 5.91 (d, 1H), 7.32-7.41 (m, 5H) |
| 2-125 | 7.45 (d, 1H, J = 1.6 Hz), 6.49 (d, 1H, J = 3.2 Hz), 6.40 (dd, 1H, J = 1.8, 3.4 Hz), 5.59 (s, 1H), 4.11 (s, 3H), 3.91 (s, 3H) |
| 2-128 | 7.37 (d, 2H, J = 8.4 Hz), 7.25 (d, 2H, J = 8.8 Hz), 5.49 (s, 1H), 4.08 (s, 3H), 3.90 (s, 3H) |
| 2-130 | 2.54 (dd, 1H), 2.88 (dd, 1H), 3.74 (s, 3H), 4.18 (s, 3H), 5.07 (dd, 1H), 5.12 (s, 1H, OH) |
| 2-131 | 0.88 (t, 3H), 1.26-1.37 (m, 24H), 1.71 (tq, 2H), 2.57 (t, 2H), 2.66 (dd, 1H), 2.91 (dd, 1H), 3.74 (s, 3H), 4.06 (s, 3H), 5.15 (dd, 1H) |
| 3-55 | 3.73 (s, 3H), 4.17 (s, 3H), 5.45 (s, 1H) (CD₃OD) |
| 3-59 | 0.88 (t, 3H), 1.26-1.40 (m, 24H), 1.73 (dq, 2H), 2.57 (t, 2H), 3.81 (s, 3H), 4.11 (s, 3H), 5.67 (s, 1H) |
| 4-1 | 7.47 (d, 2H, J = 8.4 Hz), 7.36 (d, 2H, J = 8.0 Hz), 4.08 (s, 3H), 4.02 (s, 3H), 2.63 (br s, 1H) |

### Test Example 1

### (Tomato Yellow Leaf Curl Virus Test)

Each of the compounds 1-244 and 1-249 was dissolved in dimethyl sulfoxide, and each of the obtained solutions was mixed with water containing an added nonionic surfactant, thus preparing a chemical agent composed of an aqueous solution containing the compound 1-244 in a concentration of 600 ppm, the nonionic surfactant in a concentration of 0,02% and dimethyl sulfoxide in a concentration of 1%, and a chemical agent composed of an aqueous solution containing the compound 1-249 in a concentration of 600 ppm, the nonionic surfactant in a concentration of 0.02% and dimethyl sulfoxide in a concentration of 1%.

Tomato plants (variety: Reiyo) were inoculated with the yellow leaf curl virus by graft inoculation, and the chemical agents described above were sprayed onto the tomato plants 3 days before inoculation, 1 week after inoculation, 2 weeks after inoculation, and 3 weeks after inoculation, in an amount of 3 to 5 ml per plant.

The health of the tomato plants was inspected 3 weeks after inoculation and then 4 weeks after inoculation, and the plant antiviral effect was evaluated.

Both of the above compounds exhibited an excellent plant antiviral effect. Further, the compounds also displayed excellent stability.

### INDUSTRIAL APPLICABILITY

The compound of the present invention or a salt thereof is useful as an active ingredient in a plant antiviral agent.

The plant antiviral agent of the present invention contains at least one component selected from the group consisting of compounds of the present invention and salts thereof, and has a high plant antiviral activity. When the plant antiviral agent of the present invention is applied to a normal plant, infection with plant viruses can be effectively prevented (preventative effect). Further, when the plant antiviral agent of the present invention is applied to plant that has been infected with a plant virus, onset of the plant disease can be suppressed (curative effect).

Furthermore, the compound of the present invention or a salt thereof exhibits excellent stability, and is therefore ideal for agricultural and horticultural use.

## Claims

1. A compound represented by formula (1), or a salt thereof: 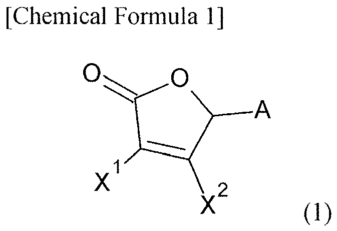 wherein within formula (1):
each of X¹ and X² independently represents a hydrogen atom, halogeno group, cyano group, nitro group, -OR¹, -NR²R³ or -CR⁴R⁵R⁶, and X¹ and X² may be linked to form an unsubstituted or substituent-bearing 5- to 8-membered ring,
each of R¹, R², R³, R⁴, R⁵ and R⁶ independently represents a hydrogen atom, unsubstituted or substituent-bearing glycosyl group, unsubstituted or substituent-bearing Cl to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing 5- to 10-membered heterocyclic group, -COR¹¹, -COOR¹², -CONR¹³R¹⁴, -SO₂R¹⁵, - PO(OH)OR¹⁶, -SO₂NR¹⁷R¹⁸, or -SiR¹⁹R²⁰R²¹,
any of R² and R³, R⁴and R⁵, R⁶ and R⁴, or R⁵ and R⁶ may be linked to form an unsubstituted or substituent-bearing 3- to 8-membered ring, and any of R⁴ and R⁵, R⁶ and R⁴, or R⁵ and R⁶ may be combined to form =O, =S or =NR²²,
each of R¹¹, R¹², R¹³, R¹⁴,R¹⁵ , R¹⁶, R¹⁷, R¹⁸, R¹⁹, R²⁰ and R²¹ independently represents a hydrogen atom, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing 5- to 10-membered heterocyclic group, or a substituent-bearing carbonyl group,
R²² represents a hydrogen atom, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, or unsubstituted or substituent-bearing 5- to 10-membered heterocyclic group,
A represents an unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, or unsubstituted or substituent-bearing C5 to C10 heterocyclic group,
although those cases in which each of X¹, X² , R²³ and R²⁴ simultaneously represents a hydroxyl group, glycosyloxy group, -OSO₃H, -OPO₃H₂ or -COR' are excluded,
when A represents an unsubstituted ethyl group, those cases in which X¹ and X² both represent hydrogen atoms are excluded, and
R' represents an unsubstituted or substituent-bearing C1 to C30 alkyl group or an unsubstituted or substituent-bearing C2 to C30 alkenyl group.

2. A compound represented by formula (2), or a salt thereof: 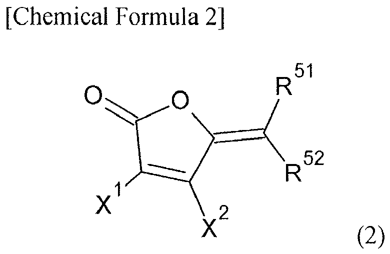 wherein within formula (2):
each of X¹ and X² independently represents a hydrogen atom, halogeno group, cyano group, nitro group, -OR¹, -NR²R³ or -CR⁴R⁵R⁶, and X¹ and X² may be linked to form an unsubstituted or substituent-bearing 5- to 8-membered ring,
each of R¹, R², R³, R⁴, R⁵ and R⁶ independently represents a hydrogen atom, unsubstituted or substituent-bearing glycosyl group, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing 5- to 10-membered heterocyclic group, -COR¹¹, -COOR¹², -CONR¹³R¹⁴, -SO₂R¹⁵, - PO(OH)OR¹⁶, -SO₂NR¹⁷R¹⁸, or -SiR¹⁹R²⁰R²¹,
any of R² and R³, R⁴ and R⁵, R⁶ and R⁴, or R⁵ and R⁶ may be linked to form an unsubstituted or substituent-bearing 3- to 8-membered ring, and any of R⁴ and R⁵, R⁶ and R⁴, or R⁵ and R⁶ may be combined to form =O, =S or =NR²²,
each of R¹¹, R¹², R¹³, R¹⁴, R¹⁵, R¹⁶, R¹⁷, R¹⁸, R¹⁹, R²⁰ and R²¹ independently represents a hydrogen atom, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing 5- to 10-membered heterocyclic group, or a substituent-bearing carbonyl group,
R²² represents a hydrogen atom, unsubstituted or substituent-bearing C1 to C30 alkyl group, unsubstituted or substituent-bearing C2 to C30 alkenyl group, unsubstituted or substituent-bearing C2 to C30 alkynyl group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, or unsubstituted or substituent-bearing 5- to 10-membered heterocyclic group,
each of R⁵¹ and R⁵² independently represents a hydrogen atom, hydroxyl group, unsubstituted or substituent-bearing amino group, halogeno group, cyano group, nitro group, unsubstituted or substituent-bearing C1 to C30 alkyl group, unsubstituted or substituent-bearing C2 to C30 alkenyl group, unsubstituted or substituent-bearing C2 to C30 alkynyl group, carboxyl group, formyl group, substituent-bearing carbonyl group, substituent-bearing carbonyloxy group, unsubstituted or substituent-bearing C3 to C20 cycloalkyl group, unsubstituted or substituent-bearing C4 to C20 cycloalkenyl group, unsubstituted or substituent-bearing C8 to C20 cycloalkynyl group, unsubstituted or substituent-bearing C6 to C10 aryl group, unsubstituted or substituent-bearing C5 to C10 heterocyclic group, unsubstituted or substituent-bearing C1 to C30 alkoxy group, unsubstituted or substituent-bearing C2 to C30 alkenyloxy group, unsubstituted or substituent-bearing C2 to C30 alkynyloxy group, unsubstituted or substituent-bearing C3 to C20 cycloalkyloxy group, unsubstituted or substituent-bearing C4 to C20 cycloalkenyloxy group, unsubstituted or substituent-bearing C8 to C20 cycloalkynyloxy group, unsubstituted or substituent-bearing C6 to C10 aryloxy group, or unsubstituted or substituent-bearing C5 to C10 heterocyclic oxy group, and R⁵¹ and R⁵² may be linked to form an unsubstituted or substituent-bearing 5- to 8-membered ring.

3. A plant antiviral agent comprising the compound according to Claim 1 or a salt thereof.

4. A plant antiviral agent comprising the compound according to Claim 2 or a salt thereof.

5. A method of preventing or curing a plant virus, the method comprising use of a plant antiviral agent comprising the compound according to Claim 1 or a salt thereof.

6. A method of preventing or curing a plant virus, the method comprising use of a plant antiviral agent comprising the compound according to Claim 2 or a salt thereof.
